# EUROPEAN PATENT APPLICATION

(11) **EP 2 548 863 A1**
(43) Date of publication of application: **23.01.2013**
(21) Application number: 11382244.9
(22) Date of filing: 18.07.2011
(51) Int. Cl.: C07D 209/14, C07D 209/18, C07D 231/54, C07D 401/04, A61K 31/405, A61K 31/416, A61P 11/00, A61P 29/00, A61P 37/00

(54) **New CRTh2 antagonists.**

(71) Applicant: Almirall, S.A., 08022 Barcelona (ES)
(72) Inventor: Eastwood, Paul Robert, 08980 Sant Feliú de Llobregat (ES); Gomez Castillo, Elena, 08980 Sant Feliú de Llobregat (ES); Gonzalrz Rodrigez, Jacob, 08980 Sant Feliú de Llobregat (ES); Lozoya Toribio, María Estrella, 08980 Sant Feliú de Llobregat (ES); Matassa, Victor Giulio, 08980 Sant Feliú de Llobregat (ES); Mir Cepeda, Marta, 08980 Sant Feliú de Llobregat (ES); Roberts, Richard Spurring, 08980 Sant Feliú de Llobregat (ES); Vidal Juan, Bernat, 08980 Sant Feliú de Llobregat (ES)
(74) Representative: Elzaburu Marquez, Alberto

(57) **Abstract**

The present invention relates to compounds of formula (I), to the process for preparing such compounds and to their use in the treatment of a pathological condition or disease susceptible to amelioration by CRTh2 antagonist activity.

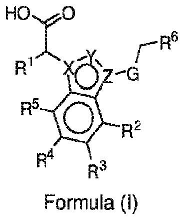

## Description

### FIELD OF THE INVENTION

The present invention relates to novel compounds having CRTh2 activity. This invention also relates to pharmaceutical compositions containing them, processes for their preparation and their use in respiratory therapies.

### BACKGROUND OF THE INVENTION

Prostaglandin D2 (PGD2) is a prostaglandin derived from the metabolism of arachidonic acid by cyclooxygenases and downstream PGD2 synthases. In the immune system, PGD2 is mainly produced by mast cells (Lewis et al., J. Immunol., 1982, 129, 1627-1631) but also although at lower levels, by macrophages and Th2 lymphocytes (Urade; J. Immunol, 1989, 143, 2982-2989. Tanaka; J. Immunol, 2000, 164, 2277-2280) in response to local tissue damage and allergic inflammation in diseases such as asthma (Murray et al., N. Engl. J. Med., 1986, 315, 800-804; Liu et al; Am. Rev. Respir. Dis., 1990, 142, 126-132; Wenzel et al., J. Allergy Clin. Immunol., 1991, 87, 540-548), rhinitis (Naclerio et al., Am. Rev. Respir. Dis., 1983, 128, 597-602; Brown et al., Arch. Otolarynol. Head Neck Surg., 1987, 113, 179-183; Lebel et al; J. Allergy Clin. Immunol., 1988, 82, 869-877) and atopic dermatitis among others. Exogenous PGD2 applied to bronchial airways elucidates many characteristics of asthmatic response suggesting that this prostaglandin plays an important pro-inflammatory role in allergic diseases (Hardy; N. Engl. J. Med., 1980, 311, 209-213. Sampson; Thorax, 1997, 52, 513-518). PGD2 binds to three different receptors, the thromboxane-type prostanoid receptor (TP) (Coleman; Br. J. Pharmacol., 1989, 96, 688-692. Francis; Br. J. Pharmacol., 1991, 104, 596-602), the PGD2 receptor (DP also known as DP1) (Boie et al; J. Biol. Chem., 1995, 270, 18910-18916) and the chemoattractant receptor-homologous molecule expressed on Th2 (CRTh2 also known as DP2) (Nagata et al; J. Immunol., 1999, 162, 1278- 1289; Powell; Prostaglandins Leukot. Essent. Fatty Acids, 2003, 69, 179-185). CRTh2 is a Gi-coupling GPCR signalling through reduction of intracellular cAMP and calcium mobilization and it is involved in the chemotaxis of Th2 lymphocytes, eosinophils and basophils (Hirai, J. Exp. Med. 2001, 193, 255-261. Shichijo; J. Pharmacol. Exp. Ther., 2003, 307, 518-525). CRTh2 also inhibits the apoptosis of Th2 lymphocytes (Xue; J. Immunol., 2009, 182, 7580-7586) and stimulates the production of IL4, IL5, and IL13 (Xue; J. Immunol., 2005, 175, 6531-6536) which are cytokines involved in important biological responses such as eosinophil recruitment and survival, mucus secretion, airway hyperresponsiveness and immunoglobulin E production among others. Therefore, molecules that antagonize the mentioned pro-inflammatory PGD2 effects mediated by CRTh2 on key cell types associated with allergic inflammation like basophils, eosinophils and Th2 lymphocytes would have a potential benefit in related pathologies.

In view of these physiological effects, CRTh2 inhibitors of varied chemical structures have been recently disclosed for the treatment or prevention of chronic and acute inflammatory diseases and other pathological conditions, diseases and disorders known to be susceptible to amelioration by inhibition of CRTh2. Several structural families of antagonists are observed.

In general antagonists can be grouped into one of the following families: indole acetic acids, phenylacetic acids, phenoxyacetic acids and tetrahydroquinolines derivatives.

Examples of indole acetic acids derivatives are those described in GB2407318, WO2003101981, WO2005019171, WO2006036994, WO2007022501, WO2008012511, WO2009063215, US2010063103.

Structurally similar analogues of the indole group have also been reported. Compounds disclosed induce structural variations such as: azaindoles (for example: WO2007068418), indolizines (for example: WO2009044147), tetrahydoindazoles (for example: WO201000694 tetrahydroindoles (for example: WO201003998 benzimidazoles (for example: WO2006034418), oxindoles (for example: WO2009061676) and thienopyrroles (for example: WO2009102462).

Additionally, tricyclic variations of some of the above structures have been disposed. For examples: WO2010008864, WO2010031183.

For examples of phenylacetic acids and structurally similar analogues, see: WO2004058164, WO2007039736, WO2008024746, WO2009061730 and WO2010003120.

Structurally similar analogues of the phenylacetic group have also been reported. Compounds disclosed include structural variations such as: naphthalene acetic acids (for example: WO2010055006), pyrimidine acetic acids (for example: WO2010027448), pyrrole acetic acids (for example: W02007144127), thiophene acetic acids (for example: W02007062797) and other heterocycle-acetic acids (for example: W02010057118).

For examples of phenoxyacetic acids see: WO2004089884 WO200501852 WO2006005909, WO2007036743, WO2008119917, WO2009004379 and W02010018109.

For examples of tetrahydroquinolines see: WO2004032848 W02006091674.

Ramatroban, {(3R)-3-[(4-fluorophenyl)sulphonylamino]-1,2,3,4-tetrahydro-9H-carbazole-9-propanoic add}, a thromboxane A2 receptor antagonist is also known to be a CRTh2 antagonist (Sugimoto et al; J. Pharmacol. Exp. Ther., 2003, 305, 347-352). A few compounds having the capacity to selectively inhibit CRTh2 are in active development. Examples of these compounds are ODC-91 01, AZD-1981, ACT-129968, AP-761, AM211, AM461, ADC-3680, ARRY-502, MK-7246 and RG-7185.

In addition, compounds having the capacity to selectively inhibit both DP1 and CRTh2 have been reported. An example of this type of compound is AMG-853.

These observations suggest that CRTh2 antagonists may have valuable properties for the treatment of diseases mediated by PGD2.

### SUMMARY OF THE INVENTION

The invention provides a compound of formula (I), or a pharmaceutically acceptable salt, N-oxide, solvate, stereiosomer or deuterated derivative thereof: 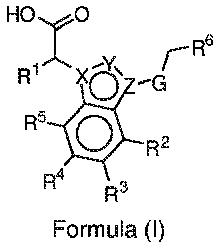 wherein:
- either X represents a nitrogen atom, Z represents a carbon atom and Y represents a nitrogen atom or a -CR⁹ group; or X represents a carbon atom, Z represents a nitrogen atom and Y represents a nitrogen atom or a -CR⁹ group;
- R¹ is selected from the group consisting of a hydrogen atom and a linear or branched C₁₋₄ alkyl group;
- R², R³, R⁴ and R⁵ are each independently selected from the group consisting of a hydrogen atom, a halogen atom, a linear or branched C₁₋₄ alkyl group, a C₃₋₇ cycloalkyl group, a linear or branched C₁₋₄ haloalkyl group, a -CN group, a -OH group, a -OR^{a} group, a -CO₂H group, a -CO₂R^{a} group, a -SR^{a} group, and a - SO₂R^{a} group;
- G is selected from the group consisting of a phenyl group and a 5- to 6-membered N-containing heteroaryl group, wherein the phenyl and heteroaryl groups are optionally substituted by one or more substituents selected from the group consisting of a halogen atom, a cyano group, a nitro group, a -CHF₂ group, a -CF₃ group, a linear or branched C₁₋₄ alkyl group, a C₃₋₇ cycloalkyl group, a -OH group, a -OR^{a} group, a -SR^{a} group and a -SO₂R^{a} group;
- R⁶ is selected from the group consisting of a -N(R⁷)C(O)R³ group, a - N(R⁷)SO₂R⁸ group and a heteroaryl group selected from the group consisting of a triazolyl group, an isoxazolyl group, a pyrazolyl group and an oxazolyl group, wherein the heteroaryl group is optionally substituted by one or more substituents selected from the group consisting of a halogen atom, a -CF₃ group, a linear or branched C₁₋₄ alkyl group, a C₃₋₇ cycloalkyl group, a benzyl group, a phenyl group, a hydroxy group and a carboxy group;
- R⁷ is selected from the group consisting of a linear or branched C₁₋₄ alkyl group; a C₃₋₇ cycloalkyl group, a linear or branched C₁-C₄ haloalkyl group and a benzyl group;
- R⁸ is selected from the group consisting of a linear or branched C₁₋₄ alkyl group, a -(CH₂)ₙ-C₃₋₇ cycloalkyl group, a -CF₃ group, a linear or branched C₁₋₄ alkoxy group, a -C₁₋₄ alkyl-C₁₋₄ alkoxy group, a -(CH₂)ₙNR^{b}R^{c} group, a 4- to 6-membered heterocyclyl group containing at least one heteroatom selected from N, S and O, a (CH₂)ₙ-phenyl group, a -(CH₂)ₙ-(5- to 6-membered heteroaryl) group containing at least one heteroatom selected from N, S and O, wherein the phenyl, heteroaryl and heterocyclyl groups are optionally substituted by one or more substituents selected from the group consisting of a halogen atom, a linear or branched C₁₋₄ alkyl group, an acyl group and a linear or branched C₁₋₄ alkoxy group;
- R⁹ is selected from the group consisting of a hydrogen atom, a linear or branched C₁₋₄ alkyl group, a -CF₃ group, a C₃₋₇ cycloalkyl group and a halogen atom;
- R^{a} is selected from the group consisting of a linear or branched C₁₋₄ alkyl group, a linear or branched C₁₋₄ haloakyl group and a C₃₋₇ cycloalkyl group;
- R^{b} and R^{c} are independently selected from the group consisting of a hydrogen atom, a linear or branched C₁₋₄ alkyl group, a C₃₋₇ cycloalkyl group, a phenyl group, a 5- to 6-membered heteroaryl containing at least one heteroatom selected from N, S and O, a -C₁₋₄ alkyl-phenyl group and -C₁₋₄ alkyl-5- to 6-membered heteroaryl group; or R^{b} and R^{c} together with the nitrogen atom to which they are attached form a 3-to 7-membered N-containing heterocycyl group which is optionally substituted by one or more substituents selected by the group consisting of a halogen atom, a linear or branched C₁₋₄ alkyl group and a linear or branched C₁₋₄ alkoxy group; and
- n is an integer selected from 0 to 2.

The invention provides synthetic processes and intermediates described herein, which are useful for preparing compounds of the invention.

The invention also provides a pharmaceutical composition comprising a compound of the invention and a pharmaceutically-acceptable carrier. The invention further provides combinations comprising a compound of the invention and one or more other therapeutic agents and pharmaceutical compositions comprising such combinations.

The invention also provides a compound of the invention for use in the treatment of the human or animal body by therapy.

The invention also provides a compound of the invention for use in the treatment of a disease or condition susceptible to amelioration by CRTh2 antagonists, in a mammal, in particular wherein the pathological condition or disease is selected from asthma, chronic obstructive pulmonary disease (COPD), rhinitis, emphysema, allergic airway syndrome, allergic rhinobronchitis, psoriasis, dermatitis, allergic conjunctivitis, atopic dermatitis, pulmonary hypertension, interstitial lung fibrosis, allergy, psoriasis, adult respiratory distress syndrome (ARDS), myocardial infarction, aneurysm, stroke, cancer, wound healing, endotoxic shock, pain, inflammatory conditions, eosinophilic esophagitis, eosinophil-associated gastrointestinal disorders (EGID), idiopathic otitis, airway constriction, mucus secretion, nasal congestion, increased microvascular permeability and recruitment of eosinophils, urticaria, sinusitis, angioedema, anaphylaxia, chronic cough, Churg Strauss syndrome, arthritis, irritable bowel disease and inflammatory bowel disease (such as Crohn's disease and ulcerative colitis).

The invention also provides the use of a compound of the invention in the manufacture of a formulation or medicament for treating a disease or condition susceptible to amelioration by CRTh2 antagonists, in particular wherein the condition or disease is as described above.

The invention also provides a method of treating a disease or condition as described above; comprising such method administering to the mammal, a therapeutically effective amount of a compound of the invention. The invention further provides a method of treatment comprising administering a therapeutically effective amount of a combination of a compound of the invention together with one or more other therapeutic agents.

### DETAILED DESCRIPTION OF THE INVENTION

When describing the compounds, compositions, combinations and methods of the invention, the following terms have the following meanings, unless otherwise indicated.

As used herein the term C₁₋₄ alkyl embraces optionally substituted, linear or branched radicals having 1 to 4 carbon atoms. Examples include methyl, ethyl, n-propyl, i-propyl, n-butyl, sec-butyl or t-butyl. Optional substituents of a C₁₋₄alkyl group include a halogen atom and C₁₋₄alkoxy group.

As used herein, the term C₁₋₄ alkoxy (or alkyloxy) embraces optionally substituted, linear or branched oxy-containing radicals each having alkyl portions of 1 to 4 carbon atoms. Examples of C₁₋₄ alkoxy radicals include methoxy, ethoxy, n-propoxy, i-propoxy, n-butoxy, sec-butoxy and t-butoxy. Optional substituents of a C₁₋₄ alkoxy group include a halogen atom, a C₁₋₄ alkyl group and a C₁₋₄ alkoxy group.

As used herein, the term C₁-C₄ haloalkyl group is a linear or branched alkyl group, which is substituted by one or more, preferably 1, 2 or 3 halogen atoms. Examples indue CCl₃, CF₃ and CHF₂.

As used herein, the term C₃₋₇ cycloalkyl group embraces saturated carbocyclic radicals monocyclic or polycyclic ring having from 3 to 7 carbon atoms. Examples include cyclopropyl, cyclobutyl, cyclopentyl, cyclohexyl and cycloheptyl.

As used herein, the term C₆₋₁₀ aryl radical embraces typically a C₆-C₁₀ monocyclic or polycyclic aryl radical such as phenyl and naphthyl.

As used herein, the term 5- to 6- membered heteroaryl radical embraces typically a 5-to 6- membered ring system containing at least one heteroatom selected from O, S and N. Examples include pyridinyl, pyrazinyl, pyrimidinyl, pyridazinyl, furyl, oxadiazolyl, oxazolyl, isoxazolyl, imidazolyl, thiazolyl, thiadiazolyl, isothiazolyl, thienyl, pyrrolyl, pyridinyl, triazolyl, imidazolidinyl, and pyrazolyl.

As used herein, the term 5- to 6- membered N-containing heteroaryl radical embraces typically a 5- to 6- membered ring system containing at least one N atom and optionally further containing one or more heteroatoms selected from O, S and N. Examples include pyridinyl, pyrazinyl, pyrimidinyl, pyridazinyl, oxadiazolyl, oxazolyl, isoxazolyl, imidazolyl, thiazolyl, thiadiazolyl, isothiazolyl, pyrrolyl, pyridinyl, triazolyl, imidazolidinyl, and pyrazolyl.

As used herein, the term 4 to 6-membered heterocyclyl radical embraces typically a non-aromatic, saturated or unsaturated C₄-C₆ carbocyclic ring system in which at least one carbon is replaced by a heteroatom selected from N, O and S. Exampels include pyrrolinyl, piperazinyl, morpholinyl, thiomorpholinyl, pyrazolinyl and azetidinyl.

As used herein, the term 3 to 7-membered N-containing heterocyclyl radical embraces typically a non-aromatic, saturated or unsaturated C₃-C₇ carbocyclic ring system in which at least one carbon is replaced by a N atom and optionally additional carbon atoms are replaced by a heteroatom selected from N, O and S. Examples of 3 to 7-membered N-containing heterocyclic radicals include piperidinyl, pyrrolidinyl, pyrrolinyl, piperazinyl, morpholinyl, thiomorpholinyl, pyrazolinyl and azetidinyl.

As used herein, the term halogen atom embraces chlorine, fluorine, bromine or iodine atoms typically a fluorine, chlorine or bromine atom. The term halo when used as a prefix has the same meaning.

As used herein, the term acyl embraces a -C(O)C₁₋₄ alkyl group and a -C(O)cycloalkyl group.

The term "therapeutically effective amount" refers to an amount sufficient to effect treatment when administered to a patient in need of treatment.

The term "treatment" as used herein refers to the treatment of a disease or medical condition in a human patient which includes:
(a) preventing the disease or medical condition from occurring, i.e., prophylactic treatment of a patient;
(b) ameliorating the disease or medical condition, i.e., causing regression of the disease or medical condition in a patient;
(c) suppressing the disease or medical condition, i.e., slowing the development of the disease or medical condition in a patient; or
(d) alleviating the symptoms of the disease or medical condition in a patient.

The phrase "disease or condition associated with CRTh2 activity" includes all disease states and/or conditions that are acknowledged now, or that are found in the future, to be associated with CRTh2 activity. Such disease states include, but are not limited to, asthma, chronic obstructive pulmonary disease (COPD), rhinitis, emphysema, allergic airway syndrome, allergic rhinobronchitis, psoriasis, dermatitis, allergic conjunctivitis, atopic dermatitis, pulmonary hypertension, interstitial lung fibrosis, allergy, psoriasis, adult respiratory distress syndrome (ARDS), myocardial infarction, aneurysm, stroke, cancer, wound healing, endotoxic shock, pain, inflammatory conditions, eosinophilic esophagitis, eosinophil-associated gastrointestinal disorders (EGID), idiopathic otitis, airway constriction, mucus secretion, nasal congestion, increased microvascular permeability and recruitment of eosinophils, urticaria, sinusitis, angioedema, anaphylaxia, chronic cough, Churg Strauss syndrome, arthritis, irritable bowel disease and inflammatory bowel disease (such as Crohn's disease and ulcerative colitis). Preferably, such disease states are asthma or chronic obstructive pulmonary disease (COPD), rhinitis and atopic dermatitis.

The term "pharmaceutically-acceptable salt" refers to a salt prepared from a base or acid which is acceptable for administration to a patient, such as a mammal. Such salts can be derived from pharmaceutically-acceptable inorganic or organic bases and from pharmaceutically-acceptable inorganic or organic acids.

Salts derived from pharmaceutically-acceptable acids include acetic, benzenesulfonic, benzoic, camphosulfonic, citric, ethanesulfonic, formic, fumaric, gluconic, glutamic, hydrobromic, hydrochloric, hydrofluoric, lactic, maleic, malic, mandelic, methanesulfonic, mucic, nitric, pantothenic, phosphoric, succinic, sulfuric, tartaric, p-toluenesulfonic, xinafoic (1-hydroxy-2-naphthoic acid), napadisilic (1,5-naphthalenedisulfonic acid), triphenyl acetic and the like. Particularly preferred are salts derived from formic, fumaric, hydrobromic, hydrochloric, hydrofluoric, acetic, sulfuric, methanesulfonic, xinafoic, tartaric, maleic, succinic and napadisilic acids.

Salts derived from pharmaceutically-acceptable inorganic bases include aluminum, calcium, copper, ferric, ferrous, lithium, magnesium, manganic, manganous, potassium, sodium, zinc and the like. Particularly preferred are calcium, magnesium, potassium and sodium salts.

Salts derived from pharmaceutically-acceptable organic bases include salts of ammonia, primary, secondary and tertiary amines, including substituted amines, cyclic amines, naturally-occurring amines and the like, such as ammonia, arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

Other preferred salts according to the invention are quaternary ammonium compounds wherein an equivalent of an anion (X⁻) is associated with the positive charge of the N atom. X⁻ may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and p-toluenesulphonate. X⁻ is preferably an anion selected from chloride, bromide, iodide, sulphate, nitrate, acetate, maleate, oxalate, succinate or trifluoroacetate. More preferably X⁻ is chloride, bromide, trifluoroacetate or methanesulphonate.

As used herein, an N-oxide is formed from the tertiary basic amines or imines present in the molecule, using a convenient oxidising agent.

As used herein, the term solvate means a compound which further includes a stoichiometric or non-stoichiometric amount of solvent such as water, acetone, methanol, methanol, dichloromethane, 2-propanol, or the like, bound by non-covalent intermolecular forces. When the solvent is water, the term hydrate is used instead of solvate.

The invention also includes isotopically-labeled compounds of the invention, wherein one or more atoms is replaced by an atom having the same atomic number, but an atomic mass or mass number different from the atomic mass or mass number usually found in nature. Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P , and sulfur, such as ³⁵S. Certain isotopically-labeled compounds of the invention, for example, those incorporating a radioactive isotope, are useful in drug and/or substrate tissue distribution studies. The radioactive isotopes tritium, ³H, and carbon-14, ¹⁴C , are particularly useful for this purpose in view of their ease of incorporation and ready means of detection. Substitution with heavier isotopes such as deuterium, ²H, may afford certain therapeutic advantages resulting from greater metabolic stability, for example, increased in vivo half-life or reduced dosage requirements, and hence may be preferred in some circumstances. Substitution with positron emitting isotopes, such as ¹¹C, ¹⁸F, ¹⁵O and ¹³N, can be useful in Positron Emission Topography (PET) studies for examining substrate receptor occupancy.

Isotopically-labeled compounds of the invention can generally be prepared by conventional techniques known to those skilled in the art or by processes analogous to those described herein, using an appropriate isotopically-labeled reagent in place of the non-labeled reagent otherwise employed.

Preferred isotopically-labeled compounds include deuterated derivatives of the compounds of the invention. As used herein, the term deuterated derivative embraces compounds of the invention where in a particular position at least one hydrogen atom is replaced by deuterium. Deuterium (D or ²H) is a stable isotope of hydrogen which is present at a natural abundance of 0.015 molar %.

Hydrogen deuterium exchange (deuterium incorporation) is a chemical reaction in which a covalently bonded hydrogen atom is replaced by a deuterium atom. Said exchange (incorporation) reaction can be total or partial.

Typically, a deuterated derivative of a compound of the invention has an isotopic enrichment factor (ratio between the isotopic abundance and the natural abundance of that isotope, i.e. the percentage of incorporation of deuterium at a given position in a molecule in the place of hydrogen) for each deuterium present at a site designated as a potential site of deuteration on the compound of at least 3500 (52.5% deuterium incorporation).

In a preferred embodiment, the isotopic enrichment factor is at least 5000 (75% deuterium). In a more preferred embodiment, the isotopic enrichment factor is at least 6333.3 (95% deuterium incorporation). In a most preferred embodiment, the isotopic enrichment factor is at least 6633.3 (99.5% deuterium incorporation). It is understood that the isotopic enrichment factor of each deuterium present at a site designated as a site of deuteration is independent from the other deuteration sites.

The isotopic enrichment factor can be determined using conventional analytical methods known too en ordinary skilled in the art, including mass spectrometry (MS) and nuclear magnetic resonance (NMR).

The term "amino-protecting group" refers to a protecting group suitable for preventing undesired reactions at amino nitrogen. Representative amino-protecting groups include, but are not limited to, formyl; acyl groups, for example alkanoyl groups such as acetyl, benzoyl, pivaloyl or trifluoroacetyl; alkoxycarbonyl groups such as tert-butoxycarbonyl (Boc); arylmethoxycarbonyl groups such as benzyloxycarbonyl (Cbz) and 9-fluorenylmethoxycarbonyl (Fmoc); arylmethyl groups such as benzyl (Bn), trityl (Tr), and 1,1-di-(4'-methoxyphenyl)methyl and the like.

The term "hydroxy-protecting group" refers to a protecting group suitable for preventing undesired reactions at a hydroxy group. Representative hydroxy-protecting groups include, but are not limited to, alkyl groups, such as methyl, ethyl, and tert-butyl; acyl groups, for example alkanoyl groups, such as acetyl, pivaloyl or trifluoroacetyl; arylcarbonyl groups, such as benzoyl; alkoxycarbonyl groups such as tert-butoxycarbonyl (Boc); arylmethoxycarbonyl groups such as benzyloxycarbonyl (Cbz) and 9-fluorenylmethoxycarbonyl (Fmoc); arylmethyl groups, such as benzyl (Bn), p-methoxybenzyl (PMB), 9-fluorenylmethyl (Fm), and diphenylmethyl (benzhydryl, DPM); silyl groups, such as trimethylsilyl (TMS) and tert-butyldimethylsilyl (TBS); and the like.

The compounds of the invention may contain one or more chiral centers. Accordingly, the invention includes racemic mixtures, enantiomers, and mixtures enriched in one or more stereoisomer. The scope of the invention as described and claimed encompasses the racemic forms of the compounds as well as the individual enantiomers, diastereomers, and stereoisomer-enriched mixtures.

Typically, either X represents a nitrogen atom, Z represents a carbon atom and Y represents a nitrogen atom or a CR⁹ group; or X represents a carbon atom, Z represents a nitrogen atom and Y represents a CR⁹ group.

Typically, R¹ is selected from the group consisting of a hydrogen atom and methyl group.

Typically, R², R³, R⁴ and R⁵ are each independently selected from the group consisting of a hydrogen atom, a fluorine atom, a chlorine atom, a methyl group, a methoxy group, a -CN group, a cyclopropyl group, a -CF₃ group and a -CHF₂ group.

Typically at least one of R², R³, R⁴ and R³ represents a hydrogen atom, for example two or three of R², R³ R⁴ and R⁵ represent a hydrogen atom.

Preferably R⁵ represents a hydrogen atom.

Typically, G is selected from the group consisting of a phenyl group, a pyridyl group, a pyrimidinyl group, a pyridazinyl group and a pyrazolyl group, wherein said ring groups are optionally substituted by one or two substituents selected from the group consisting of a halogen atom, a methyl group, a -SO₂Me group, a -CHF² group and a -CF₃ group. In a preferred embodiment, G represents a phenyl group optionally substituted by one substitutent selected from the group consisting of a fluorine atom and a -CF₃ group.

Typically, the Z moiety and the -CH₂R₆ moiety are connected to the ring that G represents at positions which are ortho to each other.

Typically, R⁶ is selected from the group consisting of a -N(Ethyl)COR⁸ group, - N(Ethyl)SO₂R⁸ and a heteroaryl group selected from the group consisting of a triazolyl group and an isoxazolyl group, wherein the heteroaryl group is optionally substituted by one or more substituents selected from the group consisting of a methyl group and a cyclopropyl group.

Typically, R⁷ is selected from the group consisting of an ethyl group and a benzyl group.

Typically, R⁸ is selected from the group consisting of a linear or branched C₁₋₄ alkyl group, a -(CH₂)ₙ-C₃₋₇ cycloalkyl group, a linear or branched C₁₋₄ alkoxy group, a -C₁₋₂ alkyl-C₁₋₂ alkoxy group, a benzyl group, a -(CH₂)ₙNR^{b}R^{c} group, a 4- to 6-membered heterocyclyl group containing at least one heteroatom selected from N, S and O, and a -(CH₂)ₙ-(5- to 6-membered N-containing heteroaryl) group, wherein the benzyl, heterocyclyl and heteroaryl groups are optionally substituted by one or more substituents selected from the group consisting of a halogen atom, an acyl group and a methyl group.

Typically, R^{a} is selected from the group consisting of a linear or branched C₁₋₄ alkyl group, a linear or branched C₁₋₄ haloakyl group and a C₃₋₇ cycloalkyl group

Typically, R^{b} and R^{c} are independently selected from the group consisting of a hydrogen atom, a linear or branched C₁₋₄ alkyl group, a C₃₋₇ cycloalkyl group, a phenyl group and a benzyl group, preferably from a hydrogen atom, a linear or branched C₁₋₄ alkyl group and a benzyl group.

Typically, n is 0 or 1.

Typically, R⁹ is selected from the group consisting of a hydrogen atom and a methyl group.

In a preferred embodiment of the present invention:
- either X represents a nitrogen atom, Z represents a carbon atom and Y represents a nitrogen atom or a CR⁹ group; or X represents a carbon atom, Z represents a nitrogen atom and Y represents a CR⁹ group;
- R¹ is selected from the group consisting of a hydrogen atom and methyl group;
- R², R³, R⁴ and R⁵ are each independently selected from the group consisting of a hydrogen atom, a fluorine atom, a chlorine atom, a methyl group, a methoxy group, a -CN group, a cyclopropyl group, a -CF₃ group and a -CHF₂ group;
- G is selected from the group consisting of a phenyl group, a pyridyl group, a pyrimidinyl group, a pyridazinyl group and a pyrazolyl group, wherein said ring groups are optionally substituted by one or two substituents selected from the group consisting of a halogen atom, a methyl group and a -CF₃ group; preferably G represents a phenyl group optionally substituted by one substitutent selected from the group consisting of a fluorine atom and a -CF₃ group;
- R⁶ is selected from the group consisting of a -N(R⁷)COR⁸ group, a -N(R⁷)SO₂R⁸ group and a heteroaryl group selected from the group consisting of a triazolyl group, an isoxazolyl group and a pyrazolyl group, wherein the heteroaryl group is optionally substituted by one or more substituents selected from the group consisting of a methyl group and a cyclopropyl group;
- R⁷ is selected from the group consisting of an ethyl group and a benzyl group;
- R⁸ is selected from the group consisting of a linear or branched C₁₋₄ alkyl group, a -(CH₂)ₙ-C₃₋₇ cycloalkyl group, a linear or branched C₁₋₄ alkoxy group, a -C₁₋₂ alkyl-C₁₋₂ alkoxy group, a benzyl group, a -(CH₂)ₙNR^{b}R^{c} group, a 4- to 6-membered heterocyclyl group containing at least one heteroatom selected from N, S and O, and a -(CH₂)ₙ-(5- to 6-membered N-containing heteroaryl) group, wherein the benzyl, heterocyclyl and heteroaryl groups are optionally substituted by one or more substituents selected from the group consisting of a halogen atom, an acyl group and a methyl group, wherein R^{b} and R^{c} are preferably independently selected from a hydrogen atom, a linear or branched C₁₋₄ alkyl group, a C₃₋₇ cycloalkyl group, a phenyl group and a benzyl group;
- R⁹ is selected from the group consisting of a hydrogen atom and a methyl group; and
- n is 0 or 1.

In an alternative preferred embodiment of the present invention:
- either X represents a nitrogen atom, Z represents a carbon atom and Y represents a nitrogen atom or a -CR⁹ group; or X represents a carbon atom, Z represents a nitrogen atom and Y represents a -CR⁹ group;
- R¹ is selected from the group consisting of a hydrogen atom and methyl group;
- R², R³, R⁴ and R⁵ are each independently selected from the group consisting of a hydrogen atom, a fluorine atom, a chlorine atom, a methyl group, a methoxy group, a -CN group, a cyclopropyl group and a -CF₃ group;
- G is selected from the group consisting of a pyridyl group and a phenyl group optionally substituted by one substitutent selected from the group consisting of a fluorine atom and a -CF₃ group;
- R⁶ is selected from the group consisting of a -N(R⁷)C(O)R⁸ group, -N(R⁷)SO₂R⁸ and a heteroaryl group selected from the group consisting of a triazolyl group and an isoxazolyl group, wherein the heteroaryl group is optionally substituted by one or more substituents selected from the group consisting of a methyl group and a cyclopropyl group;
- R⁷ is an ethyl group;
- R⁸ is selected from the group consisting of a linear or branched C₁₋₄ alkyl group, a -(CH₂)ₙ-C₃₋₇ cycloalkyl group, a linear or branched C₁₋₄ alkoxy group, and a - (CH₂)ₙNR^{b}R^{c} group wherein R^{b} represents a hydrogen atom and R^{c} represents a benzyl group;
- R⁹ is selected from the group consisting of a hydrogen atom and a methyl group; and
- n is 0 or 1.

Particular individual compounds of the invention include:
{3-[2-{[(Cyclopropylcarbonyl)(ethyl)amino]methyl}-4-(trifluoromethyl}phenyl]-1H-indazol-1-yl}acetic acid;
{4-Chloro-3-[2-{[(cyclopropylcarbonyl)(ethyl)amino]methyl]-4-(trifluoromethyl)phenyl]-1H-indazol-1-yl}acetic acid;
{3-[2-{[(Cyclopropylcarbonyl)(ethyl)amino]methyl}-4-(trifluoromethyl)phenyl]-5-fluoro-1H-indazol-1-yl}acetic acid;
[4-Chloro-3-{2-{[(cyclopropylcarbonyl)(ethyl)amino]methyl}-4-fluorophenyl)-1H-indazol-1-yl]acetic acid;
(5-Chloro-3-[2-([(cyclopropylcarbonyl)(ethyl)amino]methyl)-4-(trifluoromethyl)phenyl]-1H-indazol-1-yl}acetic acid;
{3-[2-{[(Cyclopropylcarbonyl)(ethyl)amino]methyl}-4-(trifluoromethyl)phenyl]-6-fluoro-1H-indazol-1-yl}acetic acid;
{3-[2-{[(Cyclopropylcarbonyl)(ethyl)amino]methyl}-4-(trifluoromethyl)phenyl]-4-methoxy-1H-indazol-1-yl}acetic acid;
[3-(2-{[(Cyclopropylcarbonyl)(ethyl)amino]methyl}-4-fluorophenyl)-5-(trifluoromethyl)-1H-mdazol-1-yl]acetic acid;
2-{4-Chloro-3-[2-{[(cyclopropylcarbonyl)(ethyl)amino]methyl}-4-(trifluoromethyl)phenyl]-1H-indazol-1-yl}propanoic acid;
[3-(2-{[(Cyclopropylcarbonyl)(ethyl)amino]methyl}-4-fluorophenyl)-4-(trifluoromethyl)-1H-indazol-1-yl]acetic acid;
[5-Chloro-3-(2-{[(cyclopropylcarbonyl)(ethyl)amino]methyl}-4-fluorophenyl)-1H-indazol-1 -yl]acetic acid;
{4-Cyano-3-[2-{[(cyclopropylcarbonyl)(ethyl)amino]methyl}-4-(trifluoromethyl)phenyl]-1H-indazol-1-yl}acetic acid;
2-{5-Chloro-3-[2-{[(cyclopropylcarbonyl)(ethyl)amino]methyl}-4-(trifluoromethyl)phenyl]-1H-indazol-1-yl}propanoic acid;
{3-[2-{[(Cyclopropylcarbonyl)(ethyl)amino]methyl}-4-(trifluoromethyl)phenyl]-4-fluoro-1H-indazol-1-yl}acetic acid;
{3-[2-{[(Cyclopropylcarbonyl)(ethyl)amino]methyl}-4-(trifluoromethyl)phenyl]-5-methyl-1H-indazol-1-yl}acetic acid;
{3-[2-{[(Cyclopropylcarbonyl)(ethyl)amino]methyl}-4-(trifluoromethyl)phenyl]-4-methyl-1H-indazol-1-yl}acetic acid;
{4-Chloro-3-[2-{[(cyclopropylcarbonyl)(ethyl)amino]methyl)-4-(trifluoromethyl)phenyl]-5-fluoro-1H-indazol-1-yl}acetic acid;
[3-(2-{[(tert-Butoxycarbonyl)(ethyl)amino]methyl}-4-fluorophenyl)-5-chloro-1H-indazol-1-yl]acetic acid;
[5-Chloro-3-(2-([(cyclobutylcarbonyl)(ethyl)amino]methyl)-4-fluorophenyl)-1H-indazol-1-yl]acetic acid;
[5-Chloro-3-(2-{[ethyl(isobutyryl)amino]methyl}-4-fluorophenyl)-1H-indazol-1-yl]acetic acid;
[5-Chloro-3-{2-{[ethyl(methylsulfonyl)amino]methyl}-4-fluorophenyl)-1H-indazol-1-yl]acetic acid;
[3-(2-([Acetyl(ethyl)amino]methyl)-4-fluorophenyl)-5-chloro-1H-indazol-1-yl]acetic acid;
{3-[2-{[[(Benzylamino)carbonyl](ethyl)amino]methyl}-4-{trifluoromethyl)phenyl]-4-chloro-1H-indazol-1-yl}acetic acid;
[5-Chloro-3-(2-{[(cyclopropylcarbonyl)(ethyl)amino]methyl}phenyl)-1H-indazol-1-yl]acetic acid;
[5-Chloro-3-(2-{[(cyclopentylcarbonyl)(ethyl)amino]methyl}-4-fluorophenyl)-1H-indazol-1-yl]acetic acid;
[5-Chloro-3-(2-([(cyclopropylacetyl)(ethyl)amino]methyl)-4-fluorophenyl)-1H-indazol-1 - yl]acetic acid;
{5-Chloro-3-[2-{[(cyclopropylcarbonyl)(ethyl)amino]methyl)-4-(trifluoromethyl)phenyl]-4-fluoro-1H-indazol-1-yl}acetic acid;
{1-[2-{[(Cyclopropylcarbonyl)(ethyl)amino]methyl}-4-(trifluoromethyl)phenyl]-1H-indol-3-yl}acetic acid;
[1-(2-{[(Cyclopropylcarbonyl)(ethyl)amino]methyl}-4-fluorophenyl)-1H-indol-3-yl]acetic acid;
{1-[2-{[(Cyclopropylcarbonyl)(ethyl)amino]methyl}-4-(influoromethyl)phenyl]-6-fluoro-1H-indol-3-yl)acetic acid;
{3-[2-{[(Cyclopropylcarbonyl}(ethyl)amino]methyl}-4-(trifluoromethyl)phenyl]-1H-indol-1-yl}acetic acid;
{3-[2-{[(Cyclopropylcarbonyl)(ethyl)amino]methyl}-4-(trifluoromethyl)phenyl]-2-methyl-1H-indol-1-yl}acetic acid;
{3-[2-{[(Cyclopropylcarbonyl)(ethyl)amino]methyl}-4-(trifluoromethyl)phenyl]-5-fluoro-1H-indol-1-yl}acetic acid;
{4-Chloro-3-[2-{[(cyclopropylcarbonyl)(ethyl)amino]methyl}-4-(trifluoromethyl)phenyl]-1H-indol-1-yl}acetic acid;
{6-Chloro-3-[2-{[(cyclopropylcarbonyl)(ethyl)amino]methyl}-4-(trifluoromethyl)phenyl]-1H-indol-1-yl}acetic acid;
{5-Cyano-3-[2-{[(cyclopropylcarbonyl)(ethyl)amino]methyl}-4-(trifluoromethyl)phenyl]-1H-indol-1-yl}acetic acid;
[3-(2-{[(Cyclopropylcarbonyl)(ethyl)amino]methyl}-4-fluorophenyl)-1H-indol-1-yl]acetic acid;
{5-Cyclopropyl-3-[2-{[(cyclopropylcarbonyl)(ethyl)amino]methyl}-4-(trifluoromethyl)phenyl]-1H-indal-1-yl}acetic acid;
[3-(2-{[(Cyclopropylcarbonyl)(ethyl)amino]methyl}-4-fluorophenyl)-5-(trifluoromethyl)-1H-indol-1-yl]acetic acid;
{3-[2-{[(Cyclopropylcarbonyl)(ethyl)amino]methyl}-4-(trifluoromethyl)phenyl]-4-fluoro-1H-indol-1-yl}acetic acid;
2-(3-[2-([(Cyclopropylcarbonyl)(ethyl)amino]methyl)-4-(trifluoromethyl)phenyl]-5-fluoro-1H-indol-1-yl}propanoic acid;
[3-(2-{[(Cyclopropylcarbonyl)(ethyl)amino]methyl}-4-fluorophenyl)-5-fluoro-1H-indol-1 - yl]acetic acid;
(3-[2-{[[(Benzylamino)carbonyl](ethyl)amino]methyl)-4-(trifluoromethyl)phenyl]-5-fluoro-1H-indol-1-yl}acetic acid;
2-(4-Chloro-3-[2-([(cyclopropylcarbonyl)(ethyl)amino]methyl)-4-(trifluoromethyl)phenyl]-1H-indol-1-yl}propanoic acid;
[4-Chloro-3-{2-{[(cyclopropylcarbonyl)(ethyl)amino]methyl}-4-fluorophenyl)-1H-indol-1-yl]acetic acid;
{3-[2-[(3-Cyclopropyl-5-methyl-4H-1,2,4-triazol-4-yl)methyl]-4-(trifluoromethyl)phenyl]-5-fluoro-1H-indol-1-yl}acetic acid;
{4-Chloro-3-[2-{[(cyclopropylcarbonyl)(ethyl)amino]methyl}-4-(trifluoromethyl)phenyl]-5-fluoro-1H-indol-1-yl}acetic acid;
[4-Chloro-3-(2-{[(cyclopropylcarbonyl)(ethyl)amino]methyl}-4-fluorophenyl)-5-fluoro-1H-indol-1-yl]acetic acid;
[3-(3-{[(Cyclopropylcarbonyl}(ethyl)amino]methyl}pyridin-2-yl)-5-fluoro-1H-indol-1-yl]acetic acid;
{3-[2-{[(Cyclopropylcarbonyl)(ethyl)amino]methyl}-4-(trifluoromethyl)phenyl]-4,5-difluoro-1H-indol-1-yl}acetic acid;
[5-Chloro-3-(2-{[(cyclopropylcarbonyl)(ethyl)amino]methyl}-4-fluorophenyl}-1H-indol-1-yl]acetic acid;
[3-{2-{[(Cyclopropylcarbonyl)(ethyl)amino]methyl}-4-fluorophenyl)-4-fluoro-1H-indol-1 - yl]acetic acid;
{5-Chloro-3-[2-{[(cyclopropylcarbonyl)(ethyl)amino]methyl}-4-(trifluoromethyl)phenyl]-1H-indol-1-yl}acetic acid;
[3-(2-{[{Cyclopropylcarbonyl)(ethyl)amino]methyl}phenyl)-5-fluoro-1H-indol-1-yl]acetic acid;
[3-(3-{[(Cyclopropylcarbonyl)(ethyl)amino]methyl}pyridin-2-yl}-5-(trifluoromethyl)-1H-indal-1-yl]acatic acid; and
{3-[2-[(5-Cyclopropyl-3-methylisoxazol-4-yl)methyl]-4-(trifluoromethyl)phenyl]-5-fluoro-1H-indol-1-yl}acetic acid;
or a pharmaceutically acceptable salt, N-oxide, solvate, stereiosomer or deuterated derivative thereof.

Of particular interest are the compounds:
{3-[2-{[(Cyclopropylcarbonyl){ethyl)amino]methyl}-4-{trifluoromethyl)phenyl]-1H-indazol-1-yl}acetic acid;
{4-Chloro-3-[2-{[(cyclopropylcarbonyl)(ethyl)amino]methyl}-4-(trifluoromethyl)phenyl]-1H-indazol-1-yl}acetic acid;
{3-[2-{[(Cyclopropylcarbonyl)(ethyl)amino]methyl}-4-(trifluoromethyl)phenyl]-5-fluoro-1H-indazol-1-yl}acetic acid;
[4-Chloro-3-(2-{[(cyclopropylcarbonyl)(ethyl)amino]methyl}-4-fluorophenyl)-1H-indazol-1-yl]acetic acid;
{5-Chloro-3-[2-{[(cyclopropylcarbonyl)(ethyl)amino]methyl)-4-(trifluoromethyl)phenyl]-1H-indazol-1-yl}acetic acid;
{3-[2-{[(Cyclopropylcarbonyl)(ethyl)amino]methyl}-4-(trifluoromethyl)phenyl]-4-methoxy-1H-indazol-1-yl}acetic acid;
[3-(2-{[(Cyclopropylcarbonyl)(ethyl)amino]methyl}-4-fluorophenyl)-5-(trifluoromethyl)-1H-indazol-1-yl]acetic acid;
2-{4-Chloro-3-[2-{[(cyclopropylcarbonyl)(ethyl)amino]methyl}-4-(trifluoromethyl)phenyl]-1H-indazol-1-yl}propanoic acid;
[3-(2-{[(Cyclopropylcarbonyl)(ethyl)amino]methyl}-4-fluorophenyl)-4-(trifluoromethyl)-1H-indazol-1-yl]acetic acid;
[5-Chloro-3-(2-{[(cyclopropylcarbonyl)(ethyl)amino]methyl}-4-fluorophenyl)-1H-indazol-1-yl]acetic acid;
{4-Cyano-3-[2-{[(cyclopropylcarbonyl)(ethyl)amino]methyl}-4-(trifluoromethyl)phenyl]-1H-indazol-1-yl}acetic acid;
2-{5-Chloro-3-[2-{[(cyclopropylcarbonyl)(ethyl)amino]methyl}-4-(trifluoromethyl)phenyl]-1H-indazol-1-yl}propanoic acid;
{3-[2-{[(Cyclopropylcarbonyl)(ethyl)amino]methyl}-4-(trifluoromethyl)phenyl]-4-fluoro-1H-indazol-1-yl}acetic acid;
{3-[2-{[(Cyclopropylcarbonyl)(ethyl)amino]methyl}-4-(trifluoromethyl)phenyl]-5-methyl-1H-indazol-1-yl}acetic acid;
{3-[2-{[(Cyclopropylcarbonyl)(ethyl)amino]methyl}-4-(trifluoromethyl)phenyl]-4-methyl-1H-indazol-1-yl}acetic acid;
{4-Chloro-3-[2-{[(cyclopropylcarbonyl)(ethyl)amino]methyl}-4-(trifluoromethyl)phenyl]-5-fluoro-1H-indazol-1-yl}acetic acid;
[3-(2-{[(tert-Butoxycarbonyl)(ethyl)amino]methyl}-4-fluorophenyl)-5-chloro-1H-indazol-1-yl]acetic acid;
[5-Chloro-3-(2-([(cyclobutylcarbonyl)(ethyl)amino]methyl)-4-fluorophenyl)-1H-indazol-1-yl]acetic acid;
[5-Chloro-3-{2-{[ethyl(isobutyryl)amino]methyl}-4-fluorophenyl)-1H-indazol-1-yl]acetic acid;
[3-(2-{[Acetyl(ethyl)amino]methyl}-4-fluorophenyl)-5-chloro-1H-indazol-1-yl]acetic acid;
{3-[2-{[[(Benzylamino)carbonyl](ethyl)amino]methyl}-4-(trifluoromethyl)phenyl]-4-chloro-1H-indazol-1-yl}acetic acid;
[5-Chloro-3-(2-{[(cyclopropylcarbonyl)(ethyl)amino]methyl}phenyl)-1H-indazol-1-yl]acetic acid;
[5-Chloro-3-(2-{[(cyclopentylcarbonyl)(ethyl)amino]methyl}-4-fluorophenyl)-1H-indazol-1-yl]acetic acid;
[5-Chloro-3-(2-{[(cyclopropylacetyl)(ethyl)amino]methyl}-4-fluorophenyl)-1H-indazol-1-yl]acetic acid;
{5-Chloro-3-[2-{[(cyclopropylcarbonyl)(ethyl)amino]methyl}-4-(trifluoromethyl)phenyl]-4-fluoro-1H-indazol-1-yl]acetic acid;
{1-[2-{[(Cyclopropylcarbonyl)(ethyl)amino]methyl}-4-(trifluoromethyl)phenyl]-1H-indol-3-yl}acetic acid;
{1-[2-{[(Cyclopropylcarbonyl)(ethyl)amino]methyl}-4-{trifluoromethyl)phenyl]-6-fluoro-1H-indol-3-yl}acetic acid;
{3-[2-{[(Cyclopropylcarbonyl){ethyl)amino]methyl}-4-(trifluoromethyl)phenyl]-1H-indol-1-yl}acetic acid;
{3-[2-{[(cyclopropylcarbonyl)(ethyl)amino]methyl}-4-(trifluoromethyl)phenyl]-5-fluoro-1H-indol-1-yl}acetic acid;
{4-Chloro-3-[2-{[(cyclopropylcarbonyl)(ethyl)amino]methyl}-4-(trifluoromethyl)pheny]-1H-indol-1-yl}acetic acid;
[3-(2-{[(Cyclopropylcarbonyl)(ethyl)amino]methyl}-4-fluorophenyl)-1H-indol-1-yl]acetic acid;
[3-(2-([(Cyclopropylcarbonyl)(ethyl)amino]methyl)-4-fluorophenyl)-5-(trifluoromethyl)-1H-indol-1-yl]acetic acid;
{3-[2-{[(Cyclopropylcarbonyl)(ethyl)amino]methyl}-4-(trifluoromethyl)phenyl]-4-fluoro-1H-indol-1-yl}acetic acid;
[3-(2-{[(Cyclopropylcarbonyl)(ethyl)amino]methyl}-4-fluorophenyl)-5-fluoro-1H-indol-1-yl]acetic acid;
(3-[2-([[(Benzylamino)carbonyl](ethyl)amino]methyl)-4-(trifluoromethyl)phenyl]-5-fluoro-1H-indol-1-yl}acetic acid;
[4-Chloro-3-(2-{[(cyclopropylcarbonyl)(ethyl)amino]methyl}-4-fluorophenyl)-1H-indol-1-yl]acetic acid;
{3-[2-[(3-Cyclopropyl-5-methyl-4H-1,2,4-triazol-4-yl)methyl]-4-(trifluoromethyl)phenyl]-5-fluoro-1H-indol-1-yl}acetic acid;
{3-[2-{[(Cyclopropylcarbonyl)(ethyl)amino]methyl}-4-(trifluoromethyl)phenyl]-4,5-difluoro-1H-indol-1-yl}acetic acid;
[5-Chloro-3-(2-{[(cyclopropylcarbonyl)(ethyl)amino]methyl}-4-fluorophenyl)-1H-indol-1-yl]acetic acid;
[3-(2-{[(Cyclopropylcarbonyl)(ethyl)amino]methyl}-4-fluorophenyl)-4-fluoro-1H-indol-1-yl]acetic acid;
[3-(2-{[(Cyclopropylcarbonyl)(ethyl)amino]methyl}phenyl)-5-fluoro-1H-indol-1-yl]acetic acid; and
{(3-[2-[(5-Cyclopropyl-3-methylisoxazol-4-yl)methyl]-4-(trifluoromethyl)phenyl]-5-fluoro-1H-indol-1-yl}acetic acid.

### GENERAL SYNTHETIC PROCEDURES

The compounds of the invention can be prepared using the methods and procedures described herein, or using similar methods and procedures. It will be appreciated that where typical or preferred process conditions (i.e., reaction temperatures, times, mole ratios of reactants, solvents, pressures, etc.) are given; other process conditions can also be used unless otherwise stated. Optimum reaction conditions may vary with the particular reactants or solvent used, but such conditions can be determined by one skilled in the art by routine optimization procedures.

Additionally, as will be apparent to those skilled in the art, conventional protecting groups may be necessary to prevent certain functional groups from undergoing undesired reactions. The choice of a suitable protecting group for a particular functional group, as well as suitable conditions for protection and de-protection, are well known in the art. For example, numerous protecting groups, and their introduction and removal are described in T. W. Greene and G. M. Wuts, Protecting Groups in Organic Synthesis, Third Edition, Wiley, New York, 1999, and references cited herein.

Processes for preparing compounds of the invention are provided as further embodiments of the invention and are illustrated by the procedures below.

R¹, R², R³, R⁴, R⁵, R⁶, R⁷, R⁸, R⁹, R^{a}, R^{b}, R^{c}, G, X, Y and Z are defined according to claim 1.

Specific synthetic processes not covered by Schemes 1 - 11 are described in detail in the Experimental section.

In the case wherein R⁶ is a -N(R⁷)COR⁸ group or a -N(R⁷)SO₂R⁸ group, intermediates of the general formula (VIII) may be prepared following the synthetic scheme depicted in Scheme 1.

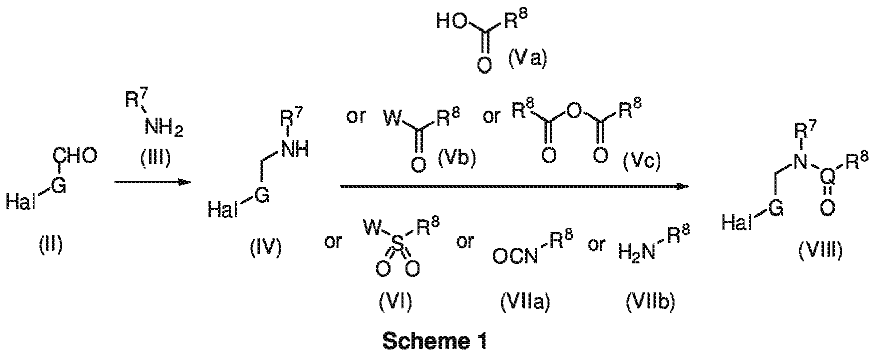

Compounds of general formula (IV) wherein Hal represents a halogen atom such as chlorine, bromine or iodine may be prepared by mixing compounds of formula (II) and an amine of formula (III) in the presence of a reducing agent such as sodium cyanoborohydride in the presence of an acid such as acetic acid in a solvent such as methanol, tetrahydrofuran or mixtures thereof at a temperature from 0 °C to the boiling point of the solvent.

Compounds of general formula (VIII) wherein Q represents a carbon or a S=O group may be prepared from compounds of formula (IV) and an appropriate acylating or sulfonylating agent such as:
- a carboxylic acid of formula (Va) in the presence of a peptide coupling reagent such as dicyclohexylcarbodiimide,
- an acyl halide of formula (Vb) wherein W represents a halogen atom such as fluorine, chlorine or bromine,
- an anhydride such of formula (Vc),
- a sulfonyl halide of formula (VI) wherein W is hereinbefore defined,
- an isocyanate of formula (VIIa) or
- an amine of formula (VIIb) in the presence of a phosgene equivalent such as phosgene, triphosgene or carbonyl diimidazole.

The reaction is carried out by mixing a compound of formula (IV) and a compound of formula (Va) - (VIIb) according to standard literature methods known to those skilled in the art of formation of amide, carbamates, ureas and sulfonamides to yield the compound of formula (VIII).

In the particular case wherein R⁶ is a substituted isoxazolyl group or a substituted pyrazolyl group, intermediates of the general formula (XII) and (XIV) wherein R¹⁰ and R¹¹ each independently represent a group selected from a -CF₃ group, a linear or branched C₁₋₄ alkyl group, a C₃₋₇ cycloalkyl group, a benzyl group, a phenyl group, a hydroxy group or a carboxy group and R¹² represents a -CF₃ group, a linear or branched C₁₋₄ alkyl group, a C₃₋₇ cycloalkyl group, a benzyl group or a phenyl group, may be prepared following the synthetic scheme depicted in Scheme 2.

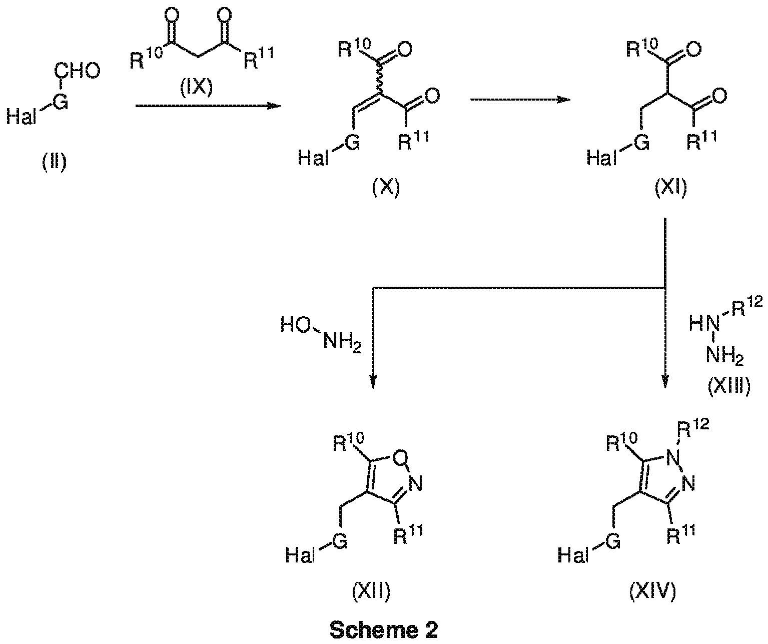

Compounds of general formula (X) wherein Hal represents a halogen atom such as chlorine, bromine or iodine may be prepared by mixing compounds of formula (II) and a dicarbonyl of formula (IX) in the presence of a base such as piperidine and acid such as acetic acid and in the presence or absence of a dessicant such as molecular sieves in a solvent such as ethanol at a temperature from ambient to the boiling point of the solvent to yield the compound of formula (X).

Compounds of general formula (XI) may be prepared by mixing a compound of formula (X) in the presence of a reducing agent such as zinc metal in the presence of an acid such as acetic acid at a temperature from 0 °C to the boiling point of the solvent.

Compounds of general formula (XII) may be prepared by mixing a compound of formula (XI) with hydroxylamine hydrochloride in a solvent such as ethanol at a temperature from 0 °C to the boiling point of the solvent.

Compounds of general formula (XIV) may be prepared by mixing a compound of formula (XI) with a hydrazine hydrochloride of formula (XIII) in the presence of an acid such as acetic acid at a temperature from 0 °C to the boiling point of the solvent.

In the particular case wherein R⁶ is a substituted triazolyl, intermediates of the general formula (XIX) wherein R¹⁰ and R¹¹ are as hereinbefore defined may be prepared following the synthetic scheme depicted in Scheme 3.

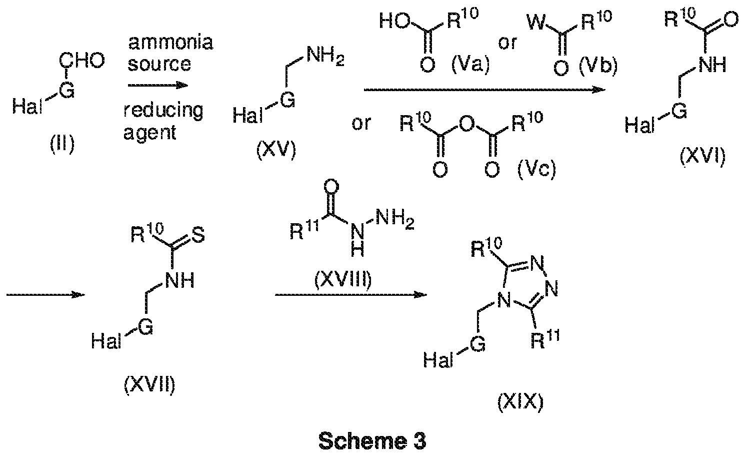

Compounds of general formula (XV) may be prepared from compounds of formula (II) and an ammonia source in the presence of a reducing agent.

In one example, the reaction is carried out by mixing a compound of formula (II) and an ammonia source such as ammonium acetate in the presence of a reducing agent such as sodium cyanoborohydride in the presence of an acid such as acetic acid in a solvent such as methanol, tetrahydrofuran or mixtures thereof at a temperature from 0 °C to the boiling point of the solvent to yield the compound of formula (XV).

In another example, the reaction is carried out by mixing a compound of formula (II) and hydroxylamine in a solvent such as methanol, followed by the subsequent addition of a reducing agent such as zinc metal in the presence of an acid such as acetic acid at a temperature from 0 °C to the boiling point of the solvent to yield the compound of formula (XV).

Compounds of general formula (XVI) may be prepared from compounds of formula (XV) and appropriate acylating agent such as:
- a carboxylic acid of formula (Va) wherein R¹⁰ is as hereinbefore defined in the presence of a peptide coupling reagent such as dicyclohexylcarbodiimide,
- an acyl halide of formula (Vb) wherein W represents a halogen atom such as fluorine, chlorine or bromine and wherein R¹⁰ is as hereinbefore defined or
- an anhydride such of formula (Vc) wherein R¹⁰ is as hereinbefore defined.

The reaction is carried out by mixing a compound of formula (XVI) and compound of formula (Va) - (Vc) according to standard literature methods known to those skilled in the art of formation of amides to yield the compound of formula (XVI).

Compounds of general formula (XVII) may be prepared by mixing a compound of formula (XVI) and a thionating agent such as phosphorus pentasulfide in the presence of a base such as diisopropylethylamine in a solvent such as tetrahydrofuran at a temperature from 0°C to the boiling point of the solvent to yield the compound of formula (XVII).

Compounds of general formula (XIX) may be prepared by mixing a compound of formula (XVII) and an acyl hydrazide of formula (XVIII) in the presence of a Lewis acid such as mercuric(II) acetate in a solvent such as acetonitrile at a temperature from 0°C to the boiling point of the solvent to yield the compound of formula (XIX).

Intermediates of the general formula (XXI) and (XXIII) may be prepared following the synthetic scheme depicted in Scheme 4.

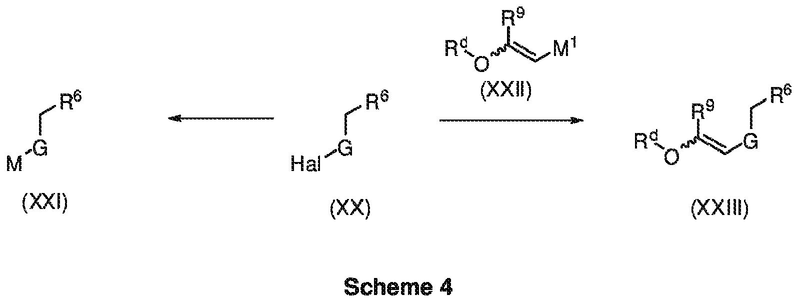

Compounds of general formula (XXI) wherein M represents lithium or salts or complexes of magnesium, zinc, copper, tin or boron may be prepared from compounds of formula (XX) wherein Hal represents a halogen atom such as chlorine, bromine or iodine.

The reaction is carried out by treating a compound of formula (XX) according to standard literature methods known to those skilled in the art of transmetalation. For example, in one instance the reaction can be carried out by treating a compound of formula (XX) with 4,4,4',4',5,5,5',5'-octamethyl-2,2'-bis(1,3,2-dioxaborolane) in the presence of a suitable transition metal catalyst such as [1,1'-bis(diphenylphosphino)-ferrocene]dichloropalladium(II) in the presence of a base such as potassium acetate in a solvent such as dioxane under an inert atmosphere such as argon at a temperature from 50 °C to the boiling point of the solvent to yield the compound of formula (XXI).

Compounds of general formula (XXIII) wherein R^{d} represents an alkyl group such as methyl, ethyl or propyl may be prepared from compounds of formula (XX) with an organometallic reagent of formula (XXII) wherein M¹ represents lithium or salts or complexes of magnesium, zinc, copper, tin or boron.

The reaction is carried out by treating a compound of formula (XX) according to standard literature methods known to those skilled in the art of carbon-carbon cross-coupling reactions. For example, in one instance the reaction can be carried out by treating a compound of formula (XX) with a vinyl stanane of formula (XXII) wherein M¹ is tributyl tin in the presence of a suitable transition metal catalyst such as tetrakis(triphenylphosphine)palladium in a solvent such as toluene under an inert atmosphere such as argon at a temperature from ambient to the boiling point of the solvent to yield the compound of formula (XXIII).

In the particular case where X is nitrogen, Y is a C(R⁹) group and Z is carbon, compounds of the general formula (Ia) may be prepared following the synthetic scheme depicted in Scheme 5.

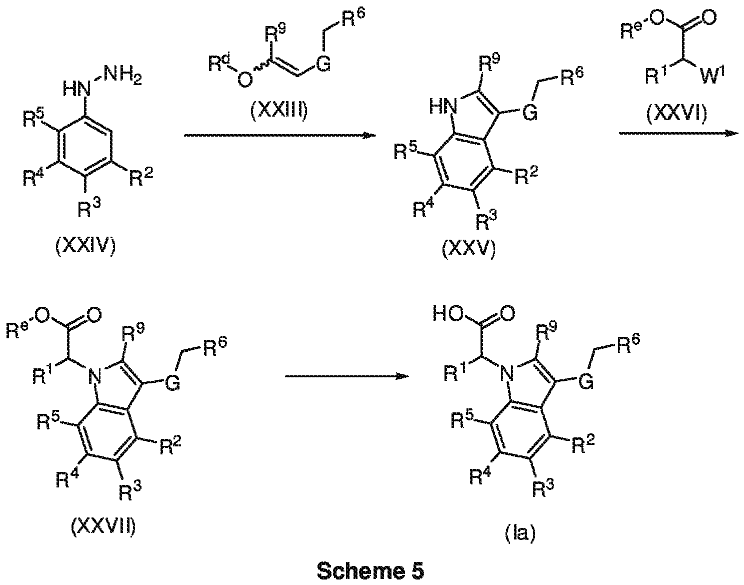

Compounds of general formula (XXV) wherein may be prepared by treating a hydrazine of formula (XXIV), either as the free base or as a hydrochloride salt, with a compound of formula (XXIII) in the presence of an acid such as hydrochloric acid or trifluoroacetic acid in a solvent such as ethanol or chloroform or mixtures thereof at a temperature from ambient to the boiling point of the solvent.

Compounds of general formula (XXVII) wherein R^{e} represents an alkyl group such as methyl, ethyl, propyl, tert-butyl or a benzyl group may be prepared from compounds of formula (XXV) and an alkylating agent of formula (XXVI) wherein W¹ represents a halogen atom such as chloride, bromine or iodine or a pseudohalogen such as paratoluenesulphonate.

The reaction is carried out by mixing a compound of formula (XXV) with an alkylating agent of formula (XXVI) in the presence of a base such as sodium hydride or caesium carbonate in a solvent such as dimethylformamide or acetonitrile at a temperature from 0 °C to the boiling point of the solvent to yield the compound of formula (XXVII). Compounds of general formula (Ia) may be prepared from compounds of formula (XXVII) by ester hydrolyses.

The reaction may be carried out by treating a compound of formula (XXVII) with an acid such as trifluoroacetic acid or hydrochloric acid and in a solvent such as chloroform, dichloromethane or dioxane at a temperature from 0 °C to the boiling point of the solvent to yield the compound of formula (Ia). Alternatively, the reaction may be carried out by treating a compound of formula (XXVII) with a base such as lithium hydroxide in a solvent such as water, tetrahydrofuran, acetonitrile or mixtures thereof, at a temperature from 0 °C to the boiling point of the solvent to yield the compound of formula (Ia).

Intermediates of formula (XXVII) may also be prepared following the synthetic scheme depicted in Scheme 6.

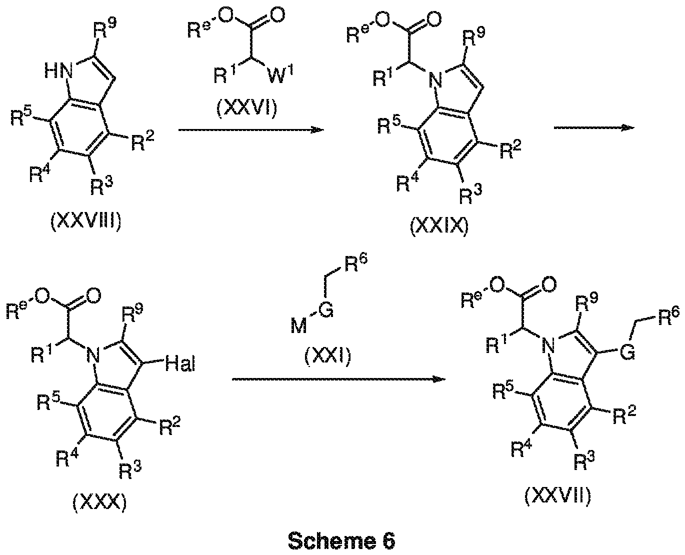

Compounds of general formula (XXIX) may be prepared by mixing a compound of formula (XXVIII) with an alkylating agent of formula (XXVI) in the presence of a base such as sodium hydride or caesium carbonate in a solvent such as dimethylformamide or acetonitrile at a temperature from 0 °C to the boiling point of the solvent. Compounds of general formula (XXX) may be prepared by mixing a compound of formula (XXIX) with a halogenating agent such as N-bromosuccinimide in a solvent such as dimethylformamide at a temperature from 0 °C to the boiling point of the solvent. Compounds of general formula (XXVII) may be prepared from compounds of formula (XXI) wherein Hal represents a halogen atom such as chlorine, bromine or iodine with an organometallic reagent of formula (XXI) wherein M is as hereinbefore defined.

The reaction is carried out by treating a compound of formula (XXX) with a compound of formula (XXI) according to standard literature methods known to those skilled in the art of carbon-carbon cross-coupling reactions. For example, in one instance the reaction can be carried out by treating a compound of formula (XXX) with a boronate ester of formula (XXI) wherein M is 4,4,5,5-tetramethyl-1,3,2-dioxaborolan-2-yl in the presence of a transition metal catalyst such as [1,1'-bis(diphenylphosphino)ferrocene]-dichloropalladium(II) in the presence of a base such as sodium carbonate or caesium carbonate in a solvent such as dioxane, toluene, ethanol, water or mixtures thereof at a temperature from 0 °C to the boiling point of the solvent under an inert atmosphere such as argon to yield the compound of formula (XXVII).

Intermediates of formula (XXV) may also be prepared following the synthetic scheme depicted in Scheme 7.

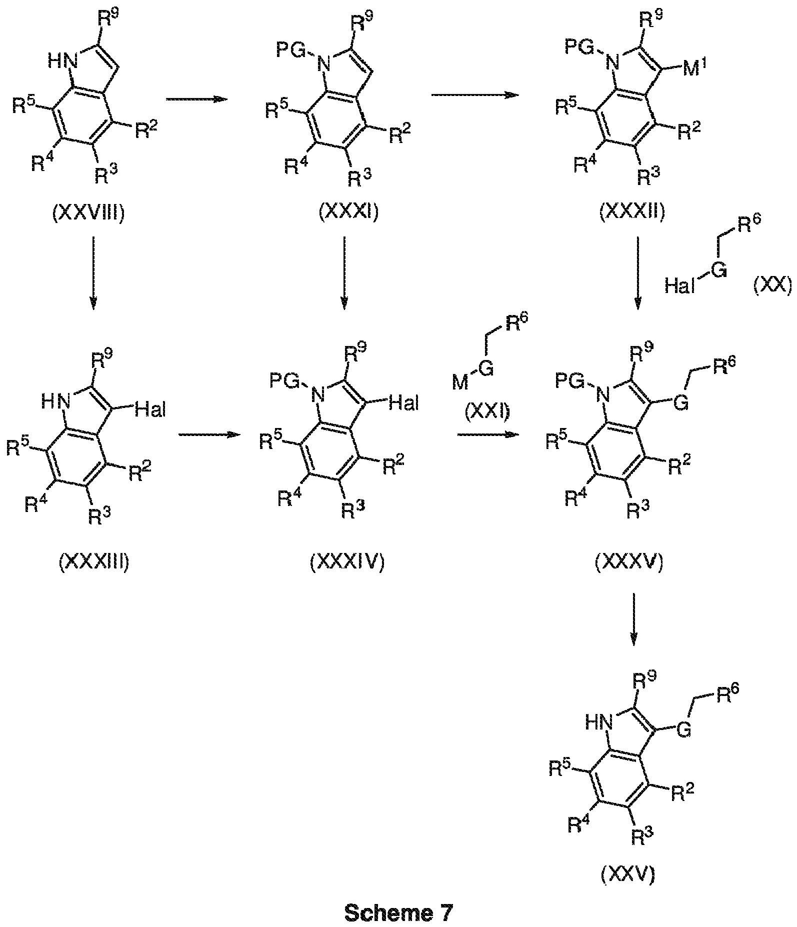

Compounds of general formula (XXXI) wherein PG represents an amino protecting group such as para-toluenesulfonyl or phenylsulfonyl may be prepared from compounds of formula (XXVIII) and a sulfonylating agent.

The reaction is carried out by mixing a compound of formula (XXVIII) with a sulfonylating agent in the presence of a base such as sodium hydride in a solvent such as dimethylformamide at a temperature from 0 °C to the boiling point of the solvent to yield the compound of formula (XXXI).

Compounds of general formula (XXXIII) may be prepared by mixing a compound of formula (XXVIII) with a halogenating agent such as N-bromosuccinimide in a solvent such as dimethylformamide at a temperature from 0 °C to the boiling point of the solvent.

Compounds of general formula (XXXIV) may be prepared by mixing a compound of formula (XXXIII) with a sulfonylating agent in the presence of a base such as sodium hydroxide in the presence of a phase-transfer catalyst such as tert-butylammonium hydrogen sulphate in a solvent such as dichloromethane at a temperature from ambient to the boiling point of the solvents.

Compounds of general formula (XXXIV) may also be prepared by mixing a compound of formula (XXXI) with a halogenating agent such as N-bromosuccinimide in a solvent such as dimethylformamide at a temperature from 0 °C to the boiling point of the solvent.

Compounds of general formula (XXXII) wherein M¹ represents a metal salt or complex such as mercury acetate or a boronic acid may be prepared from compounds of formula (XXXI).

In one example, the reaction is carried out by mixing a compound of formula (XXXI) with a metal salt such as mercuric acetate in the presence of a catalyst such as perchloric acid in a solvent such as acetic acid. Subsequently, the product is mixed with a boron-containing reagent such a borane in a solvent such as tetrahydrofuran at a temperature from 0 °C to the boiling point of the solvent to yield the compound of formula (XXXII).

Compounds of general formula (XXXV) may be prepared from compounds of formula (XXXIV) with an organometallic reagent of formula (XXI) wherein M is as hereinbefore defined.

The reaction is carried out by treating a compound of formula (XXXIV) according to standard literature methods known to those skilled in the art of carbon-carbon cross-coupling reactions. For example, in one instance the reaction can be carried out by treating a compound of formula (XXXIV) with a boronate ester of formula (XXI) wherein M is 4,4,5,5-tetramethyl-1,3,2-dioxaborolan-2-yl in the presence of a transition metal catalyst such as [1,1'-bis(diphenylphosphino)ferrocene]dichloropalladium(II) in the presence of a base such as or caesium carbonate in a solvent such as dioxane, toluene, ethanol, water or mixtures thereof at a temperature from 0 °C to the boiling point of the solvent under an inert atmosphere such as argon to yield the compound of formula (XXXV).

Compounds of general formula (XXXV) may also be prepared by treating a compound of formula (XXXII) with a compound of formula (XX) according to standard literature methods known to those skilled in the art of carbon-carbon cross-coupling reactions. For example, in one instance the reaction can be carried out by treating a compound of formula (XXXII) wherein M is boronic acid with a halide of formula (XX) in the presence of a transition metal catalyst such as [1,1'-bis(diphenylphosphino)ferrocene]dichloro-palladium(II) in the presence of a base such as caesium carbonate in a solvent such as dioxane or water or mixtures thereof at a temperature from ambient to the boiling point of the solvent under an inert atmosphere such as argon to yield the compound of formula (XXXV).

Compounds of general formula (XXV) may be prepared by treating a compound of formula (XXXV) with a base such as sodium hydroxide in a solvent such as ethanol or water or mixtures thereof at a temperature from ambient to the boiling point of the solvent.

In the particular case where X is carbon, Y is a C(R⁹) group and Z is nitrogen, compounds of the general formula (Ib) may be prepared following the synthetic scheme depicted in Scheme 8.

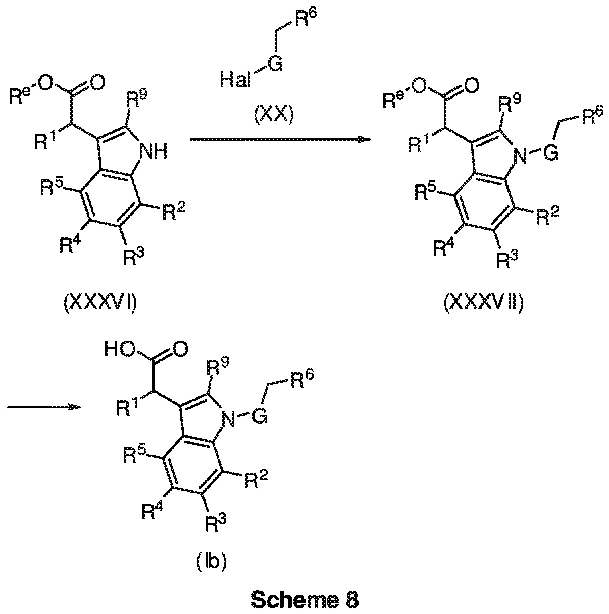

Indoles of general formula (XXXVI) may be prepared according to known literature procedures. The specific Preparations of this invention are detailed in the Experimental section.

Compounds of general formula (XXXVII) may be prepared by treating a compound of formula (XXXVI) with a compound of formula (XX) in the presence of a transition metal catalyst such as copper(I) oxide and ligand such as salicylaldoxime in the presence of a base such as caesium carbonate in a solvent such as acetonitrile at a temperature from 50 °C to 180 °C under an inert atmosphere such as argon.

Compounds of general formula (Ib) may be prepared from compounds of formula (XXXVII) by ester hydrolysis.

The reaction may be carried out by treating a compound of formula (XXXVII) with an acid such as trifluoroacetic acid or hydrochloric acid and in a solvent such as chloroform, dichloromethane or dioxane at a temperature from 0 °C to the boiling point of the solvent to yield the compound of formula (Ib). Alternatively, the reaction may be carried out by treating a compound of formula (XXXVII) with a base such as lithium hydroxide in a solvent such as water, tetrahydrofuran, acetonitrile or mixtures thereof, at a temperature from 0 °C to the boiling point of the solvent to yield the compound of formula (lb).

Compounds of the general formula (XXXVII) may also be prepared following the synthetic scheme depicted in Scheme 9.

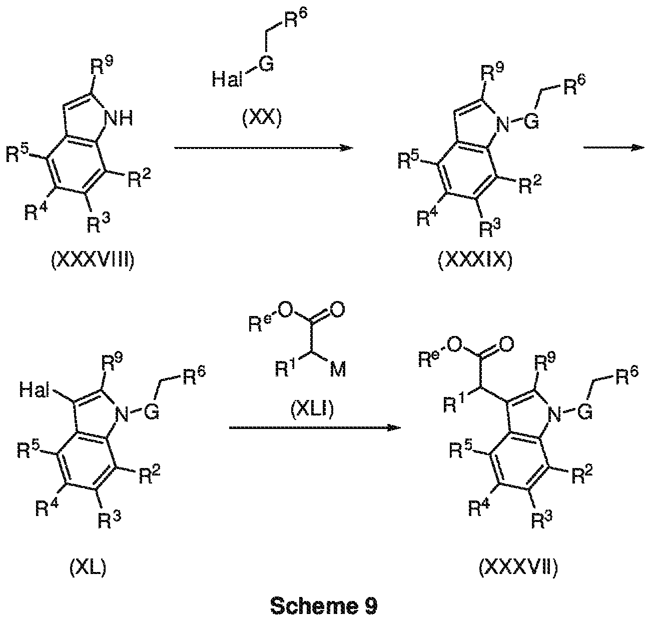

Compounds of general formula (XXXIX) may be by treating a compound of formula (XXXVIII) with a compound of formula (XX) in the presence of a transition metal catalyst such as copper(I) oxide and ligand such as salicylaldoxime in the presence of a base such as caesium carbonate in a solvent such as acetonitrile at a temperature from 50 °C to 180 °C under an inert atmosphere such as argon.

Compounds of general formula (XL) may be prepared by mixing a compound of formula (XXXIX) and a halogenating reagent such as N-bromosuccinimide, in a solvent such as ethyl acetate at a temperature from 0 °C to the boiling point of the solvent.

Compounds of general formula (XXXVII) by treating a compound of formula (XL) with a compound of formula (XLI), wherein M is as hereinbefore defined, in the presence of a transition metal catalyst such as bis(dibenzylideneacetone)palladium and ligand such as 1,2,3,4,5-pentaphenyl-1'-(di-tert-butylphosphino)ferrocene in a solvent such as ether at a temperature from 50 °C to 180 °C under an inert atmosphere such as argon.

In the particular case where X and Y are nitrogen and Z is carbon, compounds of the general formula (Ic) may be prepared following the synthetic scheme depicted in Scheme 10.

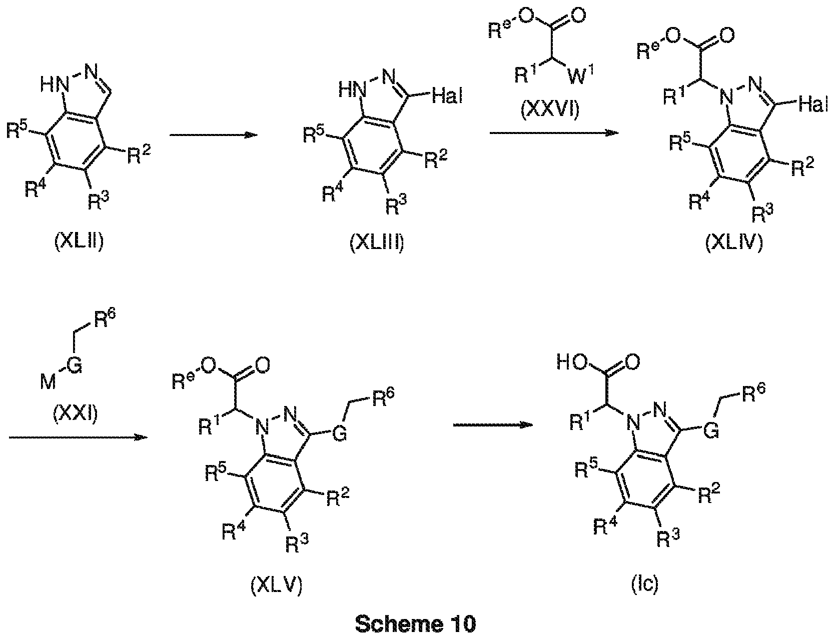

Indazoles of general formula (XLII) may be prepared according to known literature procedures. The specific Preparations of this invention are detailed in the Experimental section.

Compounds of general formula (XLIII) may be prepared by mixing a compound of formula (XLII) and a halogenating reagent such as iodine, in a solvent such as methanol in the presence of a base such as sodium hydroxide at a temperature from 0°C to the boiling point of the solvents.

Compounds of general formula (XLIV) may be prepared from compounds of formula (XLIII) and an alkylating agent of formula (XXVI) wherein W¹ is as hereinbefore defined. The reaction is carried out by mixing a compound of formula (XLIII) with an alkylating agent of formula (XXVI) in the presence of a base such as caesium carbonate in a solvent such as dimethylformamide at a temperature from 0 °C to the boiling point of the solvent to yield the compound of formula (XLIV).

Compounds of general formula (XLV) may be prepared by treating a compound of formula (XLIV) with a compound of formula (XXI) according to standard literature methods known to those skilled in the art of carbon-carbon cross-coupling reactions For example, in one instance the reaction can be carried out by treating a compound of formula (XLIV) with a boronate ester of formula (XXI) wherein M is 4,4,5,5-tetramethyl-1,3,2-dioxaborolan-2-yl in the presence of a transition metal catalyst such as [1,1'-bis(diphenylphosphino)ferrocene]dichloropalladium(II) in the presence of a base such as caesium carbonate in a solvent such as dioxane at a temperature from 0 °C to the boiling point of the solvent under an inert atmosphere such as argon to yield the compound of formula (XLV).

Compounds of general formula (Ic) may be prepared from compounds of formula (XLV) by ester hydrolysis.

The reaction may be carried out by treating a compound of formula (XLV) with an acid such as trifluoroacetic acid or hydrochloric acid and in a solvent such as chloroform, dichloromethane or dioxane at a temperature from 0 °C to the boiling point of the solvent to yield the compound of formula (Ic). Alternatively, the reaction may be carried out by treating a compound of formula (XLV) with a base such as lithium hydroxide in a solvent such as water, tetrahydrofuran, acetonitrile or mixtures thereof, at a temperature from 0 ºC to the boiling point of the solvent to yield the compound of formula (Ic).

In the particular case wherein R⁶ is a -N(R⁷)COR⁸ group or a -N(R⁷)SO₂R⁸ group, compounds of the general formula (Id) may also be prepared following the synthetic scheme depicted in Scheme 11.

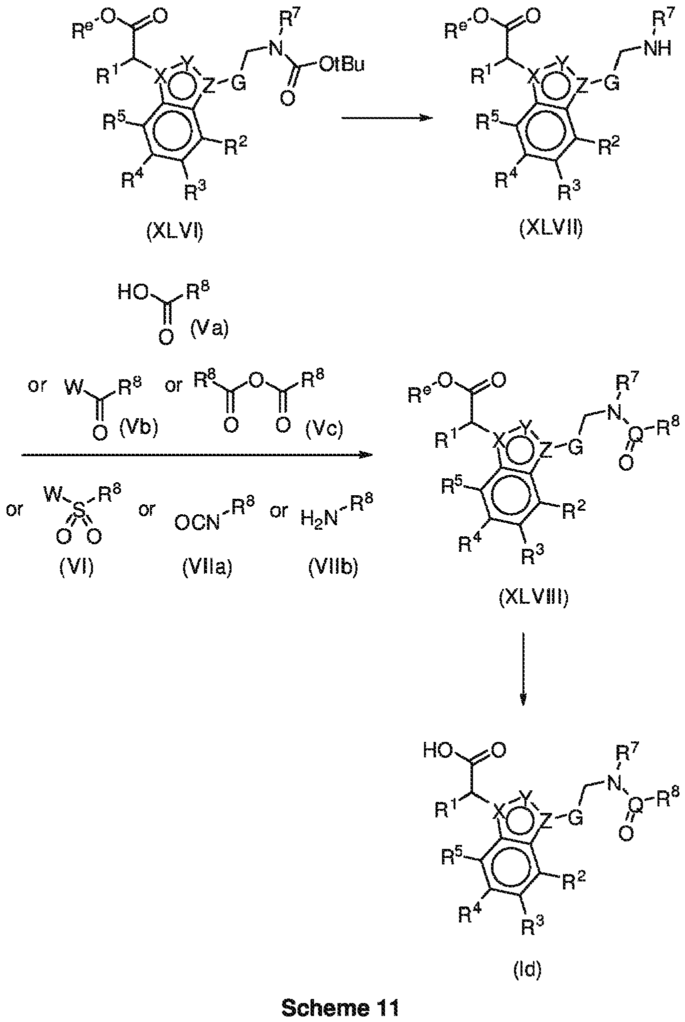

Compounds of general formula (XLVII) may be prepared from compounds of formula (XLVI) by amine deprotection.

The reaction is carried out by treating a compound of formula (XLVI) with an acid such as trifluoroacetic acid in a solvent such as dichloromethane at a temperature from 0 °C to the boiling point of the solvent to yield the compound of formula (XLVII).

Compounds of general formula (XLVIII) wherein R^{e} and Q are as hereinbefore defined may be prepared from compounds of formula (XLVII) and appropriate acylating or sulfonylating agent such as:
- a carboxylic acid of formula (Va) in the presence of a peptide coupling reagent such as dicyclohexylcarbodiimide,
- an acyl halide of formula (Vb) wherein W represents a halogen atom such as fluorine, chlorine or bromine,
- an anhydride such of formula (Vc), a sulfonyl halide of formula (VI) wherein W is hereinbefore defined,
- an isocyanate of formula (VIIa) or
- an amine of formula (VIIb) in the presence of a phosgene equivalent such as phosgene, triphosgene or carbonyl diimidazole.

The reaction is carried out by mixing a compound of formula (XLVII) and a compound of formula (Va) - (VIIb) according to standard literature methods known to those skilled in the art of formation of amide, carbamates, ureas and sulfonamides to yield the compound of formula (XLVIII).

Compounds of general formula (Id) may be prepared from compounds of formula (XLVIII) by ester hydrolysis.

The reaction may be carried out by treating a compound of formula (XLVIII) with an acid such as trifluoroacetic acid or hydrochloric acid and in a solvent such as chloroform, dichloromethane or dioxane at a temperature from 0 °C to the boiling point of the solvent to yield the compound of formula (Id). Alternatively, the reaction may be carried out by treating a compound of formula (XLVIII) with a base such as lithium hydroxide in a solvent such as water, tetrahydrofuran, acetonitrile or mixtures thereof, at a temperature from 0 ºC to the boiling point of the solvent to yield the compound of formula (Id).

### PREPARATION EXAMPLES

The syntheses of the compounds of the invention are illustrated by the following Examples (1 to 56) including Preparation Examples (1 to 156) which do not limit the scope of the invention in any way.

### General

Reagents, starting materials, and solvents were purchased from commercial suppliers and used as received. Concentration or evaporation refer to evaporation under vacuum using a Büchi rotatory evaporator.

Reaction products were purified, when necessary, by flash chromatography on silica gel (40-63 µm) with the solvent system indicated.

Purifications in reverse phase were made in a Biotage Isolera® automated purification system equipped with a C18 column and using a gradient of water-acetonitrile/MeOH (1:1) (0.1% v/v ammonium formate both phases) from 0% to 100% acetonitrile/MeOH (1:1) in 40 column volumes. The conditions "formic acid buffer" refer to the use of 0.1% v/v formic acid in both phases. The appropriate fractions were collected and the solvents evaporated under reduced pressure and/or liofilized.

Purifications in reverse phase were also made in a Biotage SP1® automated purification system equipped with a C18 column and using a gradient of water-acetonitrile/MeOH (1:1) (0.1% v/v ammonium formate both phases) from 0% to 100% acetonitrile/MeOH (1:1) in 80 column volumes. The conditions "formic acid buffer" refer to the use of 0.1% v/v formic acid in both phases. The appropriate fractions were collected and freeze dried.

Gas chromatography was performed using a Thermo Trace Ultra gas chromatograph, coupled to a DSQ mass detector. Injections were performed on a split/splitless injector and a HP-1MS was the capillary column. Mass spectra were obtained by electron impact ionisation at 70eV.

Preparative HPLC-MS were performed on a Waters instrument equipped with a 2767 injector/collector, a 2525 binary gradient pump, a 2996 PDA detector, a 515 pump as a make-up pump and a ZQ4000 Mass spectrometer detector.

The chromatographic separations were obtained using a Waters 2795 system equipped with a Symmetry C18 (2.1 x 50 mm, 3.5 µM) column for methods A, B and C and a Symmetry C18 (2.1 x 100 mm, 3.5 µM) for method D. The mobile phases were (B): formic acid (0.4 ml), ammonia (0.1 ml), methanol (500 ml) and acetonitrile (500 ml) and (A): formic acid (0.5 ml), ammonia (0.125 ml) and water (1000 ml) (A), the gradients are specified in the following table for each method used.

| Method | Run time | 0% B | 0 to 95% B | 95% B |
|---|---|---|---|---|
| A | 5 min | 0.2 min | 3 min | 0.8 min |
| B | 9 min | 0.5 min | 6.5 min | 1 min |
| c | 15 min | 0 min | 10.5 min | 1.5 min |
| D | 30 min | 0 min | 20 min | 4 min |

The flow rate was 0.8 ml/min for method A and 0.4 ml/min for method B, C and D. The injection volume was 5 microliter. A Waters 2996 diode array was used as a UV detector. Chromatograms were processed at 210 nM or 254 nM. Mass spectra of the chromatograms were acquired using positive and negative electrospray ionization in a Micromass ZMD or in a Waters ZQ detectors coupled to the HPLC.

The UPLC chromatographic separations were obtained using a Waters Acquity UPLC system coupled to a SQD mass spectrometer detector. The system was equipped with an ACQUITY UPLC BEH C-18 (2.1x50mm, 1.7 mm) column. The mobile phase was formic acid (0.4 ml), ammonia (0.1 ml), methanol (500 ml) and acetonitrile (500 ml) (B) and formic acid (0.5 ml), ammonia (0.125 ml) and water (1000 ml) (A). A gradient between 0 to 95% of B was used. The run time was 3 or 5 minutes The injection volume was 0.5 microliter. Chromatograms were processed at 210 nM or 254 nM. Mass spectra of the chromatograms were acquired using positive and negative electrospray ionization.

Mass Spectra (m/z) were recorded on a Micromass ZMD or in a Waters ZQ mass spectrometer using ESI ionization. The mass spectrum for the following compounds was not recorded: Preparations 25, 97 and 98.

¹H Nuclear Magnetic Resonance Spectra were recorded on a Varian Gemini-2000 spectrometer operating at a frequency of 300MHz for the ¹H spectra or in a Varian Mercury plus operating at a frequency of 400MHz for the ¹H spectra. Samples were dissolved in the specified deuterated solvent. Tetramethylsilane was used as reference. The H NMR spectrum for the following compounds was not recorded: Preparations 14, 15, 18, 19, 21, 27, 29, 35, 53, 58, 78, 79, 80, 82, 83, 84, 88, 90, 93, 99, 100, 101, 102, 103, 104, 107, 109, 110, 111, 112, 113, 114, 115, 116. 117, 118, 120, 121, 122, 124, 126, 127, 128, 129, 131, 132, 133, 134, 136, 140, 141, 143, 144, 147, 148, 150, 153, 155, 156 and Example 40.

Due to the presence of rotameric amides in the ¹H NMR spectra of many of the Preparations and Examples, in many cases only representative NMR peaks are quoted.

### PREPARATION 1

### Methyl 2-{[(4-methylphenyl)sulfonyl]oxy}propanoate

Methyl-2-hydroxypropanoate (1.0 g, 9.6 mmol) was dissolved in 12 ml acetonitrile. The mixture was cooled in an ice-bath and triethylamine (1.3 ml, 9.6 mmol) and trimethylamine hydrochloride (100 mg, 1.05 mmol) were added. A solution of p-toluensulfonic acid (1.83 g, 9.60 mmol) in 4 ml acetonitrile was added dropwise. The mixture was stirred at 0 °C for 1 h forming a precipitate. The solid was removed by filtration, washed with acetonitrile and the combined filtrate was evaporated under reduced pressure. The residue was dissolved in ether, washed sequentially with water, saturated sodium bicarbonate solution and brine, dried over sodium sulphate, filtered and evaporated under reduced pressure to give 2.0 g of the crude title compound as acolourless oil. Used as such without further purification. Purity 60%.
¹H NMR (300 MHz, CHLOROFORM-d) δ ppm 7.82 (d, *J*=8.24 Hz, 2 H), 7.35 (d, *J*=7.97 Hz, 2 H), 4.95 (q, *J*=6.96 Hz, 1 H), 3.67 (s, 3 H), 2.45 (s, 3 H), 1.51 (d, *J*=7.14 Hz, 3 H).
UPLC/MS (3 min) retention time 1.56 min.
LRMS: m/z 259 (M+1).

### PREPARATION 2

### Methyl (2R)-2-{[(4-methylphenyl)sulfonyl]oxy}propanoate

(R)-Methyl-2-hydroxypropanoate (2.5 g, 24 mmol) was treated with triethylamine (4.0 ml, 29 mmol), trimethylamine hydrochloride (0.25 g, 2.6 mmol) and p-toluensulfonic acid (4.5 g, 24 mmol) according to the method of Preparation 1 to give 5.2 g (20 mmol, 85%) of the title compound as a pale yellow oil, Purity 100%.
¹H NMR (400 MHz, CHLOROFORM-d) δ ppm 7.79 (2 H, d, *J*=8.2 Hz), 7.33 (2 H, d, *J*=8.2 Hz), 4.92 (1 H, q, *J*=7.0 Hz), 3.64 (3 H, s), 2.43 (3 H, s), 1.48 (3 H, d, *J*=7.0 Hz).
UPLC/MS (3 min) retention time 1.54 min.
LRMS:m/z276(M+18).

### PREPARATION 3

### N-[2-Bromo-5-(trifluoromethyl)benzyl]ethanamine

2-Bromo-5-(trifluoromethyl)benzaldehyde (20.0 g, 79.0 mmol) was dissolved in 300 ml methanol. Ethylamine (2.0 M solution in tetrahydrofuran, 60 ml, 120 mmol) was added followed by the portion-wise addition of sodium cyanoborohydride (7.50 g, 120 mmol). Acetic acid (8.0 ml, 140 mmol) was added drop-wise and the reaction mixture was stirred overnight at room temperature. The organics were evaporated under reduced pressure and the mixture was partitioned between dichloromethane and water. The aqueous phase was extracted twice with dichloromethane and the combined organics were washed with 4% w/v sodium bicarbonate solution, dried over sodium sulphate, filtered and evaporated under reduced pressure to give 15.0 g (53.2 mmol, 67%) of the title compound as an oil. Purity 100%.
¹H NMR (300 MHz, CHLOROFORM-*d*) δ ppm 7.70 (1 H, s), 7.66 (1 H, d, *J*=8.2 Hz), 7.37 (1 H, dd, *J*=8.2, 1.4 Hz), 3.91 (2 H, s), 2.71 (2 H, q, *J*=7.1 Hz), 1.68 (1 H, br. s.), 1.17 (3 H, t, *J*=7.1 Hz).
UPLC/MS (3 min) retention time 0.95 min.
LRMS:m/z282,284(M+1).

### PREPARATION 4

### N-[2-Bromo-5-(trifluoromethyl)benzyl]-N-ethylcyclopropanecarboxamide

The title compound of Preparation 3 (6.0 g, 18.1 mmol) was dissolved in 50 ml dichloromethane and triethylamine (3.0 ml, 21.5 mmol). Cyclopropanecarbonyl chloride (2.30 ml, 25.3 mmol) was added drop-wise over 15 min and the mixture was stirred at room temperature for 2 h. The mixture was diluted with dichloromethane and was washed sequentially with 4% w/v sodium bicarbonate solution, water and brine. The organics were dried over sodium sulphate, filtered and evaporated under reduced pressure.The residue was purified using the Isolera Purification System (ethyl acetate-hexane gradient, 0:100 rising to 30:70) to give 5.50 g (15.7 mmol, 87%) of the title compound as a colourless oil. Purity 100%.
¹H NMR (300 MHz, CHLOROFORM-d) 60:40 mixture of two rotamers. Major rotamer δ ppm 7.57 - 7.80 (1 H, m), 7.30 - 7.54 (2 H, m), 4.71 (2 H, s), 3.42 - 3.63 (2 H, m), 1.76 - 1.96 (1 H, m), 1.28 (3 H, t, *J*=7.1 Hz), 0.98 - 1.11 (2 H, m), 0.79 - 0.92 (2 H, m).
Minor rotamer δ ppm 7.57 - 7.80 (1 H, m), 7.30 - 7.54 (2 H, m), 4.74 (2 H, s), 3.42 - 3.63 (2 H, m), 1.36 - 1.48 (1 H, m), 1.16 (3 H, t, *J*=7.0 Hz), 0.98 - 1.11 (2 H, m), 0.63 - 0.76 (2 H, m).
UPLC/MS (3 min) retention time 1.90 min.
LRMS: m/z 350, 352 (M+1).

### PREPARATION 5

### N'-Benzyl-N-[2-bromo-5-(trifluoremethyl)benzyl]-N-ethylurea

The title compound of Preparation 3 (0.5 g, 1.77 mmol) was dissolved in 7 ml dichloromethane and the mixtire was cooled in an ice bath. Diisopropylethylamine (0.68 ml, 3.9 mmol) and phosgene (20% solution in toluene, 1.13 ml, 2.1 mmol) were added and the mixture was stirred for 2 h in the ice-bath. Benzylamine (0.29 ml, 2.66 mmol) was added and the mixture was stirred for 15 min. Triethylamine (0.35 ml, 2.5 mmol) was added and the mixture was stirred for 90 min at room temperature. The mixture was partitioned between dichloromethane and water. The aqueous phase was extracted twice with dichloromethane and the combined organics were washed with brine, dried over sodium sulphate, filtered and evaporated under reduced pressure. The residue was purified using the Isolera Purification System (ethyl acetate-hexane gradient, 0:100 rising to 50:50) to give 0.64 g (1.54 mmol, 87%) of the title compound. Purity 100%.
¹H NMR (400 MHz, CHLOROFORM-d) δ 7.67 (d, J = 8.1 Hz, 1H), 7.50 (s, 1H), 7.39 (d, J = 8.3 Hz, 1H), 7.36 - 7.30 (m, 2H), 7.28 (m, 3H), 4.73 (s, 1 H), 4.60 (s, 2H), 4.48 (d, J = 5.4 Hz, 2H), 3.34 (q, J = 7.1 Hz, 2H), 1.19 (t, J = 7.1 Hz, 3H).
UPLC/MS (3 min) retention time 1.95 min.
LRMS: m/z 415, 417 (M+1).

### PREPARATION 6

### N-(2-Bromo-5-fluorobenzyl)ethanamine

2-Bromo-5-fluorobenzaldehyde (4.88 g, 24.0 mmol) was treated with ethylamine (2 M in tetrahydrofuran, 18 ml, 36 mmol), sodium cyanoborohydride (2.27 g, 36.1 mmol) and acetic acid (2.06 ml, 36.0 mmol) according to the method of Preparation 3 to give 4.00 g (17.2 mmol, 72%) of the title compound as a colourless oil. Purity 100%.
¹H NMR (300 MHz, CHLOROFORM-*d*) δ ppm 7.49 (dd, *J*=8.65, 5.36 Hz, 1 H), 7.19 (dd, *J*=9.34, 3.02 Hz, 1H), 6.86 (td, *J*=8.24, 3.02 Hz, 1H), 3.85 (s, 2 H), 2.70 (q, *J*=7.14 Hz, 2 H), 1.17 (t, *J*=7.14 Hz, 3 H).
UPLC/MS (3 min) retention time 0.69 min.
LRMS: m/z 232, 234 (M+1).

### PREPARATION

### N-(2-Brome-5-fluorobenzyl)-N-ethylcyclepropanecarboxamide

The title compound of Preparation 6 (3.90 g, 16.8 mmol) was treated with triethylamine (2.8 ml, 20 mmol) and cyclopropanecarbonyl chloride (2.20 ml, 24.2 mmol) according to the method of Preparation 4. The resulting residue was purified using the Isolera Purification System (ether-hexane gradient, 0:100 rising to 30:70) to give 2.80 g (9.33 mmol, 55%) of the title compound as a colourless oil. Purity 90%.
Partial ¹H NMR (300 MHz, CHLOROFORM-d) 65:35 mixture of two rotamers.
Major rotamer δ ppm 4.66 (s, 2 H).
Minor rotamer δ ppm 4.67 (s, 2 H).
UPLC/MS (3 min) retention time 1.78 min.
LRMS: m/z 300, 302 (M+1).

### PREPARATION 8

### tert-Butyl (2-bromo-5-fluorobenzyl)ethylcarbamate

The title compound of Preparation 6 (4.00 g, 17.2 mmol) was dissolved in 50 ml tetrahydrofuran. Di(tert-butyl)carbonate (4.51 g, 20.6 mmol) was added and the mixture was stirred for 3 h at room temperature. The mixture was concentrated under reduced pressure and the residue was re-dissolved in ethyl acetate. The organics were washed with water and brine, dried over sodium sulphate, filtered and evaporated under reduced pressure. The residue was purified using the Isolera Purification System (ether-hexane gradient, 0:100 rising to 10:90) to give 5.50 g (16.5 mmol, 96%) of the title compound as a colourless oil. Purity 100%.
Partial ¹H NMR (300 MHz, CHLOROFORM-d) 60:40 mixture of two rotamers.
Major rotamer δ ppm 4.48 (br. s., 2 H), 3.26 (br. s., 2 H).
Minor rotamer δ ppm 4.43 (br. s., 2 H), 3.34 (br. s., 2 H).
UPLC/MS (3 min) retention time 2.15 min.
LRMS: m/z 276, 278 (M-55).

### PREPARATION 9

### tert-Butyl ethyl[5-fluoro-2-(4,4,5,5-tetramethyl-1,3,2-dioxaborolan-2-yl)benzyl]carbamate

The title compound of Preparation 8 (4.50 g, 15.0 mmol) and 4,4,4',4',5,5,5',5'-octamethyl-2,2'-bis(1,3,2-dioxaborolane) (4.47 g, 17.6 mmol) were dissolved in 30 ml dioxane in a Schlenk vessel. Potassium acetate (4.0 g, 40.6 mmol) was added and the reaction mixture was submitted to three vacuum-argon cycles. [1,1'-bis(diphenylphosphino)ferrocene]dichloropalladium(II), complex with dichloromethane (0.60 g, 0.68 mmol) was added and the mixture was further submitted to three vacuum-argon cycles. The reaction vessel was sealed and the mixture was stirred at 100 °C for 3 h. The mixture was cooled to room temperature, diluted with ethyl acetate and filtered through celite. The residue was evaporated under reduced pressure and purified using the Isolera Purification System (ether-hexane gradient, 0:100 rising to 20:80) to give 3.20 g (8.44 mmol, 56%) of the title compound as a colourless oil. Purity 100%.
Martial ¹H NMR (300 MHz, CHLOROFORM-d) 60:40 mixture of two rotamers.
Major rotamer δ ppm 4.77 (s, 2 H), 3.18 (m, 2 H)
Minor rotamer δ ppm 4.72 (s, 2 H), 3.26 (m, 2 H).
UPLC/MS (3 min) retention time 2.33 min.
LRMS: m/z 380 (M+1).

### PREPARATION 10

### N-Ethyl-N-[2-(4,4,5,5-tetramethyl-1,3,2-dioxaborolan-2-yl)-5-(trifluoromethyl)benzyl]cyclopropanecarboxamide

The title compound of Preparation 4 (3.76 g, 10.7 mmol) was treated with 4,4,4',4',5,5,5',5'-octamethyl-2,2'-bis(1,3,2-dioxaborolane) (2.54 g, 14.0 mmol), potassium acetate (3.16 g, 32.2 mmol) and [1,1'-bis(diphenylphosphino)-ferrocene]dichloropalladium(II), complex with dichloromethane (0.47 g, 0.54 mmol) according to the method of Preparation 9. The resulting residue was purified using the Isolera Purification System (ether-hexane gradient, 0:100 rising to 100:0) to give 3.20 g (8.06 mmol, 75%) of the title compound as a colourless oil. Purity 90%.
Martial ¹H NMR (300 MHz, CHLOROFORM-d) 55:45 mixture of two rotamers.
Major rotamer δ ppm 4.99 (s, 2 H), 3.49 - 3.42 (m, 2 H).
Minor rotamer δ ppm 5.02 (s, 2 H), 3.49 - 3.42 (m, 2 H).
UPLC/MS (3 min) retention time 2.19 min.
LRMS: m/z 398 (M+1).

### PREPARATION 11

### N-Ethyl-N-[5-fluoro-2-(4,4,5,5-tetramethyl-1,3,2-dioxaborolan-2-yl)benzyl]cyclopropanecarboxamide

The title compound of Preparation 7 (1.50 g, 5.00 mmol) was treated with 4,4,4',4',5,5,5',5'-octamethyl-2,2'-bis(1,3,2-dioxaborolane) (1.65 g, 6.50 mmol), potassium acetate (1.47 g, 15.0 mmol) and [1,1'-bis(diphenylphosphino)-ferrocene]dichloropalladium(II), complex with dichloromethane (0.20 g, 0.24 mmol) according to the method of Preparation 9. The resulting residue was purified using the Isolera Purification System (ether-hexane gradient, 0:100 rising to 0:100) to give 1.2 g (3.46 mmol, 69%) of the title compound as a colourless oil.
¹H NMR (300 MHz, CHLOROFORM-d) δ ppm 8.06 - 8.48 (m, 1 H), 7.58 - 7.78 (m, 1 H), 7.33 - 7.51 (m, 1 H), 5.26 - 5.56 (m, 2 H), 3.69 - 4.07 (m, 2 H), 2.38 - 2.59 (m, 1 H), 1.72 - 1.90 (m, 12 H), 1.36 - 1.74 (m, 5 H), 1.01 - 1.36 (m,2H).
UPLC/MS (3 min) retention time 2.08 min.
LRMS: m/z 348 (M+1).

### PREPARATION12

### N-Ethyl-N-[2-(4,4,5,5-tetramethyl-1,3,2-dioxaborolan-2-yl)benzyl]cyclopropanecarboxamide

N-(2-(4,4,5,5-Tetramethyl-1,3,2-dioxaborolan-2-yl)benzyl)ethanamine (70 mg, 0.27 mmol) and triethylamine (44 µl, 0.32 mmol) were dissolved in 2 ml dichloromethane and the mixture was cooled in an ice-bath. A solution of cyclopropanecarbonyl chloride (29 µl, 0.32 mmol) dissolved in 1 ml dichloromethane was added drop-wise and the mixture was stirred at room temperature for 1 h. The mixture was washed with water and the organics were dried over sodium sulphate, filtered and evaporated under reduced pressure to give 82 mg (0.25 mmol, 91%) of the title compound as a colourless oil. Purity 100%.
Partial ¹H NMR (300 MHz, CHLOROFORM-d) 55:45 mixture of two rotamers.
Major rotamer δ ppm 5.05 (s, 2 H), 1.19 (3 H, t, J=7.0 Hz).
Minor rotamer δ ppm 5.02 (s, 2 H), 1.27 (3 H, t, J=7.1 Hz).
UPLC/MS (3 min) retention time 2.05 min.
LRMS: m/z 330 (M+1).

### PREPARATION 13

### N'-Benzyl-N-ethyl-N-[2-(4,4,5,5-tetramethyl-1,3,2-dioxaborolan-2-yl)-5-(trifluoromethyl)benzyl]urea

The title compound of Preparation 5 (400 mg, 0.96 mmol) was treated with 4,4,4',4',5,5,5',5'-octamethyl-2,2'-bis(1,3,2-dioxaborolane) (490 mg, 1.9 mmol), potassium acetate (280 mg, 2.9 mmol) and [1,1'-bis(diphenylphosphino)ferrocene]-dichloropalladium(II), complex with dichloromethane (43 mg, 0.05 mmol) according to the method of Preparation 9. The resulting residue was purified using the lsolera Purification System (ether-hexane gradient, 0:100 rising to 100:0) to give 204 mg (0.44 mmol, 46%) of the title compound as a colourless oil. Purity 100%.
¹H NMR (400 MHz, CHLOROFORM-d) δ 7.91 (d, J = 7.6 Hz, 1H), 7.60 (s, 1H), 7.49 (d, J = 6.7 Hz, 1H), 7.33 - 7.26 (m, 3H), 7.24 - 7.15 (m, 2H), 4.81 (s, 2H), 4.45 (d, J = 5.6 Hz, 2H), 3.40 (d, J = 7.1 Hz, 2H), 1.27 (s, 4H), 1.24 (s, 8H), 1.18 (t, J = 7.1 Hz, 3H).
UPLC/MS (3 min) retention time 2.16 min.
LRMS: m/z 463 (M+1).

### PREPARATION 14

### N-[2-[(Z)-2-Ethoxyvinyl]-5-(trifluoromethyl)benzyl]-N-ethylcyclopropanecarboxamide

The title compound of Preparation 4 (0.30 g, 0.86 mmol), (Z)-tributyl(2-ethoxyvinyl)stannane (0.34 g, 0.94 mmol) and tetrakis(triphenylphosphine)palladium (0.050 g, 0.04 mmol) were dissolved in 10 ml toluene. The mixture was submitted to three vacuum-argon cycles and was then was stirred at reflux overnight. The mixture filtered and was evaporated to dryness and the residue was partitioned between ethyl acetate and water. The organic phase was washed sequentially with water and was dried over sodium sulphate, filtered and evaporated under reduced pressure. The residue was purified by flash chromatography (ethyl acetate-hexane gradient, 0:100 rising to 30:70) to give 150 mg (0.44 mmol, 51%) of the title compound as a colourless oil. Purity 98%.
UPLC/MS (3 min) retention time 1.95 min.
LRMS: m/z 342 (M+1).

### PREPARATION 15

### N-Benzyl-N-[2-[(Z)-2-ethoxyvinyl]-5-(trifluoromethyl)benzy]-N-ethylurea

The title compound of Preparation 5 (0.35 g, 0.84 mmol) was treated with (Z)-tributyl(2-ethoxyvinyl)stannane (0.26 g, 0.72 mmol) and tetrakis(triphenylphosphine)-palladium (42 mg, 0.04 mmol) according to the method of Preparation 14. Purification was carried out by reverse-phase chromatography using the SP1 Purification system to give 130 mg (0.32 mmol, 38%) of the title compound as a colourless oil. Purity 98%.
UPLC/MS (3 min) retention time 2.01 min.
LRMS: m/z 407 (M+1).

### PREPARATION 16

### N-{2-[(Z)-2-Ethoxyvinyl]-5-fluorobenzyl}-N-ethylcyclopropanecarboxamide

The title compound of Preparation 7 (1.50 g, 5.0 mmol) was treated with (Z)-tributyl(2-ethoxyvinyl)stannane (1.80 g, 5.0 mmol) and tetrakis(triphenylphosphine)palladium (0.25 g, 0.22 mmol) according to the method of Preparation 14 to give 0.45 g (1.54 mmol, 31%) of the title compound as a colourless oil. Purity 98%.
Partial ¹H NMR (400 MHz, CHLOROFORM-d) 53:47 mixture of two rotamers.
Major rotamer δ ppm 6.23 (1 H, d, *J*=7.0 Hz), 5.25 (1 H, d, *J*=7.0 Hz), 4.62 (2 H, s).
Minor rotamer δ ppm 6.30 (1 H, d, *J*=7.0 Hz), 5.19 (1 H, d, *J*=7.0 Hz), 4.66 (2 H, s).
HPLC/MS (9 min) retention time 6.55 min.
LRMS: m/z 292 (M+1).

### PREPARATION 17

### [2-promo-5 (trifluoromethyl)benzyl]amine

2-Bromo-5-(trifluoromethyl)benzaldehyde (10 g, 39.5 mmol) was dissolved in 400 ml methanol. Hydroxylamine (2.4 ml, 39.5 mmol) was added and the mixture was stirred at room temperature for 3 h. The mixture was evaporated under reduced pressure and the mixture was partitioned between ethyl acetate and water. The organic layer was washed with water, dried over sodium sulphate, filtered and evaporated under reduced pressure. The solid residue was dissolved in 300 ml acetic acid. Zinc dust (5.2 g, 79 mmol) was added and the suspension was stirred for 5 h. Further zinc dust (2.6 g, 40 mmol) was added and the suspension was stirred overnight. Further zinc dust (2.6 g, 40 mmol) was added and the suspension was stirred for 1 h. The mixture was filtered through Celite. The filtrate was evaporated and the residue re-evaporated twice from cyclohexane. The semi-solid residue was triturated with ether to give a solid which was collected by filtration, washed with ether and dried in the oven to give 6.5 g (25.6 mmol, 65%) of the title compound as a white solid. Purity 100%.
¹H NMR (400 MHz, CHLOROFORM-*d*) ) δ ppm 3.21 (s, 2 H), 4.00 (s, 2 H), 7.40 (d, *J*=8.21 Hz, 1 H), 7.65 - 7.73 (m, 2 H).
UPLC/MS (3 min) retention time 0.90 min.
LRMS: m/z 254, 256 (M+1).

### PREPARATION 18

### N-[2-Bromo-5-(trifluoromethyl)benzyl]cyclopropanecarboxamide

The title compound of Preparation 17 (6.5 g, 25.6 mmol) was dissolved in 50 ml dichloromethane and triethylamine (3.0 ml, 21.5 mmol). Cyclopropanecarbonyl chloride (3 ml, 33 mmol) was added drop-wise over 15 min and the mixture was stirred at room temperature for 2 h. The mixture was diluted with dichloromethane and was washed sequentially twice with water and once with brine. The organics were dried over sodium sulphate, filtered and evaporated under reduced pressure to give 7.4 g of the crude title compound as an oil. Purity 87%.
UPLC/MS (3 min) retention time 1.63 min.
LRMS: m/z 322, 324 (M+1).

### PREPARATION 19

### N-[2-Bromo-5-(trifluoromethyl)benzyl]cyclopropanecarbothioamide

The crude title compound of Preparation 18 (7.4 g) was dissolved in 200 ml dioxane. Diisopropylethylamine (32 ml, 183 mmol) and phosphorous pentasulfide (20 g, 90 mmol) were added and the mixture was stirred at 100 °C overnight. The mixture was evaporated and the residue was dissolved in ethyl acetate. The organics were washed with water, dried over sodium sulphate, filtered and evaporated under reduced pressure to give 7.7 g of the crude title compound. Purity 90%.
UPLC/MS (3 min) retention time 1.90 min.
LRMS: m/z 338, 340 (M+1).

### PREPARATION 20

### 4-[2-Bromo-55-(trifluoromethyl)benzyl]-3-cyclopropyl-5-methyl-4H-1,2,4-triazole

The crude title compound of Preparation 19 (3.5 g) was dissolved in 30 ml acetonitrile. Acetic hydrazide (1.5 g, 20 mmol) and mercuric acetate (5 g, 15.7 mmol) were added and the mixture was stirred for 4 h at room temperature. The mixture was filtered through Celite, the Celite was washed with acetonitrile and the combined filtrate was evaporated. The residue was partitioned between ethyl acetate and water. The organic layer was washed with water, dried over sodium sulphate, filtered and evaporated under reduced pressure. The residue was purified using the Isolera Purification system (acetone-hexane gradient, 0:100 rising to 50:50, and then methanol-dichloromethane gradient, 0:100 rising to 10:90) to give 1.5 g (4.2 mmol, 34% over three steps) of the title compound as a white solid. Purity 100%.
¹H NMR (400 MHz, CHLOROFORM-*d*) δ ppm 7.81 (1 H, d, *J*=8.2 Hz), 7.50 (1 H, d, *J*=7.8 Hz), 6.78 (1 H, s), 5.22 (2 H, s), 2.36 (3 H, s), 1.49 - 1.59 (1 H, m), 1.07 - 1.15 (2 H, m), 0.91 - 0.98 (2 H, m).
UPLC/MS (3 min) retention time 1.52 min.
LRMS: m/z 360, 362 (M+1).

### PREPARATION 21

### 3-Cyclopropyl-4-[2-[(Z)-2-ethoxyvinyl]-5-(trifluoromethyl)benzyl]-5-methyl-4H-1,2,4-triazole

The title compound of Preparation 20 (0.50 g, 1.39 mmol), (Z)-tributyl(2-ethoxyvinyl)stannane (0.60 g, 1.67 mmol) and tetrakis(triphenylphosphine)palladium (80 mg, 0.07 mmol) were dissolved in 10 ml toluene. The mixture was submitted to three vacuum-argon cycles and was then was stirred and heated for 2 h at 150 °C in the microwave. The mixture filtered and was evaporated to dryness and the residue was partitioned between ethyl acetate and water. The organic phase was washed sequentially twice with water, once with brine and was dried over sodium sulphate, filtered and evaporated under reduced pressure to give 0.45 g of the crude title compound as a brown oil. Purity 60%.
UPLC/MS (3 min) retention time 1.73 min.
LRMS: m/z 352 (M+1).

### PREPARATION 22

### N-[(2-Chloropyridin-3-yl)methyl]ethanamine

2-Chloronicotinaldehyde (2.0 g, 14.3 mmol) was treated with ethylamine (2M in tetrahydrofuran, 12 ml, 24 mmol), sodium cyanoborohydride (1.33 g, 21 mmol) and acetic acid (1.2 ml, 21 mmol) according to the method of Preparation 3 to give 2.5 g of the crude title compound as a colourless oil. Purity 70%.
¹H NMR (300 MHz, CHLOROFORM-*d*) δ ppm 8.29 (dd, *J*=4.67, 1.92 Hz, 1 H), 7.78 (dd, *J*=7.69, 1.92 Hz, 1 H), 7.24 (dd, *J*=7.42, 4.67 Hz, 1 H), 3.88 (s, 2 H), 2.70 (q, *J*=7.14 Hz, 2 H), 1.16 (t, *J*=7.14 Hz, 3 H).
UPLC/MS (3 min) retention time 0.34 min.
LRMS: m/z 171 (M+1).

### PREPARATION 23

### N-[(2-Chloropyridin-3-yl)methyl]-N-ethylcyclopropanecarboxamide

The crude title compound of Preparation 22 (2.5 g) was treated with triethylamine (1.7 ml, 12.3 mmol) and cyclopropanecarbonyl chloride (0.85 ml, 9.3 mmol) according to the method of Preparation 4. The resulting residue was purified using the Isolera Purification System (ethyl acetate-hexane gradient, 0:100 rising to 100:0) to give 2.10 g (8.8 mmol, 61% over two steps) of the title compound as a colourless oil. Purity 100%.
Partial ¹H NMR (300 MHz, CHLOROFORM-d) 65:35 mixture of two rotamers.
Major rotamer δ ppm 8.29 (1 H, d, *J*=4.1 Hz), 4.68 (2 H, s), 1.29 (3 H, t, *J*=7.1 Hz),
Minor rotamer δ ppm 8.36 (1 H, d, *J*=4.1 Hz), 4.74 (2 H, s), 1.16 (3 H, t, *J*=7.0 Hz).
UPLC/MS (3 min) retention time 1.29 min.
LRMS: m/z 239 (M+1).

### PREPARATION 24

### N-({2-[(Z)-2-Ethoxyvinyl]pyridin-3-yl}methyl)-N-ethylcyclopropanecarboxamide

The title compound of Preparation 23 (0.60 mg, 2.5 mmol) was treated with (Z)-tributyl(2-ethoxyvinyl)stannane (1.09 g, 3.0 mmol) and tetrakis(triphenylphosphine)-palladium (0.29 g, 0.25 mmol) according to the method of Preparation 14 to give 0.61 g (2.2 mmol, 89%) of the title compound as a colourless oil. Purity 90%.
Partial ¹H NMR (400 MHz, CHLOROFORM-d) 50:50 mixture of two rotamers.
Rotamer 1 δ ppm 6.43 (1 H, d, *J*=7.4 Hz), 5.25 (1 H, d, *J*=7.4 Hz), 4.72 (2 H, s).
Rotamer 2 δ ppm 6.38 (1 H, d, *J*=7.4 Hz), 5.25 (1 H, d, *J*=7.0 Hz), 4.65 (2 H, s).
UPLC/MS (3 min) retention time 0.83 min.
LRMS: m/z 275 (M+1).

### PREPARATION 25

### 1-Cyclopropylbutane-1,3-dione

Sodium hydride (60% dispersion in oil, 1.9 g, 47.5 mmol) was washed several times with pentane and then suspended in 35 ml tetrahydrofuran under nitrogen. Ethyl acetate (4.7 ml, 48 mmol) and 2 drops of ethanol were added. A solution of cyclopropyl methyl ketone (2.0 g, 23.8 mmol) dissolved in 10 ml tetrahydrofuran was added drop-wise with stirring, maintaining the reaction temperature below 20 ºC with an ice-bath. 18-Crown-6 (130 mg, 0.49 mmol) was added and the mixture was stirred at 40 ºC for 3 h. The mixture was evaporated under reduced pressure and the residue was partitioned between ether and water. The aqueous phase was extracted with ether. The aqueous was then acidified to pH 2 with 5N hydrochloric acid. The aqueous was further extracted three times with ether and the combined organics were washed with brine, dried over sodium sulphate, filtered and evaporated under reduced pressure to give 3.1 g of the crude title compound.
¹H NMR (400 MHz, CHLOROFORM-*d*) ) δ ppm 0.88 - 0.95 (m, 2 H), 1.07 - 1.14 (m, 2 H), 1.56 - 1.68 (m, 1 H), 2.02 (s, 3 H), 3.70 (s, 2 H).
UPLC/MS (3 min) retention time 1.33 min.
LRMS: *m*/*z* 127 (M+1).

### PREPARATION 26

### (2Z)-2-[2-Bromo-5-(trifluoromethyl)benzylidene]-1-cyclopropylbutane-1,3-dione

The crude title compound of Preparation 25 (1.0 g) was dissolved in 22 ml ethanol. 2-Bromo-5-(trifluoromethyl)benzaldehyde (2.0 g, 7.9 mmol) was added followed by 0.3 ml piperidine and 0.6 ml acetic acid. The mixture was stirred at reflux for 3.5 h. The mixture was then evaporated under reduced pressure and the residue was purified using the SP1 Purification System (ethyl acetate-hexane gradient, 0:100 rising to 5:95) to give 1.13 g of the crude title compound as a white waxy solid. Purity 88%.
¹H NMR (400 MHz, CHLOROFORM-*d*) ) δ ppm 0.86 - 0.96 (m, 2 H), 1.17 (m, 2 H), 1.83 - 1.93 (m, 1 H), 2.45 (s, 3 H), 7.50 (d, *J*=8.21 Hz, 1 H), 7.61 (s, 1 H), 7.74 - 7.82 (m, 2 H).
UPLC/MS (3 min) retention time 1.87 min. LRMS: m/z 361, 363 (M+1).

### PREPARATION 27

### 2-[2-Bromo-5-(trifluoromethyl)benzyl]-1-cyclopropylbutane-1,3-dione

The crude title compound of Preparation 26 (1.13 g) was dissolved in 27 ml acetic acid. Zinc powder (1.08 g, 16.5 mmol) was added and the mixture was stirred at 80 ºC for 15 min. The mixture was allowed to cool and was filtered through a plug of Celite, washing several times with ethyl acetate. The combined filtrates were evaporated under reduced pressure. The residue was partitioned between dichloromethane and water. The organics were washed with brine, dried over sodium sulphate, filtered and evaporated to give 1.0 g of the crude title compound as a yellow oil. Purity 61%.
UPLC/MS (3 min) retention time 1.93 min.
LRMS: m/z 363, 365 (M+1).

### PREPARATION 28

### 4-[2-Bromo-5-(trifluoromethyl)benxyl]-5-cyclopropyl-3-methylisoxaxole

### 4-[2-Bromo-5-(trifluoromethyl)benzyl]-3-cyclopropyl-5-methylisoxazole

The crude title compound of Preparation 27 (0.50 g) was dissolved in 5 ml ethanol. Hydroxylamine hydrochloride (175 mg 2.5 mmol) was added and the mixture was stirred at 80 ºC for 2 h. The mixture was evaporated under reduced pressure and the residue was partitioned between ethyl acetate and water. The organics were washed sequentially with 4% w/v sodium bicarbonate solution, brine, dried over sodium sulphate, filtered and evaporated. The residue was partially purified using the SP1 Purification System (acetone-hexane gradient, 0:100 rising to 5:95) to give 0.28 g of a mixture of the crude title compounds as a colourless oil. Purity 83%.
¹H NMR (Spectrum reported as sum of both compounds: 400 MHz, CHLOROFORM-*d*) δ 1 ppm 0.78 - 0.86 (m, 2 H), 0.88 -1.01 (m, 4 H), 1.06 - 1.15 (m, 2 H), 1.40 - 1.50 (m, 1 H), 1.73 -1.82 (m, 1 H), 2.09 (s, 3 H), 2.29 (s, 3 H), 3.89 (d, *J*=2.34 Hz, 4 H), 7.25 (d, *J*=6.25 Hz, 2 H), 7.37 (d, *J*=8.21 Hz, 2 H), 7.72 (d, *J*=8.21 Hz, 2 H).
UPLC/MS (3 min) retention time 2.06 min.
LRMS: m/z 360, 362 (M+1).

### PREPARATION 29

### 5-Cyclopropyl-4-[2-[(Z)-2-ethoxyvinyl]55-(trifluoromethyl)benzyl]-3-methylisoxazole

### 3-Cyclopropyl-4-[2-[(Z)-2-ethoxyvimyl]-5-(trifluoromethyl)benzyl]-5-methylisoxazole

The mixture of the crude title compounds of Preparation 28 (110 mg) was treated with (Z)-tributyl(2-ethoxyvinyl)stannane (120 mg, 0.34 mmol) and tetrakis(triphenylphosphine)palladium (15 mg, 0.012 mmol) according to the method of Preparation 14. The residue was partially purified by reverse-phase chromatography using the SP1 Purification system to give 100 mg of a mixture of the crude title compounds. Purity 65%.
UPLC/MS (3 min) retention time 2.10 min.
LRMS: m/z 352 (M+1).

### PREPARATION 30

### 3-lodo-1H-indazole

Iodine (5.8 g, 22.9 mmol) was added in portions over approximately 20 min to a solution of indazole (2.5 g, 21.7 mmol) in methanol (63 ml) and 2N sodium hydroxide solution (65 ml). The mixture remained colourless and a white precipitate slowly formed. The mixture was stirred at room temperature 48 h. The mixture was cooled in an ice-bath and 7.5 ml of concentrated hydrochloric acid was slowly added. The mixture was further acidified with 2N hydrochloric acid. 20% w/v Sodium thiosulfate pentahydrate solution was added until the iodine colour disappeared. The precipitate was filtered, washed with water and dried in the oven at 50 ºC to constant weight. The solid was taken up in methanol, filtered and the filtrated was evaporated under reduced pressure to give 5.0 g (20.6 mmol, 95%) of the title compound as a white solid. Purity 100%.
¹H NMR (300 MHz, CHLOROFORM-d) δ ppm 7.43-7.59 (m, 3H), 7.21-7.26 (m, 1H).
UPLC/MS (3 min) retention time 1.56 min.
LRMS: m/z 245 (M+1).

### PREPARATION 31

### tert-Butyl (3-iodo-1H-indazol-1-yl)acetate

The title compound of Preparation 30 (3.0 g, 12.3 mmol) was dissolved in 45 ml dimethylformamide. Caesium carbonate (8.0 g, 24.5 mmol) and tert-butyl 2-bromoacetate (1.82 ml, 12.3 mmol) were added and the mixture was stirred at room temperature overnight. The mixture was partitioned between ethyl acetate and water. The organic phase was washed sequentially with water and brine, dried over sodium sulphate, filtered and evaporated under reduced pressure. The residue was purified by flash chromatography (ethyl acetate-hexane, 10:90) to give 3.2 g (8.9 mmol, 73%) of the desired compound as a pale yellow oil. Purity 95%.
¹H NMR (300 MHz, CHLOROFORM-d) δ ppm 7.45 - 7.67 (m, 2 H), 7.23 - 7.43 (m, 2 H), 5.13 (s, 2 H), 1.50 (s, 9 H).
UPLC/MS (3 min) retention time 1.96 min.
LRMS: *m*/*z* 359 (M+1).

### PREPARATION 32

### tert-Butyl {3-[2-{[(cyclopropylcarbonyl)(ethyl)amino]methyl}-4-(trifluoromethyl)phenyl]-1H-indazol-1-yl}acetate

The title compound of Preparation 31 (100 mg, 0.28 mmol) and the title compound of Preparation 10 (111 mg. 0.28 mmol) were dissolved in 2 ml dioxane in a Schlenk vessel. A solution of caesium carbonate (2M, 0.42 ml, 0.84 mmol) was added and the reaction mixture was submitted to three vacuum-argon cycles. [1,1'-bis(diphenylphosphino)ferrocene]dichloropalladium(II), complex with dichloromethane (0.45 g, 0.39 mmol) was then added and the mixture was further submitted to three vacuum-argon cycles. The reaction vessel was sealed and the contents were stirred at 100 ºC overnight. The mixture was cooled to room temperature, evaporated under reduced pressure and the residue re-suspended in ethyl acetate. The organics were washed sequentially with water and brine, dried over sodium sulphate, filtered and evaporated under reduced pressure. The residue was purified by flash chromatography (ethyl acetate-hexane, 20:80) to give 95 mg (0.19 mmol, 68%) of the title compound as a white solid. Purity 98%.
Partial ¹H NMR (300 MHz, CHLOROFORM-d) 50:50 mixture of two rotamers.
Rotamer 1: δ ppm 5.15 (s, 2 H), 4.98 (s, 2 H).
Rotamer 2: δ ppm 5.12 (s, 2 H), 4.86 (s, 2 H).
UPLC/MS (3 min) retention time 2.15 min.
LRMS: m/z 502 (M+1).

### PREPARATION 33

### 4-Chloro-3-iodo-1H-indazole

4-Chloro-1H-indazole (0.5 g, 3.28 mmol) was treated with iodine (0.91 g, 3.6 mmol) and 2N sodium hydroxide solution (10 ml, 20 mmol) according to the method of Preparation 30 to give 900 mg (3.23 mmol, 99%) of the title compound. Purity 96%.
¹H NMR (300 MHz, CHLOROFORM-d) δ ppm 7.18 (d, *J*=7.42 Hz, 1 H), 7.32 (t, *J*=7.83 Hz, 1 H), 7.47 (d, *J*=8.24 Hz, 1 H), 10.66 (br. s., 1 H).
UPLC/MS (3 min) retention time 1.71 min.
LRMS: m/z 279 (M+1).

### PREPARATION 34

### tert-Butyl (4-chloro-3-iodo-1H-indazol-1-yl)acetate

The title compound of Preparation 33 (500 mg, 1.80 mmol) was treated with caesium carbonate (1.1 g, 3.38 mmol) and *tert*-butyl 2-bromoacetate (265 µl, 1.8 mmol) according to the method of Preparation 31. The resulting residue was purified using the Isolera Purification System (ethyl acetate-hexane gradient, 0:100 rising to 100:0) to give 475 mg (1.21 mmol. 67%) of the title compound. Purity 91%.
¹H NMR (300 MHz, CHLOROFORM-d) δ ppm 7.22 - 7.37 (m, 2 H), 7.17 (d, *J*=6.59 Hz, 1 H), 5.04 (s, 2 H), 1,44 (s, 9 H).
UPLC/MS (3 min) retention time 2.03 min.
LRMS: m/z 393 (M+1).

### PREPARATION 35

### Ethyl (4-chloro-3-iodo-1H-indazol-1-yl)acetate

The title compound of Preparation 33 (0.88 g, 3.2 mmol) was treated with caesium carbonate (2.1 g, 6.3 mmol) and ethyl 2-bromoacetate (0.38 ml, 3.5 mmol) according to the method of Preparation 31. The resulting residue was purified using the Isolera Purification System (ethyl acetate-hexane gradient, 0:100 rising to 40:60) to give 0.77 g (2.1 mmol, 66%) of the title compound. Purity 91%.
UPLC/MS (3 min) retention time 1.87 min.
LRMS: m/z 365 (M+1).

### PREPARATION 36

### tert-Butyl {4-chloro-3-[2-{[(cyclopropylcarbonyl)((ethyl)amino]methyl}-4-(trifluoromethyl)phenyl]-1H-indazol-1-yl}acetate

The title compound of Preparation 34 (200 mg, 0.51 mmol) and the title compound of Preparation 10 were treated with caesium carbonate solution (2M, 0.75 ml, 1.5 mmol) and [1,1'-bis(diphenylphosphino)ferrocene] dichloropalladium(II), complex with dichloromethane (41 mg, 0.05 mmol) according to the method of Preparation 32. The resulting residue was purified using the Isolera Purification System (ethyl acetate-hexane gradient, 0:100 rising to 100:0) to give 180 mg (0.33 mmol, 65%) of the title compound. Purity 90%.
Partial ¹H NMR (300 MHz, CHLOROFORM-d) 75:25 mixture of two rotamers.
Major rotamer δ ppm 5.11 (d, *J*=5.22 Hz, 2 H), 4.60 (s, 2 H).
Minor rotamer δ ppm 5.01 (d, *J*=10.16 Hz, 2 H), 4.63 (s, 2 H).
UPLC/MS (3 min) retention time 2.19 min.
LRMS: m/z 536 (M+1).

### PREPARATION 37

### tert-Butyl [4-chloro-3-(2-([(cyclopropylcarbonyl)(ethyl)amino]methyl)-4-fluorophenyl)-1H-indazol-1-yl]acetate

The title compound of Preparation 34 (150 mg, 0.38 mmol) and the title compound of Preparation 11 (150 mg, 0.43 mmol) were treated with caesium carbonate solution (2M, 0.57 ml, 1.15 mmol) and [1,1'-bis(diphenylphosphino)ferrocene]dichloro-palladium(II), complex with dichloromethane (31 mg, 0.04 mmol) according to the method of Preparation 32. The resulting residue was purified using the Isolera Purification System (ether-hexane gradient, 0:100 rising to 100:0) to give 70 mg (0.14 mmol, 38%) of the title compound. Purity 100%.
Partial ¹H NMR (300 MHz, CHLOROFORM-d) 55:45 mixture of two rotamers.
Major rotamer δ ppm 5.09 (s, 2 H).
Minor rotamer δ ppm 5.10 (s, 2 H).
UPLC/MS (3 min) retention time 2.11 min.
LRMS: m/z 486 (M+1).

### PREPARATION 38

### 5-Fluoro-3-iodo-1H-indazole

5-Fluoro-1H-indazole (0.3 g, 2.20 mmol) was treated with iodine (0.61 g, 2.42 mmol) and 2N sodium hydroxide solution (6.6 ml, 13.2 mmol) according to the method of Preparation 30 to give 580 mg (2.20 mmol. 90%) of the title compound. Purity 90%.
¹H NMR (300 MHz, CHLOROFORM-d) δ ppm 10.52 (br. s., 1 H), 7.47 (dd, *J*=8.93, 3.98 Hz, 1 H), 7.23 - 7.32 (m, 1 H), 7.17 (dd, *J*=8.24, 2.47 Hz, 1 H).
UPLC/MS (3 min) retention time 1.60 min.
LRMS: m/z 263 (M+1).

### PREPARATION 39

### tert-Butyl (5-fluoro-3-iodo-1H-indazol-1-yl)acetate

The title compound of Preparation 38 (615 mg, 2.35 mmol) was treated with caesium carbonate (764 mg, 2.34 mmol) and *tert*-butyl 2-bromoacetate (0.69 ml, 4.7 mmol) according to the method of Preparation 31. The resulting residue was purified using the Isolera Purification System (ethyl acetate-hexane gradient, 0:100 rising to 100:0) to give 730 mg (1.94 mmol, 75%) of the title compound. Purity 90%.
¹H NMR (300 MHz, CHLOROFORM-d) δ ppm 7.21 - 7.31 (m, 2 H), 7.14 (dd, *J*=8.24 Hz, 1 H), 5.05 (s, 2 H), 1.44 (s, 9 H).
UPLC/MS (3 min) retention time 1.98 min.
LRMS: m/z 377 (M+1).

### PREPARATION 40

### tert-Butyl {3-[2-{[(cyclopropylcarbonyl)(ethyl)amine]methyl}-4-(trifluoromethyl)phenyl]-5-fluoro-1H-indazol-1-yl}acetate

The title compound of Preparation 39 (150 mg, 0.40 mmol) and the title compound of Preparation 10 (158 mg, 0.40 mmol) were treated with caesium carbonate solution (2M, 0.60 ml, 1.2 mmol) and [1,1'-bis(diphenylphosphino)ferrocene]dichloro-palladium(II), complex with dichloromethane (33 mg, 0.04 mmol) according to the method of Preparation 32. The resulting residue was purified using the Isolera Purification System (ethyl acetate-hexane gradient, 0:100 rising to 100:0) to give 130 mg (0.25 mmol, 58%) of the title compound. Purity 93%.
Partial ¹H NMR (300 MHz, CHLOROFORM-d) 55:45 mixture of two rotamers.
Major rotamer δ ppm 5.16 (m, 2 H), 4.90 (br. s., 2 H).
Minor rotamer δ ppm 5.18 (m, 2 H), 5.01 (br. s., 2 H).
UPLC/MS (3 min) retention time 2.16 min.
LRMS: m/z 520 (M+1).

### PREPARATION 41

### 3-lodo-5-(trifluoromethyl)-1H-indazole

5-(Trifluromethyl)-1H-indazole (500 mg, 2.7 mmol) was treated with iodine (750 mg, 2.95 mmol) and 2N sodium hydroxide solution (8.0 ml, 16 mmol) according to the method of Preparation 30 to give 760 mg (2.43 mmol, 91%) of the title compound. Purity 100%.
¹H NMR (300 MHz, CHLOROFORM-d) δ ppm 10.91 (br. s., 1 H), 7.86 (s, 1 H), 7.67 (q, *J*=8.79 Hz, 2 H).
UPLC/MS (3 min) retention time 1.83 min.
LRMS: m/z 313 (M+1).

### PREPARATION 42

### tert-Butyl [3-iodo-5-(trifluoromethyl)-1H-indazol-1-yl]acetate

The title compound of Preparation 41 (300 mg, 0.96 mmol) was treated with caesium carbonate (626 mg, 1.92 mmol) and tert-butyl 2-bromoacetate (0.28 ml, 1.92 mmol) according to the method of Preparation 31. The resulting residue was purified using the lsolera Purification System (ether-hexane gradient, 0:100 rising to 100:0) to give 400 mg (0.93 mmol, 88%) of the title compound. Purity 90%.
¹H NMR (300 MHz, CHLOROFORM-d) δ ppm 7.83 (s, 1 H), 7.69 (d, *J*=8.79 Hz, 1 H), 7.43 (d, *J*=8.79 Hz, 1 H), 5.11 (s, 2 H), 1.46 (s, 9 H).
UPLC/MS (3 min) retention time 2.10 min.
LRMS: m/z 427 (M+1).

### PREPARATION 43

### tert-Butyl [3-(2-{[(syclopropylcarbonyl)(ethyl)amino]methyl}-4-fluorophenyl)-5-(trifluoromethyl)-1H-indazol-1-yl]acetate

The title compound of Preparation 42 (150 mg, 0.35 mmol) and the title compound of Preparation 11 (150 mg, 0.43 mmol) were treated with caesium carbonate solution (2M, 0.53 ml, 1.06 mmol) and [1,1'-bis(diphenylphosphino)ferrocene] dichloropalladium(II), complex with dichloromethane (29 mg, 0.04 mmol) according to the method of Preparation 32. The resulting residue was purified using the Isolera Purification System (ether-hexane gradient, 0:100 rising to 100:0) to give 115 mg (0.22 mmol, 57%) of the title compound. Purity 91%.
Partial ¹H NMR (300 MHz, CHLOROFORM-d) 55:45 mixture of two rotamers.
Major rotamer δ ppm 5.14 (s, 2 H), 4.76 (s, 2 H).
Minor rotamer δ ppm 5.16 (s, 2 H), 4.86 (s, 2 H).
UPLC/MS (3 min) retention time 2.17 min.
LRMS: m/z 520 (M+1).

### PREPARATION 44

### 5-Chloro-3-iodo-1H-indazole

5-Chloro-1 H-indazole (0.40 g, 2.69 mmol) was treated with iodine (0.99 g, 3.9 mmol) and 2N sodium hydroxide solution (8.0 ml, 16.0 mmol) according to the method of Preparation 30 to give 725 mg (2.60 mmol, 97%) of the title compound. Purity 98%.
¹H NMR (300 MHz, CHLOROFORM-d) δ ppm 10.70 (br. s., 1 H), 7.36 - 7.66 (m, 3 H).
UPLC/MS (3 min) retention time 1.80 min.
LRMS: m/z 279 (M+1).

### PREPARATION 45

### Ethyl (5-chloro-3-iodo-1H-indazol-1-yl)acetate

The title compound of Preparation 44 (725 mg, 2.60 mmol) was treated with caesium carbonate (1.7 g, 5.22 mmol) and ethyl-2-bromoacetate (0.32 ml, 2.88 mmol) according to the method of Preparation 31. The resulting residue was purified using the Isolera Purification System (ethyl acetate-hexane gradient, 0:100 rising to 100:0) to give 580 mg (1.59 mmol, 58%) of the title compound. Purity 96%.
¹H NMR (300 MHz, CHLOROFORM-d) δ ppm 7.51 (d, *J*=1.65 Hz, 1 H), 7.43 (dd, *J*=9.06,1.92 Hz, 1 H), 7.28 (d, *J*=2.20 Hz, 1 H), 5.15 (s, 2 H), 4.24 (q, *J*=7.14 Hz, 2 H), 1.27 (t, *J*=7.14 Hz, 3H).
UPLC/MS (3 min) retention time 1.94 min.
LRMS: m/z 365 (M+1).

### PREPARATION 46

### Ethyl {5-chloro-3-[2-{[(cyclopropylcarbonyl)(ethyl)amino]methyl}-4-(trifluoromethyl)phenyl]-1H-indazol-1-yl}acetate

The title compound of Preparation 45 (200 mg, 0.55 mmol) and the title compound of Preparation 10 (240 mg, 0.60 mmol) were treated with caesium carbonate solution (2M, 0.82 ml, 1.64 mmol) and [1,1'-bis(diphenylphosphino)ferrocene]dichloro-palladium(II), complex with dichloromethane (45 mg, 0.06 mmol) according to the method of Preparation 32. The resulting residue was purified using the Isolera Purification System (ether-hexane gradient, 0:100 rising to 100:0) to give 160 mg (0.31 mmol, 57%) of the title compound. Purity 96%.
Partial H NMR (300 MHz, CHLOROFORM-d) 55:45 mixture of two rotamers.
Major rotamer δ ppm 5.20 (s, 2 H), 4.85 (s, 2 H).
Minor rotamer δ ppm 5.23 (s, 2 H), 4.95 (s, 2 H).
UPLC/MS (3 min) retention time 2.15 min.
LRMS: m/z 508 (M+1).

### PREPARATION 47

### 6-Fluoro-3-iodo-1H-indazole

6-Fluoro-1H-indazole (500 mg, 3.67 mmol) was treated with iodine (1.4 g, 5.52 mmol) and 2N sodium hydroxide solution (11.0 ml, 22.0 mmol) according to the method of Preparation 30 to give 900 mg (3.4 mmol, 93%) of the title compound. Purity 100%.
¹H NMR (300 MHz, CHLOROFORM-d) δ ppm 11.07 (br. s., 1 H), 7.49 (dd, *J*=8.79, 4.94 Hz, 1 H), 7.25 (dd, *J*=9.06, 2.20 Hz, 1 H), 6.89 - 7.15 (m, 1 H).
UPLC/MS (3 min) retention time 1.59 min.
LRMS: m/z 263 (M+1).

### PREPARATION 48

### tert-Butyl (6-fluoro-3-iodo-1H-indazo-1-yl)acetate

The title compound of Preparation 47 (500 mg, 1.91 mmol) was treated with caesium carbonate (1.24 g, 3.81 mmol) and *tert*-butyl 2-bromoacetate (310 µl, 2.1 mmol) according to the method of Preparation 31. The resulting residue was purified using the Isolera Purification System (ether-hexane gradient, 0:100 rising to 100:0) to give 570 mg (1.51 mmol, 77%) of the title compound. Purity 97%.
¹H NMR (300 MHz, CHLOROFORM-d) δ ppm 7.45 (dd, *J*=8.79, 4.94 Hz, 1 H), 6.94 - 7.04 (m, 2 H), 5.00 (s, 2 H), 1.45 (s, 9 H).
UPLC/MS (3 min) retention time 1.97 min.
LRMS: m/z 377 (M+1).

### PREPARATION 49

### tert-Butyl (3-[2-{[(cyclopropylcarbonyl)(ethyl)amino]methyl}-4-(trifluoromethyl)phenyl]-6-fluoro-1H-indazol-1-yl}acetate

The title compound of Preparation 48 (120 mg, 0.33 mmol) and the title compound of Preparation 10 (130 mg, 0.33 mmol) were treated with caesium carbonate solution (2M, 0.50 ml, 1.0 mmol) and [1,1'-bis(diphenylphosphino)ferrocene]dichloro-palladium(II), complex with dichloromethane (27 mg, 0.03 mmol) according to the method of Preparation 32. The resulting residue was purified using the Isolera Purification System (ether-hexane gradient, 0:100 rising to 100:0) to give 70 mg (0.13 mmol, 41%) of the title compound. Purity 100%.
Partial ¹H NMR (300 MHz, CHLOROFORM-d) 55:45 mixture of two rotamers.
Major rotamer δ ppm 5.06-5.08 (m, 2 H), 4.83 (s, 2 H).
Minor rotamer δ ppm 5.06-5.08 (m, 2 H), 4.96 (s, 2 H).
UPLC/MS (3 min) retention time 2.16 min.
LRMS: m/z 520 (M+1).

### PREPARATION 50

### 3-Iodo-4-methoxy-1H-indazole

4-Methoxy-1H-indazole (220 mg, 1.48 mmol) was treated with iodine (415 mg, 1.64 mmol) and 2N sodium hydroxide solution (4.4 ml, 8.8 mmol) according to the method of Preparation 30 to give 360 mg (1.31 mmol, 80%) of the title compound. Purity 91%.
¹H NMR (300 MHz, CHLOROFORM-d) δ ppm 7.25 - 7.37 (m, 1 H), 7.12 (d, *J*=8.24 Hz, 1 H), 6.50 (d, *J*=7.69 Hz, 1 H), 3.99 (s, 3 H).
UPLC/MS (3 min) retention time 1.46 min.
LRMS: m/z 275 (M+1).

### PREPARATION 51

### tert-Butyl (3-iodo-4-methoxy-1H-indazol-1-yl)acetate

The title compound of Preparation 50 (360 mg, 1.31 mmol) was treated with caesium carbonate (855 mg, 2.62 mmol) and tert-butyl 2-bromoacetate (0.21 ml, 1.44 mmol) according to the method of Preparation 31. The resulting residue was partially purified using the Isolera Purification System (ether-hexane gradient, 0:100 rising to 100:0) to give 420 mg of the crude title compound. Purity 70%.
¹H NMR (300 MHz, CHLOROFORM-d) δ ppm 7.24 - 7.37 (m, 1 H), 6.88 (d, *J*=8.51 Hz, 1 H), 6.49 (d, *J*=7.69 Hz, 1 H), 5.00 (s, 2 H), 3.97 (s, 3 H), 1.43 (s, 9 H).
UPLC/MS (3 min) retention time 1.89 min.
LRMS: m/z 389 (M+1).

### PREPARATION 52

### tert-Butyl {3-[2-{[(cyclopropylcarbonyl)(ethyl)amino]methyl}-4-(trifluoromethyl)phenyl-4-methoxy-1H-indaxol-1-yl}acetate

The crude title compound of Preparation 51 (135 mg) and the title compound of Preparation 10 (128 mg, 0.32 mmol) were treated with caesium carbonate solution (2M, 0.52 ml, 1.04 mmol) and [1,1'-bis(diphenylphosphino)ferrocene]dichloro-palladium(II), complex with dichloromethane (28 mg, 0.03 mmol) according to the method of Preparation 32. The resulting residue was purified using the Isolera Purification System (ether-hexane gradient, 0:100 rising to 100:0) to give 62 mg (0.11 mmol, 26% over two steps) of the title compound. Purity 98%.
Partial ¹H NMR (300 MHz, CHLOROFORM-d) 50:50 mixture of two rotamers.
Rotamer 1 δ ppm 5,07 (s, 2 H), 4.73 (br. s., 2 H).
Rotamer 2 δ ppm 5.06 (s, 2 H), 4.96 (br. s., 2 H).
UPLC/MS (3 min) retention time 2.10 min.
LRMS: m/z 532 (M+1).

### PREPARATION 53

### Methyl 2-(4-chloro-3-iodo-1H-indazol-1-yl)propanoate

The title compound of Preparation 33 (150 mg, 0.54 mmol) was treated with caesium carbonate (350 mg, 1.08 mmol) and the crude title compound of Preparation 1 (210 mg) according to the method of Preparation 31. The resulting residue was partially purified using the Isolera Purification System (ether-hexane gradient, 0:100 rising to 100:0) to give 190 mg of the crude title compound. Purity 65%.
UPLC/MS (3 min) retention time 1.89 min.
LRMS: m/z 365 (M+1).

### PREPARATION 54

### Methyl 2-{4-chloro-3-[2-{[(cyclopropylcarbonyl)(ethyl)amino]methyl}-4-(trifluoromethyl)phenyl]-1H-indazol-1-yl}propanoate

The crude title compound of Preparation 53 (190 mg) and the title compound of Preparation 10 (148 mg, 0.37 mmol) were treated with caesium carbonate solution (2M, 0.57 ml, 1.14 mmol) and [1,1'-bis(diphenylphosphino)ferrocene]dichloro-palladium(II), complex with dichloromethane (30 mg, 0.04 mmol) according to the method of Preparation 32. The resulting residue was purified using the Isolera Purification System (ether-hexane gradient, 0:100 rising to 100:0) to give 55 mg (0.101mmol, 20% over two steps) of the title compound. Purity 100%.
Partial ¹H NMR (300 MHz, CHLOROFORM-d) 50:50 mixture of two rotamers.
Rotamer 1 δ ppm 3.74 (s, 2 H).
Rotamer 2 δ ppm 3.73 (s, 2 H).
UPLC/MS (3 min) retention time 2.11 min.
LRMS: m/z 508 (M+1).

### PREPARATION 55

### 3-Iodo-4-(trifluoromethyl)-1H-indazole

4-Trifluoromethyl-1H-indazole (250 mg, 1.34 mmol) was treated with iodine (330 mg, 1.47 mmol) and 2N sodium hydroxide solution (4.0 ml, 8.0 mmol) according to the method of Preparation 30 to give 370 mg (1.18 mmol, 88%) of the title compound. Purity 100%.
¹H NMR (300 MHz, CHLOROFORM-d) δ ppm 11.62 (br. s., 1 H), 7.86 (d, *J*=8.24 Hz, 1 H), 7.61 (d, 1 H), 7.53 (d, *J*=7.97 Hz, 1 H).
UPLC/MS (3 min) retention time 1.72 min.
LRMS: m/z 313 (M+1).

### PREPARATION 56

### tert-Butyl [3-iodo-4-(trifluoromethyl)-1H-indazol-1-yl]acetate

The title compound of Preparation 55 (200 mg, 0.64 mmol) was treated with caesium carbonate (626 mg, 1.92 mmol) and *tert*-butyl 2-bromoacetate (125 µl, 0.85 mmol) according to the method of Preparation 31 to give 180 mg of the crude title compound. Used as such without further purification. Purity 80%.
¹H NMR (300 MHz, CHLOROFORM-d) δ ppm 7.40 - 7.82 (m, 3 H), 5.12 (s, 2 H), 1.46 (s, 9 H).
UPLC/MS (3 min) retention time 2.02 min.
LRMS: m/z 427 (M+1).

### PREPARATION 57

### 4-Fluoro-3-iodo-1H-indazole

4-Fluoro-1H-indazole (400 mg, 2.94 mmol) was treated with iodine (1.1 g, 4.37 mmol) and 2N sodium hydroxide solution (9.0 ml, 18.0 mmol) according to the method of Preparation 30 to give 770 mg (2.93 mmol, 99%) of the title compound. Purity 99%.
¹H NMR (300 MHz, CHLOROFORM-d) δ ppm 11.02 (br. s., 1 H), 7.31 - 7.42 (m, 2 H), 6.79 - 6.90 (m, 1 H).
UPLC/MS (3 min) retention time 1.54 min.
LRMS: m/z 263 (M+1).

### PREPARATION 58

### tert-Butyl (4-fluoro-3-iodo-1H-indazol-1-yl)acetate

The title compound of Preparation 57 (250 mg, 0.95 mmol) was treated with caesium carbonate (621 mg, 1.91 mmol) and *tert*-butyl 2-bromoacetate (160 µl, 1.24 mmol) according to the method of Preparation 31. The resulting residue was partially purified using the Isolera Purification System (ether-hexane gradient, 0:100 rising to 100:0) to give 256 mg of the crude title compound. Purity 70%.
UPLC/MS (3 min) retention time 1.94 min. LRMS: m/z 377 (M+1).

### PREPARATION 59

### tert-Butyl (3-[2-{[(cyclopropylcarbonyl)(ethyl)amino]methyl}-4-(trifluoromethyl)phenyl]-4-fluoro-1H-indazol-1-yl}-acetate

The crude title compound of Preparation 58 (120 mg) and the title compound of Preparation 10 (190 mg, 0.48 mmol) were treated with caesium carbonate solution (2M, 0.48 ml, 0.96 mmol) and [1,1'bis(diphenylphosphino)ferrocene]dichloro-palladium(II), complex with dichloromethane (26 mg, 0.03 mmol) according to the method of Preparation 32. The resulting residue was partially purified using the Isolera Purification System (ether-hexane gradient, 0:100 rising to 100:0) to give 115 mg of the crude title compound. Purity 77%.
Martial ¹H NMR (300 MHz, CHLOROFORM-d) 50:50 mixture of two rotamers.
Rotamer 1 δ ppm 5.10 (s, 2 H), 4.82 (s, 2 H).
Rotamer 2 δ ppm 5.12 (s, 2 H), 4.85 (s, 2 H).
UPLC/MS (3 min) retention time 2.15 min.
LRMS: m/z 520 (M+1).

### PREPARATION 60

### Methyl 2-(5-chloro-3-iodo-1H-indazol-1-yl)propanoate

The title compound of Preparation 44 (250 mg, 0.90 mmol) was treated with caesium carbonate (585 mg, 1.80 mmol) and the title compound of Preparation 2 (463 mg, 1.79 mmol) according to the method of Preparation 31. The resulting residue was purified using the Isolera Purification System (ether-hexane gradient, 0:100 rising to 100:0) to give 220 mg (0.60 mmol, 65%) of the title compound. Purity 97%.
¹H NMR (300 MHz, CHLOROFORM-d) δ ppm 7.50 (s, 1 H), 7.40 (d, J=9.61 Hz, 1 H), 7.40 (d, *J*=9.61 Hz, 1 H), 5.32 (q, *J*=7.23 Hz, 1 H), 3.70 (s, 3 H), 1.93 (d, *J*=6.32 Hz, 2 H).
UPLC/MS (3 min) retention time 1.96 min.
LRMS: m/z 365 (M+1).

### PREPARATION 61

### Methyl 2-{5-chloro-3-[2-{[(cyclopropylcarbonyl)(ethyl)ammo]methyl}-4-(trifluoromethyl)phenyl]-1H-indazol-1-yl}propanoate

The crude title compound of Preparation 60 (100 mg, 0.27 mmol) and the title compound of Preparation 10 (163 mg, 0.41 mmol) were treated with caesium carbonate solution (2M, 0.41 ml, 0.82 mmol) and [1,1'-bis(diphenylphosphino)-ferrocene]dichloropalladium(II), complex with dichloromethane (23 mg, 0.03 mmol) according to the method of Preparation 32. The resulting residue was purified using the Isolera Purification System (ether-hexane gradient, 0:100 rising to 100:0) to give 125 mg (0.24 mmol, 88%) of the title compound. Purity 98%.
Partial ¹H NMR (300 MHz, CHLOROFORM-d) 50:50 mixture of two rotamers.
Rotamer 1 δ ppm 5.33-5.49 (m, 1 H), 4.97 (s, 2 H).
Rotamer 2 δ ppm 5.33-5.49 (m, 1 H), 4.86 (d, J=3.30 Hz, 1 H).
UPLC/MS (3 min) retention time 2.17 min.
LRMS: m/z 508 (M+1).

### PREPARATION 62

### 3-Iodo-5-methyl-1H-indazole

5-Methyl-1H-indazole (300 mg, 2.27 mmol) was treated with iodine (700 mg, 2.76 mmol) and 2N sodium hydroxide solution (6.8 ml, 13.62 mmol) according to the method of Preparation 30 to give 550 mg (2.13 mmol, 92%) of the title compound. Purity 98%.
¹H NMR (300 MHz, CHLOROFORM-d) δ ppm 10.39 (br. s., 1 H), 7.38 (d, *J*=8.5 Hz" 1 H), 7.25-7.31 (m, 2 H), 2.49 (s, 3 H).
UPLC/MS (3 min) retention time 1.70 min.
LRMS: m/z 259 (M+1).

### PREPARATION 63

### tert-Butyl (3-iodo-5-methyl-1H-indazol-1-yl)acetate

The title compound of Preparation 62 (250 mg, 0.97 mmol) was treated with caesium carbonate (631 mg, 1.94 mmol) and *tert*-butyl 2-bromoacetate (0.21 ml, 1.45 mmol) according to the method of Preparation 31. The resulting residue was partially purified using the Isolera Purification System (ether-hexane gradient, 0:100 rising to 100:0) to give 350 mg of the crude title compound. Purity 70%.
¹H NMR (300 MHz, CHLOROFORM-d) δ ppm 7.23 - 7.35 (m, 2 H), 7.16 - 7.22 (m, 1 H), 5.03 (s, 2 H), 2.48 (s, 3 H), 1.43 (s, 9 H).
UPLC/MS (3 min) retention time 2.04 min.
LRMS: m/z 373 (M+1).

### PREPARATION 64

### tert-Butyl (3-[2-{[(cyclopropylcarbonyl)(ethyl)amino]methyl}-4-(trifluoromethyl)phenyl]-5-methyl-1H-indazol-1-yl}acetate

The crude title compound of Preparation 63 (150 mg) and the title compound of Preparation 10 (240 mg, 0.60 mmol) were treated with caesium carbonate solution (2M, 0.60 ml, 1.20 mmol) and [1,1'-bis(diphenylphosphino)-ferrocene]dichloropalladium(II), complex with dichloromethane (32 mg, 0.04 mmol) according to the method of Preparation 32. The resulting residue was purified using the Isolera Purification System (ethyl acetate-hexane gradient, 0:100 rising to 100:0) to give 195 mg (0.38 mmol, 91 % over two steps) of the title compound. Purity 92%.
Partial ¹H NMR (300 MHz, CHLOROFORM-d) 50:50 mixture of two rotamers.
Rotamer 1 δ ppm 5.08 (s, 2 H), 4.85 (s, 2 H).
Rotamer 2 δ ppm 5.10 (s, 2 H), 4.96 (s, 2 H).
UPLC/MS (3 min) retention time 2.23 min.
LRMS: m/z 516 (M+1).

### PREPARATION 65

### 4-Chloro-5-fluoro-1H-indazole

Hydrazine hydrate (3 ml, 34 mmol) was added to a solution of 2-chloro-3,6-difluorobenzaldehyde (500 mg, 2.83 mmol) in 8 ml 1,2-dimethoxyethane. The reaction was heated in the microwave for 2 h at 120 ºC. Further hydrazine hydrate (3 ml, 34 mmol) was added and the mixture was heated in the microwave at 120 ºC for 2 h. Further hydrazine hydrate (4 ml, 45 mmol) was added and the mixture was heated in the microwave for 4 h at 120 ºC. The mixture was evaporated to dryness, water was added and the aqueous was extracted with ethyl acetate. The combined organics were washed with brine, dried over sodium sulphate, filtered and evaporated under reduced pressure. The resulting residue was purified using the Isolera Purification System (ethyl acetate-hexane gradient, 0:100 rising to 100:0) to give 82 mg (0.48 mmol, 17%) of the title compound. Purity 90%.
¹H NMR (300 MHz, CHLOROFORM-d) δ ppm 10.26 (s, 1 H), 8.18 (s, 1 H), 7.33 - 7.50 (m, 1 H), 7.19 - 7.33 (m, *J*=2.47 Hz, 1 H).
UPLC/MS (3 min) retention time 1.49 min.
LRMS: m/z 171 (M+1).

### PREPARATION 66

### 4-Chloro-5-fluoro-3-iodo-1H-indazole

The title compound of Preparation 65 (150 mg, 0.88 mmol) was treated with iodine (334 mg, 1.32 mmol) and 2N sodium hydroxide solution (2.6 ml, 5.28 mmol) according to the method of Preparation 30 to give 250 mg (0.84 mmol, 96%) of the title compound. Purity 100%.
¹H NMR (400 MHz, DMSO-d6) δ ppm 7.59 (dd, *J*=8.99, 3.91 Hz, 1 H), 7.45 (t, *J*=9.38 Hz, 1 H).
UPLC/MS (3 min) retention time 1.75 min.
LRMS: m/z 297 (M+1).

### PREPARATION 67

### tert-Butyl (4-chloro-5-fluoro-3-iodo-1H-indazol-1-yl)acetate

The title compound of Preparation 66 (250 mg, 0.84 mmol) was treated with caesium carbonate (550 mg, 1.69 mmol) and *tert*-butyl 2-bromoacetate (0.16 ml, 1.10 mmol) according to the method of Preparation 31. The resulting residue was partially purified using the Isolera Purification System (ether-hexane gradient, 0:100 rising to 100:0) to give 252 mg of the crude title compound. Purity 45%.
¹H NMR (400 MHz, CHLOROFORM-d) δ ppm 7.26 - 7.28 (m, 1 H), 7.16 - 7.21 (m, 1 H), 5.03 (s, 2 H), 1.44 (s, 9 H).
UPLC/MS (3 min) retention time 2.06 min.
LRMS: m/z 411 (M+1).

### PREPARATION 68

### tert-Butyl (4-chloro-3-[2-{[(cyclopropylcarbonyl)(ethyl)amino]methyl}-4-(trifluoromethyl)phenyl]-5-fluoro-1H-indazol-1-yl}acetate

The crude title compound of Preparation 67 (225 mg) and the title compound of Preparation 10 (120 mg, 0.30 mmol) were treated with caesium carbonate solution (2M, 0.37 ml, 0.74 mmol) and [1,1'-bis(diphenylphosphino)ferrocene]dichloro-palladium(II), complex with dichloromethane (17 mg, 0.02 mmol) according to the method of Preparation 32. The resulting residue was purified using the Isolera Purification System (ether-hexane gradient, 0:100 rising to 100:0) to give 195 mg (0.35 mmol, 46% over two steps) of the title compound. Purity 92%.
Partial ¹H NMR (300 MHz, CHLOROFORM-d) 90:10 mixture of two rotamers.
Major rotamer δ ppm 5.10 (d, *J=*5.49 Hz, 2 H).
Minor rotamer δ ppm 5.01 (d, *J*=11.26 Hz, 2 H).
UPLC/MS (3 min) retention time 2.20 min.
LRMS: m/z 554 (M+1).

### PREPARATION 69

### 3-Iodo-4-methyl-1H-indazole

4-Methyl-1H-indazole (500 mg, 3.78 mmol) was treated with iodine (1.44 g, 5.69 mmol) and 2N sodium hydroxide solution (11.0 ml, 22.0 mmol) according to the method of Preparation 30 to give 820 mg (3.18 mmol, 84%) of the title compound. Purity 100%.
¹H NMR (400 MHz, DMSO-d6) δ ppm 7.40 (d, *J*=8.60 Hz, 1 H), 7.16 - 7.28 (m, 1 H), 6.86 (d, *J*=7.03 Hz, 1 H), 2.74 (s, 3 H).
UPLC/MS (3 min) retention time 1.67 min.
LRMS: m/z 259 (M+1).

### PREPARATION 70

### tert-Butyl (3-iodo-4-methyl-1H-indazol-1-yl)acetate

The title compound of Preparation 69 (300 mg, 1.16 mmol) was treated with caesium carbonate (757 mg, 2.32 mmol) and tert-butyl 2-bromoacetate (257 µl, 1.74 mmol) according to the method of Preparation 31. The resulting residue was purified using the Isolera Purification System (ether-hexane gradient, 0:100 rising to 100:0) to give 225 mg (0.60 mmol, 52%) of the title compound. Purity 97%.
¹H NMR (400 MHz, CHLOROFORM-d) δ ppm 7.26 - 7.33 (m, 1 H), 7.18 (d, *J*=8.60 Hz, 1 H), 6.93 (d, *J*=7.03 Hz, 1 H), 5.03 (s, 2 H), 2.85 (s, 3 H), 1.44 (s, 9 H).
UPLC/MS (3 min) retention time 2.03 min.
LRMS: m/z 373 (M+1).

### PREPARATION 71

### tert-Butyl (3-[2-{[(cyclopropylcarbonyl)(ethyl)amino]methyl)-4-(trifluoromethyl)phenyl]-4-methyl-1H-indazol-1-yl}acetate

The crude title compound of Preparation 70 (225 mg, 0.60 mmol) and the title compound of Preparation 10 (360 mg, 0.91 mmol) were treated with caesium carbonate solution (2M, 0.91 ml, 1.8 mmol) and [1,1'bis(diphenylphosphino)-ferrocene]dichloropalladium(II), complex with dichloromethane (50 mg, 0.06 mmol) according to the method of Preparation 32. The resulting residue was purified using the Isolera Purification System (ether-hexane gradient, 0:100 rising to 100:0) to give 278 mg (0.53 mmol, 90%) of the title compound. Purity 90%.
Partial ¹H NMR (300 MHz, CHLOROFORM-d) 60:40 mixture of two rotamers.
Major rotamer δ ppm 5.08 (s, 2 H), 3.40-3.30 (m, 2 H).
Minor rotamer δ ppm 5.10 (s, 2 H), 3.47-3.40 (m, 2 H).
UPLC/MS (3 min) retention time 2.16 min.
LRMS: m/z 516 (M+1).

### PREPARATION 72

### 3-Iodo-1H-indazole-4-carbonitrile

4-Carbonitrile-1H-indazole (400 mg, 3.78 mmol) was treated with iodine (1.06 g, 5.69 mmol) and 2N sodium hydroxide solution (8.4 ml, 16.80 mmol) according to the method of Preparation 30 to give 715 mg (2.65 mmol, 95%) of the title compound. Purity 100%
¹H NMR (300 MHz, CHLOROFORM-d) δ ppm 10.73 (br. s., 1 H), 7.80 (d, *J*.=8.51 Hz, 1 H), 7.65 (d, *J*=7.42 Hz, 1 H), 7.46 - 7.59 (m, 1 H).
UPLC/MS (3 min) retention time 1.32 min.
LRMS: m/z 270 (M+1).

### PREPARATION 73

### tert-Butyl (4-cyano-3-iodo-1H-indazol-1-yl)acetate

The title compound of Preparation 72 (250 mg, 0.93 mmol) was treated with caesium carbonate (606 mg, 1.86 mmol) and tert-butyl 2-bromoacetate (156 µl, 1.21 mmol) according to the method of Preparation 31. The resulting residue was purified using the Isolera Purification System (ether-hexane gradient, 0:100 rising to 100:0) to give 251 mg (0.65 mmol, 70%) of the title compound. Purity 97%.
¹H NMR (300 MHz, CHLOROFORM-d) δ ppm 7.46 - 7.68 (m, 3 H), 5.11 (s, 2 H), 1.45 (s, 9 H).
UPLC/MS (3 min) retention time 1.80 min.
LRMS: m/z 384 (M+1).

### PREPARATION 74

### tert-Butyl {4-cyano-3-[2-{[(cyclopropylcarbonyl)(ethyl)amino]methyl}-4-(trifluoromethyl)phenyl]-1H-indazol-1-yl}acetate

The title compound of Preparation 73 (100 mg, 0.26 mmol) and the title compound of Preparation 10 (145 mg, 0.37 mmol) were treated with caesium carbonate solution (2M, 0.39 ml, 0.78 mmol) and [1,1'-bis(diphenylphosphino)-ferrocene]dichloropalladium(II), complex with dichloromethane (22 mg, 0.03 mmol) according to the method of Preparation 32. The resulting residue was purified using the Isolera Purification System (ether-hexane gradient, 0:100 rising to 100:0) to give 130 mg (0.25 mmol, 94%) of the title compound. Purity 100%.
Partial ¹H NMR (300 MHz, CHLOROFORM-d) 50:50 mixture of two rotamers.
Rotamer 1 δ ppm 5.18 (s, 2 H), 4.70 (s, 2 H).
Rotamer 2 δ ppm 5.16 (s, 2 H), 4.69 (s, 2 H).
UPLC/MS (3 min) retention time 2.03 min.
LRMS: m/z 527 (M+1).

### PREPARATION 75

### Ethyl [5-chloro-3-(2-{[(cyclopropylcarbonyl)(ethyl)amino]methyl}-4-fluorophenyl)-1H-indazol-1-yl]acetate

The title compound of Preparation 45 (150 mg, 0.41 mmol) and the title compound of Preparation 11 (164 mg, 0.47 mmol) were treated with caesium carbonate solution (2M, 0.62 ml, 1.23 mmol) and [1,1'-bis(diphenylphosphino)ferrocene] dichloropalladium(II), complex with dichloromethane (36 mg, 0.04 mmol) according to the method of Preparation 32. The resulting residue was purified using the Isolera Purification System (ether-hexane gradient, 30:70 rising to 70:30) to give 154 mg (0.34 mmol, 82%) of the title compound as a white solid. Purity 97%.
Partial ¹H NMR (300 MHz, CHLOROFORM-d) 50:50 mixture of two rotamers.
Rotamer 1 δ ppm 5.17 (s, 2 H), 4.76 (s, 2 H).
Rotamer 2 δ ppm 5.20 (s, 2 H), 4.86 (s, 2 H).
UPLC/MS (3 min) retention time 2.06 min.
LRMS: m/z 458 (M+1).

### PREPARATION 76

### Ethyl [3-(2-{[(tert-butoxycarbonyl)(ethyl)amino]methyl}-4-fluorophenyl)-5-chloro-1H-indazol-1-yl]acetate

The title compound of Preparation 45 (540 mg, 1.48 mmol) and the title compound of Preparation 9 (675 mg, 1.78 mmol) were treated with caesium carbonate solution (2M, 2.22 ml, 4.44 mmol) and [1,1'-bis(diphenylphosphino)ferrocene] dichloropalladium(II), complex with dichloromethane (130 mg, 0.15 mmol) according to the method of Preparation 32. The resulting residue was purified using the Isolera Purification System (ether-hexane gradient, 0:100 rising to 60:40) to give 450 mg (0.92 mmol, 62%) of the title compound as a colourless oil.
Partial ¹H NMR (300 MHz, CHLOROFORM-d) 50:50 mixture of two rotamers.
Rotamer 1 δ ppm 5.18 (s, 2 H), 4.60 (s, 2 H).
Rotamer 2 δ ppm 5.18 (s, 2 H), 4.50 (s, 2 H).
UPLC/MS (3 min) retention time 2.27 min.
LRMS: m/z 490 (M+1).

### PREPARATION 77

### Ethyl (5-chloro-3-{2-[(ethylamino)methyl]-4-fluorophenyl}-1H-indazol-1-yl)acetate

The title compound of Preparation 76 (100 mg, 0.20 mmol) was dissolved in 0.8 ml dichloromethane. Trifluoroacetic acid (0.8 ml, 10.4 mmol) was added and the mixture was stirred at room temperature overnight. The solvent was evaporated under reduced pressure and the residue was dissolved in water and adjusted to pH 8 with 2N sodium hydroxide solution. The aqueous phase was extracted twice with dichloromethane, dried over sodium sulphate, filtered and evaporated under reduced pressure to give 72 mg (0.18 mmol, 90%) of the title compound as a colourless oil. Purity 96%.
¹H NMR (300 MHz, CHLOROFORM-d) δ ppm 7.68 (s, 1 H), 7.56 (dd, *J*=8.51, 5.77 Hz, 1 H), 7.38 - 7.45 (m, 1 H), 7.25 - 7.36 (m, 2 H), 7.12 (td, *J*=8.38, 2.75 Hz, 1 H), 5.17 (s, 2 H), 4.25 (q, *J*=7.14 Hz, 2 H), 3.77 (s, 2 H), 2.61 (q, *J*=7.05 Hz, 2 H), 2.03 (br. s., 1 H), 1.28 (t, *J*=7.14 Hz, 3 H), 1.09 (t, *J*=7.14 Hz, 3H).
UPLC/MS (3 min) retention time 1.56 min.
LRMS: m/z 390 (M+1).

### PREPARATION 78

### Ethyl [5-chloro-3-(2-{[(cyclobutylcarbonyl)(ethyl)amino]methyl}-4-fluorophenyl)-1H-indazol-1-yl]acetate

The title compound of Preparation 77 (35 mg, 0.090 mmol) was treated with triethylamine (15 µL, 0.11 mm1ol) and cyclobutanecarbonyl chloride (15 µL, 0.13 mmol) according to the method of Preparation 4. The resulting mixture was concentrated under reduced pressure and partitioned between ether and 4% w/v sodium bicarbonate solution. The aqueous was extracted twice with ether, washed with brine, dried over sodium sulphate, filtered and evaporated under reduced pressure to give 39 mg (0.083 mmol, 92%) of the title compound as a colourless oil. Purity 95%.
UPLC/MS (3 min) retention time 2.14 min.
LRMS:m/z472(M+1).

### PREPARATION 79

### Ethyl [5-chloro-3-(2-([ethyl(isobutyryl)amino]methyl}-4-fluorophenyl)-1H-indazol-1-yl]acetate

The title compound of Preparation 77 (30 mg, 0.077 mmol) was treated with triethylamine (13 µL, 0.09 mmol) and isobutyryl chloride (12 µL, 0.11 mmol) according to the method of Preparation 4. The resulting mixture was concentrated under reduced pressure and partitioned between ether and 4% w/v sodium bicarbonate solution. The aqueous was extracted twice with ether, washed with brine, dried over sodium sulphate, filtered and evaporated under reduced pressure to give 30 mg (0.065 mmol, 85%) of the title compound as a white solid. Purity 100%.
UPLC/MS (3 min) retention time 2.10 min.
LRMS: m/z 460 (M+1).

### PREPARATION 80

### Ethyl [6-chloro-1-(2-{[ethyl(methylsulfonyl)amino]methyl}-4-fluorophenyl)-1H-indazol-3-yl]acetate

The title compound of Preparation 77 (30 mg, 0.077 mmol) was treated with triethylamine (16 µL, 0.12 mmol) and methanesulfonyl chloride (9 µL, 0.12 mmol) according to the method of Preparation 4. The resulting mixture was concentrated under reduced pressure and partitioned between ether and 4% w/v sodium bicarbonate solution. The aqueous was extracted twice with ether, and the combined organics were washed with brine, dried over sodium sulphate, filtered and evaporated under reduced pressure to give 31 mg of the crude title compound. Used as such without further purification. Purity 78%.
UPLC/MS (3 min) retention time 2 min.
LRMS: m/z 468 (M+1).

### PREPARATION 81

### Ethyl [1(2-{[aacttyl(ethyl)amino]methyl}-4-fluorophenyl)-6-chloro-1H-indazol-3-yl]acetate

The title compound of Preparation 77 (30 mg, 0.077 mmol) was treated with triethylamine (16 µL, 0.12 mmol) and acetyl chloride (8 µL, 0.11 mmol) according to the method of Preparation 4. The resulting mixture was concentrated under reduced pressure and partitioned between ether and 4% w/v sodium bicarbonate solution. The aqueous was extracted twice with ether and the combined organics were washed with brine, dried over sodium sulphate, filtered and evaporated under reduced pressure to give 29 mg (0.067 mmol, 87%) of the title compound as a colourless oil. Purity 91%.
Partial ¹H NMR (300 MHz, CHLOROFORM-d) 60:40 mixture of two rotamers.
Major rotamer δ ppm 5.18 (s, 2 H), 4.76 (s, 2 H).
Minor rotamer δ ppm 5.19 (s, 2 H), 4.68 (s, 2 H).
UPLC/MS (3 min) retention time 1.96 min.
LRMS: m/z 4.32 (M+1).

### PREPARATION 82

### Ethyl [5-chloro-3-(2-{[(cyclopentylcarbonyl)(ethyl)amino]methyl}-4-fluorophenyl)-1H-indazol-1-yl]acetate

The title compound of Preparation 77 (30 mg, 0.077 mmol) was treated with triethylamine (16 µl, 0.12 mmol) and cyclopentanecarbonyl chloride (14 µl, 0.12 mmol) according to the method of Preparation 81 to give 40 mg of the crude title compound as a colourless oil. Purity 82%.
UPLC/MS (3 min) retention time 2.19 min.
LRMS: m/z 486 (M+1).

### PREPARATION 83

### Ethyl [5-chloro-3-(2-{[(cyclopropylacetyl)(ethyl)amino]methyl}-4-fluorophenyl)-1H-indazol-1-yl]acetate

The title compound of Preparation 77 (30 mg, 0.077 mmol) was dissolved in 0.3 ml dimethylformamide. Cyclopropylacetic acid (16 µl, 0.17 mmol), diisopropylethylamine (32 µl, 0.18 mmol) and 2-(1H-7-azabenzotriazol-1-yl)-1,1,3,3-tetramethyluronium hexafluorophosphate (HATU, 64 mg, 0.17 mmol) were added and the mixture was agitated for 4 d at room temperature. The mixture was partitioned between ethyl acetate and water. The organic phase was washed sequentially with brine and 2N hydrochloric acid. The organics were dried over sodium sulphate, filtered and evaporated under reduced pressure. The residue was purified using the Isolera purification system (ether-hexane, gradient, 0:100 rising to 100:0) to give 18 mg (0.038 mmol, 50%) of the title compound. Purity 100%.
UPLC/MS (3 min) retention time 2.10 min.
LRMS: m/z 472 (M+1).

### PREPARATION 84

### Ethyl {3-[2-{[[(benzylamino)carbonyl](ethyl)amino]methyl}-4-(trifluoromethyl)phenyl]-4-chloro-1H-indazol-1-yl}acetate

The title compound of Preparation 35 (112 mg, 0.31 mmol) and the title compound of Preparation 13 (200 mg, 0.43 mmol) were treated with caesium carbonate solution (2M, 0.46 ml, 0.92 mmol) and [1,1'-bis(diphenylphosphino)ferrocene] dichloropalladium(II), complex with dichloromethane (17 mg, 0.02 mmol) according to the method of Preparation 32. The resulting residue was purified using the Isolera Purification System (ethyl acetate-hexane gradient, 0:100 rising to 100:0) to give 59 mg (0.10 mmol, 34%) of the title compound. Purity 100%.
UPLC/MS (3 min) retention time 2.10 min.
LRMS: m/z 573 (M+1).

### PREPARATION 85

### N-(3-Fluoro-2-methylphenyl)-2,2-dimethylpropanamide

3-Fluoro-2-methylaniline (3.0 g, 24 mmol) was dissolved in 60 ml tetrahydrofuran and the mixture was cooled in an ice-bath. Triethylamine (3.7 ml, 26.5 mmol) was added followed by pivaloyl chloride (3.24 ml, 26.3 mmol), forming a thick white precipitate. The mixture was stirred for 2 h at room temperature and was then evaporated. The residue was partitioned between ethyl acetate and water. The organic phase was washed with brine, was dried over sodium sulphate, filtered and evaporated. The residue was re-crystallised from hot cyclohexane to give 4.32 g (20.6 mmol, 86%) of the title compound as colourless crystals. Purity 100%.
¹H NMR (300 MHz, CHLOROFORM-d) δ ppm 1.38 (s, 9H), 2.19 (d, J = 1.8 Hz, 3H), 6.86 (t, J = 8.8 Hz, 1H), 7.16 (q, J = 7.6 Hz, 1H), 7.32 (br s, 1H), 7.64 (d, J = 8.2 Hz, 1H).
UPLC/MS (3 min) retention time 1.48 min.
LRMS: m/z 210 (M+1).

### PREPARATION 86

### M-(4-Chloro-3-fluoro-2-methylphenyl)-2,2-dimethylpropanamide

The title compound of Preparation 85 (4.30 g, 20.5 mmol) was dissolved in 50 ml dimethylformamide under argon. N-Chlorosuccinimide (2.72 g, 20.4 mmol) was added portion-wise and the mixture was stirred at 80 ºC for 90 min. Further N-chlorosuccinimide (2.72 g, 20.4 mmol) was added and the mixture was stirred at 80 ºC for 1 h. The mixture was allowed to cool and was partitioned between ethyl acetate and water. The organic phase was washed with brine, was dried over sodium sulphate, filtered and evaporated. The residue was re-crystallised from hot cylohexane to give 2.91 g (11.9 mmol, 59%) of the title compound as white crystals. Purity 100%.
¹H NMR (300 MHz, CHLOROFORM-d) δ ppm 7.59 (dd, J = 8.8, 1.6 Hz, 1H), 7.19-7.24 (m, 2H), 2.19 (d, J = 2.1 Hz, 3H), 1.34 (s, 9H).
UPLC/MS (3 min) retention time 1.68 min.
LRMS: m/z 244 (M+1).

### PREPARATION 87

**(4-Chloro-3-fluoro-2-methylphenyl)amine**

The title compound of Preparation 86 (2.91 g, 11.9 mmol) was dissolved in 40 ml dioxane. 42 ml 6N Hydrochloric acid was added and the mixture was stirred at 100 ºC overnight. The mixture was allowed to cool and was basified by the portion-wise addition of potassium carbonate. The mixture was extracted with ethyl acetate. The organics were washes with brine, dried over sodium sulphate, filtered and evaporated under reduced pressure to give 1.54 g (9.6 mmol, 80%) of the title compound.
¹H NMR (300 MHz, CHLOROFORM-d) δ ppm 7.00 (t, J = 8.3 Hz, 1H), 6.40 (dd, J = 1.5, 8.6 Hz, 1H), 3.70 (br s, 1H), 2.10 (d, J = 2.0 Hz, 3H).
UPLC/MS (3 min) retention time 1.53 min.
LRMS: *m*/*z* 160 (M+1).

### PREPARATION 88

### 5-Chloro-4-fluoro-1H-indazole

The title compound of Preparation 87 (0.70 g, 4.4 mmol) was dissolved in 5 ml glacial acetic acid. A solution of sodium nitrite (0.34 g, 4.9 mmol) in 1 ml water was added and the mixtutre was stirred overnight at room temperature and then at 35 ºC for 2 h. The mixture was diluted with 20 ml water and was stirred for 20 min forming a white precipitate. The solid was collected by filtration and was purified using the Isolera Purification System (ethyl acetate-hexane gradient, 0:100 rising to 30:70) to give 0.21 g (1.23 mmol, 28%) of the title compound as a white solid. Purity 100%.
UPLC/MS (3 min) retention time 1.53 min.
LRMS: m/z 171 (M+1).

### PREPARATION 89

### 5-Chloro-4-fluoro-3-iodo-1H-indazole

The title compound of Preparation 88 (0.21 g, 1.23 mmol) was treated with iodine (0.37 g, 1.47 mmol) and 2N sodium hydroxide solution (2 ml, 4 mmol) according to the method of Preparation 30 to give 0.36 g (1.22 mmol, 100%) of the title compound. Purity 100%.
¹H NMR (300 MHz, CHLOROFORM-d) δ ppm 10.66 (br s, 1H), 7.40 (dd, J = 8.8, 6.5 Hz, 1H), 7.28 (d, J = 8.8 Hz, 1H).
UPLC/MS (3 min) retention time 1.80 min.
LRMS: m/z 297 (M+1).

### PREPARATION 90

### tert-Butyl (5-chloro-4-fluoro-3-iodo-1H-indazol-1-yl)acetate

The title compound of Preparation 89 (0.36 g, 1.22 mmol) was treated with caesium carbonate (0.79 g, 2.44 mmol) and *tert*-butyl 2-bromoacetate (0.20 ml, 1.35 mmol) according to the method of Preparation 31. The resulting residue was purified using the Isolera Purification System (ether-hexane gradient, 0:100 rising to 10:90) to give 167 mg (0.41 mmol, 33%) of the title compound. Purity 90%.
HPLC/MS (5 min) retention time 3.65 min.
LRMS: m/z 411 (M+1).

### PREPARATION 91

### tert-Butyl {5-chloro-3-[2-{[(cyclopropylcarbonyl)(ethyl)amino]methyl}-4-(trifluoromethyl)phenyl]-4-fluoro-1H-indazol-1-yl}acetate

The title compound of Preparation 90 (167 mg, 0.41 mmol) and the title compound of Preparation 10 (170 mg, 0.43 mmol) were treated with caesium carbonate solution (2M, 0.61 ml, 1.22 mmol) and [1,1'-bis(diphenylphosphino)ferrocene]dichloro-palladium(II), complex with dichloromethane (33 mg, 0.04 mmol) according to the method of Preparation 32. The resulting residue was purified using the Isolera Purification System (ethyl acetate-hexane gradient, 0:100 rising to 20:80) to give 156 mg (0.28 mmol, 69%) of the title compound as a yellow gum. Purity 100%.
Partial ¹H NMR (300 MHz, CHLOROFORM-d) 55:45 mixture of two rotamers.
Major rotamer δ ppm 5.08 (s, 2H), 4.80 (s, 2H), 3.45 (q, J = 7.3 Hz, 2H), 3.28 (q, J = Hz, 2H).
Minor rotamer δ ppm 5.11 (s, 2 H), 4.83 (s, 2H), 3.36 (q, J = 6.6 Hz, 2H).
HPLC/MS (3 min) retention time 2.23 min.
LRMS: m/z 554 (M+1).

### PREPARATION 92

### Ethyl 1H-indol-3-ylacetate

Indol-3-ylacetic acid (1.00 g, 5.71 mmol) was dissolved in 25 ml ethanol and concentrated sulphuric acid (1.0 ml, 18.4 mmol) was added. The mixture was stirred at reflux for 2 h and cooled to 0 ºC in an ice bath before neutralization with 2N sodium hydroxide solution. The organics were evaporated under reduced pressure and the mixture was partitioned between dichloromethane and water. The aqueous phase was extracted twice with dichloromethane and the combined organic were washed with brine, dried over sodium sulphate, filtered and evaporated under reduced pressure to give 1.02 g (5.02 mmol, 88%) of the title compound as a brown oil. Purity 100%.
¹H NMR (300 MHz, CHLOROFORM-d) δ ppm 8.09 (br. s., 1 H), 7.63 (d, *J*=7.69 Hz, 1 H), 7.35 (d, *J*=7.69 Hz, 1 H), 7.04 - 7.25 (m, 3 H), 4.17 (q, *J*=7.14 Hz, 2 H), 3.77 (s, 2 H), 1.26 (t, *J*=7.14 Hz, 3 H).
UPLC/MS (3 min) retention time 1.58 min.
LRMS: m/z 204 (M+1).

### PREPARATION 93

### Ethyl {1-[2-{[(cyclopropylcarbonyl)(ethyl)amino]methyl}-4-(trifluoromethyl)phenyl]-1H-indol-3-yl}acetate

The title compound of Preparation 4 (150 mg, 0.43 mmol) and the title compound of Preparation 92 (130 mg, 0.64 mmol) were dissolved in 2 ml acetonitrile. Salicylaldoxime (3 mg, 0.02 mmol), caesium carbonate (314 mg, 0.96 mmol) and copper(I) oxide (4 mg, 0.03 mmol) were added under argon atmosphere. The mixture was heated in the microwave for 4 h at 120 °C. Further salicylaldoxime (8 mg, 0.06 mmol), copper(I) oxide (6 mg, 0.04 mmol) and acetonitrile (0.5 ml) were added and the mixture was heated in the microwave for 20 h at 120 ºC. The mixture was filtered through Celite, washed with ethyl acetate and evaporated under reduced pressure. The residue was partially purified using the Isolera Purification System (ether-hexane gradient, 0:100 rising to 100:0, then ethanol-ether gradient, 0-100 rising to 100:0) to give 21 mg of the crude title compound. Purity 83%.
UPLC/MS (3 min) retention time 2.12 min.
LRMS: m/z 473 (M+1).

### PREPARATION 94

### N-Ethyl-N-[2-(6-fluoro-1H-indol-1-yl)-5-(trifluoromethyl)benzyl]cyclopropane-carboxamide

The title compound of Preparation 4 (300 mg, 0.86 mmol) was treated with 6-fluoro-1H-indole (173 mg, 1.28 mmol), salicylaldoxime (4.0 mg, 0.03 mmol), caesium carbonate (558 mg, 1.71 mmol) and copper(I) oxide (5.0 mg, 0.03 mmol) according to the method of Preparation 93. The mixture obtained was diluted with ethyl acetate, filtered through celite and evaporated to dryness under reduced pressure. The resulting residue was purified using the Isolera Purification System (ether-hexane gradient, 0:100 rising to 15:85) to give 191 mg (0.47 mmol, 55%) of the title compound as a pale yellow oil. Purity 96%.
Partial ¹H NMR (400 MHz, CHLOROFORM-d) 60:40 mixture of two rotamers.
Major rotamer δ ppm 4.50 (d, *J*=16 Hz, 1H), 4.21 (d, *J*= 16 Hz, 1 H), 3.33 (m, 2 H).
Minor rotamer δ ppm 4.38 (m, 2 H), 3.24 (m, 2 H).
UPLC/MS (3 min) retention time 2.09 min.
LRMS: m/z 405 (M+1).

### PREPARATION 95

### N-[2-(3-Bromo-6-fluoro-1H-indol-1-yl)-5-(trifluoromethyl)benzyl]-N-ethylcyclopropanecarboxamide

The title compound of Preparation 94 (156 mg, 0.39 mmol) was dissolved in ethyl acetate and cooled to 0 ºC in an ice-bath. N-Bromosuccinimide (76 mg, 0.43 mmol) was added and the mixture was stirred at 0 ºC for 90 min. The mixture was diluted with water and was extracted twice with ethyl acetate. The combined organics were dried over sodium sulphate, filtered and evaporated under reduced pressure. The residue obtained was purified using the Isolera Purification System (ether-hexane gradient, 0:100 rising to 10:90) to give 96 mg (0.20 mmol, 51%) of the title compound as a colourless oil. Purity 90%.
Partial ¹H NMR (300 MHz, CHLOROFORM-d) 75:25 mixture of two rotamers.
Major rotamer δ ppm 4.50 (d, *J*= 15 Hz, 1 H), 4.25 (d, *J*=15 Hz, 1 H), 3.35 (m, 2 H).
Minor rotamer δ ppm 4.42 (s, 2 H), 3.35 (m, 2 H).
UPLC/MS (3 min) retention time 2.21 min.
LRMS: m/z 483, 485 (M+1).

### PREPARATION 96

### tert-Butyl {1-[2-{[(cyclopropylcarbonyl)(ethyl)amino]methyl}-4-(trifluoromethyl)phenyl]-6-fluoro-1H-indol-3-yl}acetate

The title compound of Preparation 95 (96 mg, 0.20 mmol), (2-tert-butoxy-2-oxoethyl)zinc(II) chloride (0.5 M in ether 1.2 ml, 0.6 mmol), 1,2,3,4,5-pentaphenyl-1'-(di-tert-butylphosphino)ferrocene (5.0 mg, 0.01 mmol) and bis(dibenzylideneacetone)-palladium (6.0 mg, 0.01 mmol) were mixed together under argon atmosphere. The mixture was heated in the microwave for 1 h at 100 ºC. The mixture was partitioned between water and ether and the aqueous layer was extracted twice with ether. The combined organics were washed with brine, dried over sodium sulphate, filtered and evaporated under reduced pressure. The residue was purified using the Isolera Purification System (ether-hexane gradient, 0:100 rising to 70:30) to give 72 mg (0.14 mmol, 69%) of the title compound as a beige solid. Purity 97%.
Partial ¹H NMR (300 MHz, CHLOROFORM-d) 65:35 mixture of two rotamers.
Major rotamer δ ppm 4.51 (d, *J*= 15 Hz, 1 H), 4.23 (d, *J*= 15 Hz, 1 H), 3.71 (s, 2 H).
Minor rotamer δ ppm 4.40 (s, 2 H), 3.71 (s, 2 H).
UPLC/MS (3 min) retention time 2.23 min.
LRMS: m/z 519 (M+1).

### PREPARATION 97

### Ethyl 1H-indol-1-ylacetate

1H-Indole (1.0 g, 8.5 mmol) was dissolved in 50 ml acetonitrile. Caesium carbonate (4.1 g, 12.6 mmol), potassium iodide (0.28 g, 1.7 mmol) and ethyl 2-bromoacetate (2.8 ml, 25 mmol) were added and the mixture was stirred at room temperature overnight. The mixture was evaporated to dryness and was re-suspended in ethyl acetate. The organic phase was washed three times with water, dried over sodium sulphate, filtered and evaporated under reduced pressure. The residue was purified by reverse-phase chromatography using the SP1 Purification system to give 0.64 g (3.0 mmol, 35%) of the title compound as a pale yellow oil.
¹H NMR (partial, 400 MHz, DMSO-d₆) δ ppm 7.55 (1 H, s), 7.46 (1 H, d, *J*=8.2 Hz), 7.41 (1 H, d, *J*=7.8 Hz), 7.22 (1 H, t, *J*=7.2 Hz), 7.15 (1 H, t, *J*=7.2 Hz), 5.14 (2 H, s), 4.13 (2 H, q, *J*=7.0 Hz), 1.19 (3 H, t, *J*=7.0 Hz).

### PREPARATION 98

### Ethyl (3-bromo-1H-indol-1-yl)acetate

The title compound of Preparation 97 (0.50 g, 2.5 mmol) was dissolved in 10 ml dimethylformamide. N-Bromosuccinimide (0.54 g, 3.0 mmol) was added and the mixture was stirred for 90 min at room temperature. The mixture was partitioned between ethyl acetate and water. The organic phase was washed several times with saturated sodium bicarbonate solution and was dried over sodium sulphate, filtered and evaporated under reduced pressure. The residue was purified by reverse-phase chromatography using the SP1 Purification system to give 0.63 g of the crude title compound as a solid. Used directly to avoid degradation.
¹H NMR (400 MHz, CHLOROFORM-d) δ ppm 7.55 - 7.73 (1 H, m), 7.15 - 7.48 (4 H, m), 4.89 (1 H, d, *J*=17.0 Hz), 4.84 (1 H, d, *J*=17.0 Hz), 4.08 - 4.41 (2 H, m), 1.21 - 1.43 (3 H, m).

### PREPARATION 99

### Ethyl {3-[2-{[(cyclopropylcarbonyl)(ethyl)amino]methyl}-4-(trifluoromethyl)phenyl]-1H-indol-1-yl}acetate

### Method A

The crude title compound of Preparation 98 (100 mg) was dissolved in 10 ml toluene and 5 ml ethanol. The title compound of Preparation 10 (116 mg, 0.29 mmol), sodium carbonate (59 mg, 0.56 mmol) and [1,1'-bis(diphenylphosphino)-ferrocene] dichloropalladium(II), complex with dichloromethane (11 mg, 0.013 mmol) were added and the mixture was stirred at 85 °C for 2 h. The mixture was evaporated to dryness and was re-suspended in ethyl acetate. The organic phase was washed sequentially with water and brine and was dried over sodium sulphate, filtered and evaporated under reduced pressure. The residue was purified by flash chromatography (ethyl acetate-hexane gradient, 0:100 rising to 100:0) to give 10 mg (0.020 mmol, 5% over two steps) of the title compound as a solid. Purity 95%.

### Method B

The title compound of Preparation 103 (100 mg, 0.26 mmol) was dissolved in 5 ml dimethylformamide and the mixture was cooled in an ice-bath. Sodium hydride (60% dispersion in oil, 11 mg, 0.28 mmol) was added and the mixture was stirred for 15 min. Ethyl 2-bromoacetate (52 mg, 0.31 mmol) was added and the mixture was stirred overnight, warming to room temperature. The mixture was partitioned between ethyl acetate and water. The organic phase was dried over sodium sulphate, filtered and evaporated under reduced pressure. The residue was purified by reverse-phase chromatography using the SP1 Purification system to give 40 mg (0.08 mmol, 31%) of the desired compound as a pale yellow oil. Purity 95%.
UPLC/MS (3 min) retention time 2.09 min.
LRMS: *m*/*z* 473 (M+1).

### PREPARATION 100

### Ethyl (2-methyl-1H-indol-1-yl)acetate

2-Methyl-1H-indole (1.0 g, 7.6 mmol) was treated with caesium carbonate (4.0 g, 12.3 mmol), potassium iodide (0.30 g, 1.8 mmol) and ethyl 2-bromoacetate (3.0 ml, 27 mmol) according to the method of Preparation 97 to give 0.74 g (3.4 mmol, 45%) of the title compound as a pale yellow oil. Purity 95%.
UPLC/MS (3 min) retention time 1.73 min.
LRMS: m/z 218 (M+1).

### PREPARATION 101

### Ethyl (3-bromo-2-methyl-1H-indol-1-yl)acetate

The title compound of Preparation 100 (0.84 g, 3,9 mmol) was treated with N-bromosuccinimide (0.75 g, 4.2 mmol) according to the method of Preparation 98 to give 0.54 g (1.83 mmol, 47%) of the title compound as a grey solid. Purity 95%.
UPLC/MS (3 min) retention time 1.93 min.
LRMS: m/z 295 (M-1).

### PREPARATION 102

### Ethyl {3-[2-{[(cyclopropylcarbonyl)(ethyl)amino]methyl}-4-(trifluoromethyl)phenyl]-2-methyl-1H-indol-1-yl}acetate

The title compound of Preparation 101 (200 mg, 0.68 mmol) was dissolved in 10 ml dioxane under an atmosphere of nitrogen. The title compound of Preparation 10 (300 mg, 0.76 mmol), sodium carbonate solution (2N, 1.0 ml, 2.0 mmol) and [1,1'-bis(diphenylphosphino)ferrocene] dichloropalladium(II), complex with dichloromethane (27 mg, 0.033 mmol) were added and the mixture was microwaved with stirring at 100°C for 4 h. The mixture filtered and was evaporated to dryness. The residue was partitioned between ethyl acetate and water. The organic phase was washed sequentially with water and was dried over sodium sulphate, filtered and evaporated under reduced pressure. The residue was purified by flash chromatography (ethyl acetate-hexane gradient, 0:100 rising to 100:0) to give 50 mg (0.10 mmol, 15%) of the title compound as a solid. Purity 98%.
UPLC/MS (3 min) retention time 2.10 min.
LRMS: m/z 485 (M-1).

### PREPARATION 103

### N-Ethyl-N-[2-(1H-indol-3-yl)-5-(trifluoromethyl)benzyl]cyclopropanecarboxamide

The title compound of Preparation 14 (150 mg, 0.46 mmol) was suspended in 10 ml ethanol. Phenyl hydrazine hydrochloride (60 mg, 0.42 mmol) was added followed by 15 ml concentrated hydrochloric acid and the mixture was stirred at 90 ºC for 1 h. The organic solvents were evaporated under reduced pressure and the remaining aqueous phase was extracted three times with ethyl acetate. The combined organics were dried over sodium sulphate, filtered and evaporated under reduced pressure to give 100 mg (0.24 mmol, 58%) of the title compound as a yellow oil. Purity 95%.
UPLC/MS (3 min) retention time 2.02 min.
LRMS: *m*/*z* 387 (M+1).

### PREPARATION 104

### N-Ethyl-N-[2-(5-fluoro-1H-indol-3-yl)-5-(trifluoromethyl)benzyl]cyclopropanecarboxamide

The title compound of Preparation 14 (150 mg, 0.46 mmol) was treated with 4-fluorophenyl hydrazine hydrochloride (61 mg, 0.37 mmol) according to the method of Preparation 103 to give 110 mg (0.27 mmol, 75%) of the title compound as a yellow oil. Purity 100%.
UPLC/MS (3 min) retention time 2.02 min.
LRMS: m/z 405 (M+1).

### PREPARATION 105

### Ethyl {3-[2-{[(cyclopropylcarbonyl)(ethyl)amino]methyl}-4-(trifluoromethyl)phenyl]-5-fluoro-1H-indol-1-yl}acetate

The title compound of Preparation 104 (110 mg, 0.27 mmol) was treated with sodium hydride (60% dispersion in oil, 10 mg, 0.25 mmol) and ethyl 2-bromoacetate (55 mg, 0.33 mmol) according to Method B of Preparation 99 to give 35 mg (0.070 mmol, 26%) of the title compound as a colourless oil. Purity 98%.
Partial ¹H NMR (400 MHz, CHLOROFORM-d) 65:35 mixture of two rotamers.
Major rotamer δ ppm 4.88 (2 H, s), 4.71 (2 H, s), 3.32 (2 H, q, *J*=7.3 Hz), 0.75 - 0.84 (2 H, m).
Minor rotamer δ ppm 4.92 (2 H, s), 4.68 (2 H, s), 3.41 (2 H, q, *J*=7.0 Hz), 0.63 - 0.72 (2 H, m).
UPLC/MS (3 min) retention time 2.09 min.
LRMS: *m*/*z* 491 (M+1).

### PREPARATION 106

### N-[2-(4-Chloro-1H-indol-3-yl)-5-(trifluoromethyl)benzyl]-N-ethylcyclopropanecarboxamide

The title compound of Preparation 14 (150 mg, 0.44 mmol) was treated with 3-chlorophenyl hydrazine hydrochloride (63 mg, 0.35 mmol) according to the method of Preparation 103 followed by reverse-phase chromatography using the SP1 Purification system to give 24 mg (0.057 mmol, 13%) of the title compound as a yellow oil. Purity 99%.
Partial ¹H NMR (600 MHz, CHLOROFORM-d) 54:46 mixture of two rotamers.
Major rotamer δ ppm 8.83 (s, 1H), 4.61 (1 H, d, *J*=16.0 Hz), 4.54 (1 H, d, *J*= 16.0 Hz), 3.35 (m, 2H).
Minor rotamer δ ppm 8.68 (s, 1H), 4.72 (1 H, d, *J*=18.2 Hz), 4.39 (1 H, d, *J=*17.6 Hz), 3.48 (m, 2H).
UPLC/MS (3 min) retention time 2.08 min.
LRMS: m/z 421 (M+1).

### PREPARATION 107

### Ethyl {4-chloro-3-[2-{[(cyclopropylcarbonyl)(ethyl)amino]methyl}-4-(trifluoromethyl)phenyl]-1H-indol-1-yl}acetate

The title compound of Preparation 106 (24 mg, 0.057 mmol) was treated with sodium hydride (60% dispersion in oil, 2 mg, 0.050 mmol) and ethyl 2-bromoacetate (8 µl, 0.33 mmol) according to Method B of Preparation 99 to give 25 mg (0.048 mmol, 96%) of the title compound. Purity 98%.
UPLC/MS (3 min) retention time 2.13 min.
LRMS:m/z 507 (M+1).

### PREPARATION 108

### N-[2-(6-Chloro-1H-indol-3-yl)-5-(trifluoromethyl)benzyl]-N-ethylcyclopropanecarboxamide

Reverse-phase chromatography using the SP1 Purification of the reaction mixture of Preparation 106 also gave 35 mg (0.083 mmol, 24%) of the title compound as a yellow oil. Purity 99%.
Partial ¹H NMR (400 MHz, CHLOROFORM-d) 64:36 mixture of two rotamers.
Major rotamer δ ppm 4.67 (s, 2H), 3.36 - 3.30 (m, 2H).
Minor rotamer δ 4.65 (s, 2H), 3.40 (m, 2H).
UPLC/MS (3 min) retention time 2.12 min.
LRMS: m/z 421 (M+1).

### PREPARATION 109

### Ethyl {6-chloro-3-[2-{[(cyclopropylcarbonyl)(ethyl)amino]methyl}-4-(trifluoromethyl)phenyl]-1H-indol-1-yl}acetate

The title compound of Preparation 108 (35 mg, 0.083 mmol) was treated with sodium hydride (60% dispersion in oil, 4 mg, 0.10 mmol) and ethyl 2-bromoacetate (15 µl, 0.13 mmol) according to Method B of Preparation 99 to give 23 mg (0.045 mmol, 54%) of the title compound as a yellow oil. Purity 98%.
UPLC/MS (3 min) retention time 2.16 min.
LRMS:m/z 507 (M+1).

### PREPARATION 110

### N-[2-(5-Cyano-1H-indol-3-yl)-5-(trifluoromethyl)benzyl]-N-ethylcyclopropanecarboxamide

The title compound of Preparation 14 (400 mg, 1.17 mmol) was suspended in 2 ml ethanol. 4-Cyanophenyl hydrazine hydrochloride (200 mg, 1.18 mmol) was added followed by 2 ml concentrated hydrochloric acid and the mixture was stirred at 80 ºC for 1 h. The organic solvents were evaporated under reduced pressure and the remaining aqueous phase was extracted three times with ethyl acetate. The combined organics were dried over sodium sulphate, filtered and evaporated under reduced pressure to give 460 mg of the crude title compound. Purity 75%.
UPLC/MS (3 min) retention time 1.89 min.
LRMS:m/z 410 (M-1).

### PREPARATION 111

### Ethyl {5-cyano-3-[2-{[(cyclopropylcarbonyl)(ethyl)amino]methyl}-4-(trifluoromethyl)phenyl]-1H-indol-1-yl}acetate

The crude title compound of Preparation 110 (230 mg) was treated with sodium hydride (60% dispersion in oil, 15 mg, 0.38 mmol) and ethyl 2-bromoacetate (100 mg, 0.61 mmol) according to Method B of Preparation 99 to give 60 mg (0.12 mmol, 20% over two steps) of the title compound as a yellow oil. Purity 98%.
UPLC/MS (3min) retention time 1.98 min.
LRMS: m/z 498 (M+1).

### PREPARATION 112

### N-Ethyl-N-[5-fluoro-2-(1H-indol-3-yl)benzyl]cyclopropanecarboxamide

The title compound of Preparation 16 (150 mg, 0.51 mmol) was treated with phenyl hydrazine hydrochloride (60 mg, 0.41 mmol) according to the method of Preparation 110 to give 130 mg (0.39 mmol, 95%) of the title compound as a yellow oil. Purity 97%.
UPLC/MS (3 min) retention time 1.89 min.
LRMS:m/z 337 (M+1).

### PREPARATION 113

### Ethyl [3-(2-{[(cyclopropylcarbonyl)(ethyl)amino]methyl}-4-fluorophenyl)-1H-indol-1-yl]acetate

The title compound of Preparation 112 (130 mg, 0.37 mmol) was treated with sodium hydride (60% dispersion in oil, 20 mg, 0.50 mmol) and ethyl 2-bromoacetate (105 mg, 0.63 mmol) according to Method B of Preparation 99 to give 54 mg (0.13 mmol, 35%) of the title compound as a yellow oil. Purity 97%.
UPLC/MS (3 min) retention time 2.00 min.
LRMS:m/z 423 (M+1).

### PREPARATION 114

### N-[2-(5-Bromo-1H-indol-3-yl)-5-(trifluoromethyl)benzyl]-N-ethylcyclopropanecarboxamide

The title compound of Preparation 14 (300 mg, 0.92 mmol) was treated with 4-bromophenyl hydrazine hydrochloride (130 mg, 0.58 mmol) according to the method of Preparation 110 to give 390 mg of the crude title compound. Purity 75%.
UPLC/MS (3 min) retention time 2.16 min.
LRMS:m/z 465, 467 (M+1).

### PREPARATION115

### Ethyl {5-bromo-3-[2-{[(cyclopropylcarbonyl)(ethyl)amino]methyl}-4-(trifluoromethyl)phenyl]-1H-indol-1-yl}acetate

The crude title compound of Preparation 114 (390 mg) was treated with sodium hydride (60% dispersion in oil, 23 mg, 0.57 mmol) and ethyl 2-bromoacetate (160 mg, 0.96 mmol) according to Method B of Preparation 99 to give 125 mg of the crude title compound. Purity 75%.
UPLC/MS (3 min) retention time 2.19 min.
LRMS:m/z 551, 553 (M+1).

### PREPARATION 116

### Ethyl {5-cyclopropyl-3-[2-{[(cyclopropylcarbonyl)(ethyl)amino]methyl}-4-(trifluoromethyl)phenyl]-1H-indol-1-yl}acetate

The crude title compound of Preparation 115 (125 mg) was suspended in 2 ml toluene and 1 ml water. Cyclopropylboronic acid (60 mg, 0.70 mmol) and potassium carbonate (94 mg, 0.68 mmol) were added and the mixture was submitted to three vacuum-argon cycles. Tetrakis(triphenylphosphine)palladium (27 mg, 0.02 mmol) was added and the mixture was submitted to three further vacuum-argon cycles. The mixture was stirred at 85 ºC for 4 h. Further cyclopropylboronic acid (30 mg, 0.35 mmol), potassium carbonate (48 mg, 0.34 mmol) and tetrakis(triphenylphosphine)palladium (14 mg, 0.01 mmol) were added and the mixture as stirred at 85 ºC overnight. The mixture was allowed to cool, was filtered through a plug of Celite and the filtrate evaporated under reduced pressure. The mixture was partitioned between ethyl acetate and water. The organic layer was washed with water, dried over sodium sulphate, filtered and evaporated under reduced pressure. The residue was purified by flash chromatography (ethyl acetate-hexane gradient, 0:100 rising to 60:40) to give 20 mg (0.039 mmol, 7% over three steps) of the title compound as a colourless oil. Purity 98%.
UPLC/MS (3 min) retention time 2.20 min.
LRMS:m/z 513 (M+1).

### PREPARATION 117

### N-Ethyl-N-{5-fluoro-2-[5-(trifluoromethyl)-1H-indol-3-yl]benzyl}cyclopropanecarboxamide

The title compound of Preparation 16 (150 mg, 0.51 mmol) was treated with (4-trifluoromethyl)phenyl hydrazine (110 mg, 0.62 mmol) according to the method of Preparation 110 to give 180 mg of the crude title compound. Purity 75%.
UPLC/MS (3 min) retention time 2.04 min.
LRMS:m/z 405 (M+1).

### PREPARATION 118

### Ethyl [3-(2-{[(cyclopropylcarbonyl)(ethyl)amino]methyl}-4-fluorophenyl)-5-(trifluoromethyl)-1H-indol-1-yl]acetate

The crude title compound of Preparation 117 (180 mg) was treated with sodium hydride (60% dispersion in oil, 12 mg, 0.30 mmol) and ethyl 2-bromoacetate (89 mg, 0.53 mmol) according to Method B of Preparation 99 to give 75 mg (0.15 mmol, 30% over two steps) of the title compound. Purity 98%.
UPLC/MS (3 min) retention time 2.09 min.
LRMS:m/z 491 (M+1).

### PREPARATION 119

### N-Ethyl-N-[2-(4-fluoro-1H-indol-3-yl)-5-(trifluoromethyl)benzyl]cyclopropanecarboxamide

The title compound of Preparation 14 (150 mg, 0.46 mmol) was treated with 3-fluorophenyl hydrazine hydrochloride (65 mg, 0.40 mmol) according to the method of Preparation 110 followed by reverse-phase chromatography using the SP1 Purification system to give 20 mg (0.050 mmol, 12%) of the title compound as a yellow oil. Purity 98%.
Partial ¹H NMR (400 MHz, CHLOROFORM-d) 57:43 mixture of two rotamers.
Major rotamer δ ppm 4.69 (s, 2H).
Minor rotamer δ ppm 4.67 (s, 2H).
UPLC/MS (3 min) retention time 1.99 min.
LRMS: m/ 405 (M+1).

### PREPARATION120

### Ethyl {3-[2-{[(cyclopropylcarbonyl)(ethyl)amino]methyl}-4-(trifluoromethyl)phenyl]-4-fluoro-1H-indol-1-yl}acetate

The title compound of Preparation 119 (20 mg, 0.050 mmol) was treated with sodium hydride (60% dispersion in oil, 3 mg, 0.075 mmol) and ethyl 2-bromoacetate (10 mg, 0.060 mmol) according to Method B of Preparation 99 to give 10 mg (0.020 mmol, 40%) of the title compound. Purity 90%.
UPLC/MS (3 min) retention time 2.06 min.
LRMS: m/z 491 (M+1).

### PREPARATION 121

### N-Ethyl-N-[5-fluoro-2-(5-fluoro-1H-indol-3-yl)benzyl]cyclopropanecarboxamide

The title compound of Preparation 16 (150 mg, 0.51 mmol) was treated with 4-fluorophenyl hydrazine hydrochloride (72 mg, 0.44 mmol) according to the method of Preparation 110 to give 190 mg of the crude title compound. Purity 75%.
UPLC/MS (3 min) retention time 1.91 min.
LRMS: m/z 355 (M+1).

### PREPARATION 122

### Ethyl [3-(2-{[(cyclopropylcarbonyl)(ethyl)amino]methyl}-4-fluorophenyl)-5-fluoro-1H-indol-1-yl]acetate

The crude title compound of Preparation 121 (95 mg) was treated with sodium hydride (60% dispersion in oil, 12 mg, 0.30 mmol) and ethyl 2-bromoacetate (0.13 ml, 1.17 mmol) according to Method B of Preparation 99 to give 36 mg (0.082 mmol, 32% over two steps) of the title compound. Purity 95%.
UPLC/MS (3 min) retention time 2.00 min.
LRMS: m/z 441 (M+1).

### PREPARATION 123

### N-[2-(4-Chloro-1H-indol-3-yl)-5-fluorobenzyl]-N-ethylcyclopropanecarboxamide

The title compound of Preparation 16 (300 mg, 1.0 mmol) was treated with 3-chlorophenyl hydrazine hydrochloride (200 mg, 1.1 mmol) according to the method of Preparation 110. The crude residue obtained was partially purified by flash chromatography (ether-hexane gradient, 0:100 rising to 100:0) and further purified by reverse-phase chromatography using the SP1 Purification system to give 84 mg (0.23 mmol, 23%) of the title compound as a yellow oil. Purity 100%.
Partial ¹H NMR (400 MHz, CHLOROFORM-d) 50:50 mixture of two rotamers.
Rotamer 1 δ ppm 4.63 (1 H, d, *J*= 18.0 Hz), 4.32 (1 H, d, *J*=18.0 Hz), 3.56 - 3.42 (m, 1 H).
Rotamer 2 δ ppm 4.56 (1 H, d, *J*= 16.0 Hz), 4.40 (1 H, d, *J*= 16.0 Hz), 3.42 - 3.26 (m, 1H).
UPLC/MS (3 min) retention time 1.98 min.
LRMS: m/z 371 (M+1).

### PREPARATION 124

### Ethyl [4-chloro-3-(2-{[(cyclopropylcarbonyl)(ethyl)amino]methyl}-4-fluorophenyl)-1H-indol-1-yl]acetate

The title compound of Preparation 123 (84 mg, 0.23 mmol) was treated with sodium hydride (60% dispersion in oil, 28 mg, 0.70 mmol) and ethyl 2-bromoacetate (66 µl, 0.60 mmol) according to Method B of Preparation 99 but with purification by flash chromatography (ethyl acetate-hexane gradient, 0:100 rising to 100:0) to give 53 mg (0.12 mmol, 51%) of the title compound. Purity 100%.
UPLC/MS (3 min) retention time 2.05 min.
LRMS: *m*/*z* 457 (M+1).

### PREPARATION 125

### N-ethyl-N-[5-fluoro-2-(4-fluoro-1H-indol-3-yl)benzyl]cyclopropanecarboxamide

The title compound of Preparation 16 (300 mg, 1.0 mmol) was treated with 3-fluorophenyl hydrazine hydrochloride (143 mg, 0.88 mmol) according to the method of Preparation 110. The crude residue obtained was partially purified by flash chromatography (ether-hexane gradient, 0:100 rising to 100:0) and further purified by reverse-phase chromatography using the SP1 Purification system to give 58 mg (0.16 mmol, 16%) of the title compound. Purity 90%.
Partial ¹H NMR (400 MHz, CHLOROFORM-d) 50:50 mixture of two rotamers.
Rotamer 1 δ ppm 4.60 (s, 1H).
Rotamer 2 δ ppm 4.58 (s, 1H).
UPLC/MS (3 min) retention time 1.87 min.
LRMS: m/z 355 (M+1).

### PREPARATION126

### Ethyl [3-(2-{[(cyclopropylcarbonyl)(ethyl)amino]methyl}-4-fluorophenyl)-4-fluoro-1H-indol-1-yl]acetate

The title compound of Preparation 125 (58 mg, 0.16 mmol) was treated with sodium hydride (60% dispersion in oil, 20 mg, 0.50 mmol) and ethyl 2-bromoacetate (48 µl, 0.44 mmol) according to Method B of Preparation 99 but with purification by flash chromatography (ethyl acetate-hexane gradient, 0:100 rising to 100:0) to give 18 mg of the crude title compound. Purity 71%.
UPLC/MS (3 min) retention time 1.97 min.
LRMS: m/z 441 (M+1).

### PREPARATION 127

### N-[2-(5-chloro-1H-indol-3-yl)-5-fluorobenzyl]-N-ethylcyclopropanecarboxamide

The title compound of Preparation 16 (140 mg, 0.48 mmol) was treated with 4-chlorophenyl hydrazine hydrochloride (95 mg, 0.53 mmol) according to the method of Preparation 110 followed by purification by flash chromatography (ethyl acetate-hexane gradient, 0:100 rising to 40:60) to give 66 mg (0.18 mmol, 37%) of the title compound as a yellow oil. Purity 100%.
UPLC/MS (3 min) retention time 2.03 min.
LRMS:m/z 371 (M+1).

### PREPARATION 128

### Ethyl [5-chloro-3-(2-{[(cyclopropylcarbonyl)(ethyl)amino]methyl}-4-fluorophenyl)-1H-indol-1-yl]acetate

The title compound of Preparation 127 (66 mg, 0.18 mmol) was treated with sodium hydride (60% dispersion in oil, 22 mg, 0.56 mmol) and ethyl 2-bromoacetate (52 µl, 0.48 mmol) according to Method B of Preparation 99 but with purification by flash chromatography (ethyl acetate-hexane gradient, 0:100 rising to 100:0) to give 19 mg of the crude title compound. Purity 78%.
UPLC/MS (3 min) retention time 2.10 min.
LRMS: m/z 457 (M+1).

### PREPARATION 129

### Methyl 2-{3-[2-{[(cyclopropylcarbonyl)(ethyl)amino]methyl}-4-(trifluoromethyl)phenyl]-5-fluoro-1H-indol-1-yl}propanoate

The title compound of Preparation 104 (0.30 g, 0.74 mmol) was treated with caesium carbonate (0.60 g, 1.84 mmol) and the crude title compound of Preparation 2 (0.20 g) according to the method of Preparation 31. The resulting residue was partially purified using the Isolera Purification System (ethyl acetate-hexane gradient, 0:100 rising to 50:50) and then by reverse-phase chromatography using the SP1 Purification system to give 73 mg (0.15 mmol, 20%) of the title compound as a colourless oil. Purity 95%.
UPLC/MS (3 min) retention time 2.09 min.
LRMS: *m*/*z* 491 (M+1).

### PREPARATION 130

### Methyl 2-{4-chloro-3-[2-{[(cyclopropylcarbonyl)(ethyl)amino]methyl}-4-(trifluoromethyl)phenyl]-1H-indol-1-yl}propanoate

The title compound of Preparation 106 (60 mg, 0.14 mmol) was treated with caesium carbonate (120 mg, 0.37 mmol) and the crude title compound of Preparation 2 (40 mg) according to the method of Preparation 31. The resulting residue was purified using the Isolera Purification System (ethyl acetate-hexane gradient, 0:100 rising to 30:70) to give 35 mg (0.069 mmol, 48%) of the title compound as a colourless oil. Purity 100%.
Partial ¹H NMR (300 MHz, CHLOROFORM-d) 50:50 mixture of two rotamers.
Rotamer 1 δ ppm 5.23 (1 H, q, *J*=7.0 Hz), 4.71 (1 H, dd, *J*=18.0, 7.0 Hz), 4.37(1 H, dd, *J*= 18.0, 3.9 Hz).
Rotamer 2 δ ppm 5.18 (1 H, dq, *J*=7.2, 2.9 Hz), 4.61 (1 H, dd, *J*=16.0, 3.0 Hz).
UPLC/MS (3 min) retention time 2.13 min.
LRMS: m/z 507 (M+1).

### PREPARATION 131

### N'-benzyl-N-ethyl-N-[2-(5-fluoro-1H-indol-3-yl)-5-(trifluoromethyl)benzyl]urea

The title compound of Preparation 15 (130 mg, 1.0 mmol) was treated with 4-fluorophenyl hydrazine hydrochloride (44 mg, 1.1 mmol) according to the method of Preparation 110 to give 210 mg of the crude title compound. Purity 40%.
UPLC/MS (3 min) retention time 2.04 min.
LRMS:m/z470(M+1).

### PREPARATION 132

### Ethyl {3-[2-{[[(benzylamno)carbonyl](ethyl)amino]methyl}-4-(trifluoromethyl)phenyl]-5-fluoro-1H-indol-1-yl}acetate

The crude title compound of Preparation 131 (210 mg) was treated with sodium hydride (60% dispersion in oil, 27 mg, 0.68 mmol) and ethyl 2-bromoacetate (90 mg, 0.54 mmol) according to Method B of Preparation 99 to give 105 mg (0.19 mmol, 19% over two steps) of the title compound as a colourless oil. Purity 97%.
UPLC/MS (3 min) retention time 2.09 min.
LRMS:m/z556(M+1).

### PREPARATION 133

### 3-[2-[(3-Cyclopropyl-5-methyl-4H-1,2,4-triazol-4-yl)methyl]-4-(trifluoromethyl)phenyl]-5-fluoro-1H-indole

The crude title compound of Preparation 21 (0.45 g) was suspended in 6 ml ethanol. Phenyl hydrazine hydrochloride (0.25 g, 2.0 mmol) was added followed by 6 ml concentrated hydrochloric acid and the mixture was stirred at 100 ºC for 3 h. The organic solvents were evaporated under reduced pressure and the remaining aqueous phase was extracted three times with ethyl acetate. The combined organics were dried over sodium sulphate, filtered and evaporated under reduced pressure. The residue was purified using the Isolera Purification system (ethyl acetate-hexane gradient, 0:100 rising to 100:0) to give 0.26 g (0.63 mmol, 45% over two steps) of the title compound as a pale brown oil. Purity 90%.
UPLC/MS (3 min) retention time 1.81 min.
LRMS: m/z 415 (M+1).

### PREPARATION 134

### Ethyl {3-[2-[(3-cyclopropyl-5-methyl-4H-1,2,4-triazol-4-yl)methyl]-4-(trifluoromethyl)phenyl]-5-fluoro-1H-indol-1-yl}acetate

The title compound of Preparation 133 (0.26 g, 0.63 mmol) was treated with sodium hydride (60% dispersion in oil, 30 mg, 0.75 mmol) and ethyl 2-bromoacetate (130 mg, 0.78 mmol) according to Method B of Preparation 99 to give 60 mg (0.12 mmol, 19%) of the title compound as a colourless oil. Purity 98%.
UPLC/MS (3 min) retention time 1.90 min.
LRMS: m/z 501 (M+1).

### PREPARATION 135

### N-[2-(4-Chloro-5-fluoro-1H-indol-3-yl)-5-(trifluoromethyl)benzyl]-N-ethylcyclopropanecarboxamide

The title compound of Preparation 14 (150 mg, 0.44 mmol) was treated with 3-chloro-4-fluorophenyl hydrazine hydrochloride (88 mg, 0.45 mmol) according to the method of Preparation 103 followed by reverse-phase chromatography using the SP1 Purification system to give 25 mg (0.056 mmol, 13%) of the title compound. Purity 98%.
Martial ¹H NMR (400 MHz, CHLOROFORM-d) 60:40 mixture of two rotamers.
Major rotamer δ ppm 9.69 (1 H, br. s.), 7.20 (1 H, dd, *J*=9.0, 3.9 Hz), 4.61 (1 H, d, *J*=17.0 Hz), 4.56 (1 H, d, *J*=17.0 Hz), 1.69 - 1.79 (1 H, m), 1.10 (3 H, t, *J*=7.2 Hz).
Minor rotamer δ ppm 1 H NMR (400 MHz, CHLOROFORM-d) d ppm 9.49 (1 H, br. s.), 7.30 (1 H, dd, *J*=8.8, 3.7 Hz), 4.70 (1 H, d, *J*=18.0 Hz), 4.43 (1 H, d, *J*=18.0 Hz), 1.48 - 1.58 (0 H, m), 1.26 (3 H, t, *J*=7.0 Hz), 1.10 (3 H, t, *J*=7.2 Hz).
UPLC/MS (3 min) retention time 2.12 min.
LRMS: m/z 439 (M+1).

### PREPARATION 136

### Ethyl {4-chloro-3-[2-{[(cyclopropylcarbonyl)(ethyy)amino]methyl)-4-(trifluoromethyl)phenyl]-5-fluoro-1H-indol-1-yl}acetate

The title compound of Preparation 135 (25 mg, 0.057 mmol) was treated with sodium hydride (60% dispersion in oil, 3 mg, 0.075 mmol) and ethyl 2-bromoacetate (12 mg, 0.072 mmol) according to Method B of Preparation 99 to give 25 mg (0.048 mmol, 84%) of the title compound as a colourless oil. Purity 90%.
UPLC/MS (3 min) retention time 2.16 min.
LRMS: m/z 525 (M+1).

### PREPARATION 137

### N-[2-(4-Chloro-5-fluoro-1H-indol-3-yl)-5-fluorobenzyl]-N-ethylcyclopropanecarboxamide

The title compound of Preparation 16 (0.50 g, 1.80 mmol) was treated with 3-chloro-4-fluorophenyl hydrazine (0.35 g, 2.18 mmol) according to the method of Preparation 103 followed by reverse-phase chromatography using the SP1 Purification system to give 120 mg (0.31 mmol, 17%) of the title compound. Purity 100%.
Partial ¹H NMR (300 MHz, CHLOROFORM-d) 58:42 mixture of two rotamers.
Major rotamer δ ppm 9.25 (1 H, br. s.), 4.57 (1 H, d, *J*=17.0 Hz), 4.34 (1 H, d, *J*=17.0 Hz), 1.09 (3 H, t, *J*=7.0 Hz).
Minor Rotamer δ ppm 8.95 (1 H, br. s.), 4.60 (1 H, d, *J*=18.0 Hz), 4.38 (1 H, d, *J*=18.0 Hz), 0.99 (3 H, t, *J*=7.0 Hz).
UPLC/MS (3 min) retention time 2.03 min.
LRMS: m/z 389 (M+1).

### PREPARATION 138

### Ethyl [4-chloro-3-(2-{[(cyclopropylcarbonyl)(ethyl)amino]methyl}-4-fluorophenyl)-5-fluoro-1H-indol-1-yl]acetate

The title compound of Preparation 137 (112 mg, 0.29 mmol) was treated with sodium hydride (60% dispersion in oil, 65 mg, 1.63 mmol) and ethyl 2-bromoacetate (0.16 ml, 1.44 mmol) according to Method B of Preparation 99 to give 45 mg (0.095 mmol, 33%) of the title compound. Purity 100%.
Partial ¹H NMR (300 MHz, CHLOROFORM-d) 65:35 mixture of two rotamers.
Major rotamer δ ppm 4.84 (2 H, s), 4.59 (2 H, br. s.), 4.12 (1 H, q)
Minor Rotamer δ ppm 4.87 (2 H, br. s.), 4.56 (2 H, br. s.).
UPLC/MS (3 min) retention time 2.09 min.
LRMS: m/z 475 (M+1).

### PREPARATION 139

### N-Ethyl-N-{[2-(5-fluoro-1H-indol-3-yl)pyridin-3-yl]methyl}cyclopropanecarboxamide

The title compound of Preparation 24 (0.22 g, 0.80 mmol) was dissolved in 4 ml isopropanol. 4-Fluorophenyl hydrazine hydrochloride (0.10 g, 0.62 mmol) was added and the mixture was heated to reflux. Concentrated sulphuric acid (0.06 ml, 0.12 mmol) was added drop-wise and the mixture was stirred at reflux overnight. The mixture was allowed to cool and was diluted with water and was basified with saturated sodium bicarbonate solutions. The aqueous was extracted twice with dichloromethane and the combined organics were dried over sodium sulphate, filtered and evaporated under reduced pressure. The residue was purified using the Isolera purification system (methanol-dichloromethane, gradient, 0:100 rising to 5:95) to give 65 mg (0.19 mmol, 31%) of the title compound as a brown solid. Purity 90%.
Partial ¹H NMR (400 MHz, CHLOROFORM-d) 57:43 mixture of two rotamers.
Major rotamer δ ppm 8.56 (d, *J*=3.5 Hz, 1H), 4.71 (s, 2H).
Minor rotamer δ ppm 8.61 (d, *J*=4.6 Hz, 1H), 4.95 (s, 2H).
UPLC/MS (3 min) retention time 1.24 min.
LRMS: m/z 338 (M+1).

### PREPARATION 140

### Ethyl [3-(3-{[(cyclopropylcarbonyl)(ethyl)amino]methyl}pyridin-2-yl)-5-fluoro-1H-indol-1-yl]acetate

The title compound of Preparation 139 (65 mg, 0.19 mmol) was treated with sodium hydride (60% dispersion in oil, 9 mg, 0.23 mmol) and ethyl 2-bromoacetate (25 µl, 0.22 mmol) according to Method B of Preparation 99 to give 40 mg of the crude title compound. Purity 80%.
UPLC/MS (3 min) retention time 1.56 min.
LRMS: m/z 424 (M+1).

### PREPARATION 141

### N-[2-(4,5-Difluoro-1H-indol-3-yl)-5-(trifluoromethyl)benzyl]-N-ethylcyclopropanecarboxamide

The title compound of Preparation 14 (500 mg, 1.46 mmol) was treated with 3,4-difluorophenyl hydrazine hydrochloride (320 mg, 1.77 mmol) according to the method of Preparation 103 followed by reverse-phase chromatography using the SP1 Purification system to give 120 mg (0.28 mmol, 19%) of the title compound. Purity 100%.
UPLC/MS (3 min) retention time 2.05 min.
LRMS: m/z 423 (M+1).

### PREPARATION 142

### Ethyl {3-[2-{[(cyclopropylcarbonyl)(ethyl)amino]methyl)-4-(trifluoromethyl)phenyl]-4,5-difluoro-1H-indol-1-yl}acetate

The title compound of Preparation 141 (120 mg, 0.28 mmol) was treated with sodium hydride (60% dispersion in oil, 15 mg, 0.38 mmol) and ethyl 2-bromoacetate (50 µl, 0.45 mmol) according to Method B of Preparation 99 to give 40 mg (0.079 mmol, 28%) of the title compound as a colourless oil. Purity 100%.
Partial ¹H NMR (400 MHz, DMSO-d) 70:30 mixture of two rotamers.
Major rotamer δ ppm 4.83 (2 H, s), 4.68 (2 H, s), 3.33 (2 H, q, J=7.0 Hz).
Minor rotamer δ ppm 4.87 (2 H, s), 4.66 (s H, s), 3.40 (2 H, q, J=7.0 Hz).
UPLC/MS (3 min) retention time 2.10 min.
LRMS: m/z 509 (M+1).

### PREPARATION 143

### N-[2-(5-Chloro-1H-indol-3-yl)-5-(trifluoromethyl)benzyl]-N-ethylcyclopropanecarboxamide

The title compound of Preparation 14 (300 mg, 0.88 mmol) was treated with 4-chlorophenyl hydrazine hydrochloride (150 mg, 1.05 mmol) according to the method of Preparation 103 to give 370 mg (0.88 mmol, 100%) of the title compound. Purity 97%.
UPLC/MS (3 min) retention time 2.12 min.
LRMS: m/z 421 (M+1).

### PREPARATION 144

### Ethyl {5-chloro-3-[2-{[(cyclopropylcarbonyl)(ethyl)amino]methyl}-4-(trifluoromethyl)phenyl]-1H-indol-1-yl}acetate

The title compound of Preparation 143 (370 mg, 0.88 mmol) was treated with sodium hydride (60% dispersion in oil, 50 mg, 1.25 mmol) and ethyl 2-bromoacetate (220 mg, 1.32 mmol) according to Method B of Preparation 99 to give 200 mg (0.39 mmol, 45%) of the title compound as a colourless oil. Purity 100%.
UPLC/MS (3 min) retention time 2.15 min.
LRMS: m/z 507 (M+1).

### PREPARATION 145

### 1-[(4-Methylphenyl)sulfonyl]-5-(trifluoromethyl)-1H-indole

Sodium hydride (60% dispersion in oil, 64 mg, 1.60 mmol) was suspended in 4 ml dimethylformamide under argon and was cooled in an ice-bath. A solution of 5-(trifluoromethyl)-1H-indole (250 mg, 1.35 mmol) dissolved in 2 ml dimethylformamide was added and the mixture was stirred at 0 ºC for 1 h. A solution of tosyl chloride (310 mg, 1.62 mmol) dissolved in 2 ml dimethylformamide was added and the mixture was stirred for 30 min at room temperature. The mixture was partitioned between ethyl acetate and water. The organic phase was washed with saturated sodium bicarbonate solution and was dried over sodium sulphate, filtered and evaporated under reduced pressure. The residue was purified by flash chromatography (ethyl acetate-hexane, 33:67) to give 330 mg (0.97 mmol, 72%) of the desired compound as a white solid. Purity 100%.
¹H NMR (400 MHz, CHLOROFORM-*d*) δ ppm 8.08 (d, *J*=8.60 Hz, 1 H), 7.83 (s, 1 H), 7.78 (d, *J*=8.21 Hz, 2 H), 7.68 (d, *J*=3.52 Hz, 1 H), 7.55 (d, *J*=8.79, 1 H), 7.22 - 7.29 (m, 2 H), 6.73 (d, *J*=3.91 Hz, 1 H), 2.36 (s, 3 H).
UPLC/MS (3 min) retention time 2.11 min.
LRMS: m/z 338 (M-1).

### PREPARATION 146

### [1-[(4-Methylphenyl)sulfonyl]-5-(trifluoromethyl)-1H-indol-3-yl]boronic acid

The title compound of Preparation 145 (330 mg, 0.97 mmol) was dissolved in 5 ml acetic acid. Mercuric(II) acetate (310 mg, 0.97 mmol) was added and the mixture was stirred at room temperature overnight. Perchloric acid (2 drops) were added and the mixture was stirred for 2 h, forming a white precipitate. The mixture was diluted with water and was filtered. The solid was washed with water and was dried in a stream of air to give 505 mg of white solid. The solid was dissolved in 10 ml tetrahydrofuran and borane (1M in tetrahydrofuran, 4.2 ml, 4.2 mmol) was added and the mixture was stirred for 2 h. 5 ml water was added drop-wise and with stirring. The mixture was then filtered and the filtrate was evaporated under reduced pressure. The resulting oil was triturated with hexane to give a solid. The solid was collected by filtration and was dried in a stream of air to give 210 mg (0.55 mmol, 56%) of the title compound as a white solid. Purity 100%.
¹H NMR (300 MHz, DMSO-*d*₆) δ ppm 8.43 (br.s., 1 H), 8.39 (s, 1 H), 8.28 (s, 1 H), 8.09 (d, *J*=8.80 Hz, 1 H), 7.88 (d, *J*=8.22 Hz, 2 H), 7.65 (d, *J*=8.80 Hz, 1 H), 7.43 (d, *J*=8.22 Hz, 2 H), 2.26 - 2.41 (m, 3 H).
UPLC/MS (3 min) retention time 2.03 min.

### PREPARATION 147

### N-Ethyl-N-({2-1-[(4-methylphenyl)sulfonyl]-5-(trifluoromethyl)-1H-indol-3-yl]pyridin-3-yl}methyl)cyclopropanecarboxamide

The title compound of Preparation 146 (100 mg, 0.26 mmol) and the title compound of Preparation 23 (52 mg, 0.22 mmol) were dissolved in 3 ml toluene. Sodium carbonate (365 mg, 0.73 mmol) was added and the mixture was was submitted to three vacuum-argon cycles. Tetrakis(triphenylphosphine)palladium (28 mg, 0.02 mmol) was added and the mixture was re-submitted to three vacuum-argon cycles. The mixture was then stirred at 90 ºC for 2 h. The mixture was filtered through a plug of Celite and the filtrate was diluted with ethyl acetate. The organics were washed sequentially with water and brine, dried over sodium sulphate, filtered and evaporated under reduced pressure. The residue was purified using the Isolera purification system (methanol-dichloromethane gradient, 0:100 rising to 5:95) to give 75 mg of the crude title compound. Purity 80%.
UPLC/MS (3 min) retention time 2.10 min.
LRMS: m/z 542 (M+1).

### PREPARATION 148

### N-Ethyl-N-({2-[5-(trifluoromethy)-1H-indol-3-yl]pyridin-3-yl}methyl)cyclopropaneearboxamide

The crude title compound of Preparation 147 (75 mg) was dissolved in 4 ml ethanol. 0.3 ml 4N Sodium hydroxide solution was added and the mixture was stirred at 90 ºC for 1 h. The mixture was allowed to cool and was evaporated under reduced pressure. The residue was partitioned between ethyl acetate and water and the organic phase was dried over sodium sulphate, filtered and evaporated under reduced pressure to give 55 mg of the crude title compound. Purity 80%.
UPLC/MS (3 min) retention time 1.78 min.
LRMS: m/z 388 (M+1).

### PREPARATION 149

### Ethyl [3-(3-{[(cyclopropylcarbonyl)(ethyl)amino]methyl}pyridin-2-yl)-5-(trifluoromethyl)-1H-indol-1-yl]acetate

The crude title compound of Preparation 148 (55 mg) was treated with caesium carbonate (92 mg, 0.28 mmol) and ethyl 2-bromoacetate (23 µl, 0.21 mmol) according to the method of Preparation 31. The resulting residue was purified using the Isolera Purification System (ether-hexane gradient, 0:100 rising to 100:0) to give 40 mg (0.085 mmol, 32% over three steps) of the title compound. Purity 90%.
Partial ¹H NMR (300 MHz, CHLOROFORM-d) 65:35 mixture of two rotamers.
Major rotamer δ ppm 4.93 (s, 2 H), 4.80 (s, 2H), 3.37-3.30 (m, 2 H).
Minor rotamer δ ppm 4.97 (s, 2 H), 4.80 (s, 2H), 3.45-3.38 (m, 2 H).
UPLC/MS (3 min) retention time 1.84 min.
LRMS: m/z 474 (M+1).

### PREPARATION 150

### 3-Bromo-5-fluoro-1H-indole

5-Fluoroindole (2.0 g, 15.0 mmol) was dissolved in 15 ml dimethylformamide. N-bromosuccinimide (2.8 g, 15.8 mmol) was added portion-wise and with stirring. The mixture was stirred for 1 h and was then partitioned between ether and water. The aqueous phase was extracted with ether and the combined organics were washed with water, brine, dried over sodium sulphate, filtered and evaporated under reduced pressure. The residue was purified using the Isolera Purification system (ether-hexane gradient, 0:100 rising to 30:70) to give 2.50 g of the title compound as a pink solid. Purity 100%. Used directly in the next reaction to avoid decomposition.
UPLC/MS (3 min) retention time 1.75 min.
LRMS: m/z 212, 214 (M-1).

### PREPARATION 151

### 3-Bromo-5-fluoro-1-(phenylsulfonyl)-1H-indole

The title compound of Preparation 150 (2.50 g) and benzenesulfonyl chloride (2.24 g, 17.6 mmol) were dissolved in 60 ml toluene. tert-Butylammonium hydrogen sulphate (0.40 g, 1.2 mmol) was added followed by 2N sodium hydroxide solution (7.3 ml, 14.6 mmol). The mixture was stirred vigorously at reflux for 1 h. The mixture was allowed to cool and was diluted with water. The aqueous phase was extracted twice with dichloromethane and the combined organics were washed with brine, dried over sodium sulphate, filtered and evaporated under reduced pressure. The residue was purified using the Isolera Purification system (ether-hexane gradient, 0:100 rising to 10:90) to give 3.91 g (11.0 mmol, 73% over two steps) of the title compound as a white solid. Purity 100%.
¹H NMR (400 MHz, DMSO-*d*₆) δ ppm 8.28 (s, 1 H), 8.00 - 8.09 (m, 3 H), 7.74 (tt, *J*=8.00, 4.00 Hz, 1 H), 7.60 - 7.66 (m, 2 H), 7.27 - 7.37 (m, 2 H).
UPLC/MS (3 min) retention time 2.11 min.
LRMS: m/z 352, 354 (M-1).

### PREPARATION 152

### N-Ethyl-N-{2-[5-fluoro-1-(phenylsulfonyl)-1H-indol-3-yl]benzyl}cyclopropanecarboxamide

The title compound of Preparation 151 (150 mg, 0.42 mmol) was treated with the title compound of Preparation 12 (140 mg, 0.43 mmol), caesium carbonate solution (2M, 0.63 ml, 1.26 mmol) and [1,1,'-bis(diphenylphosphino)ferrocene]dichloro-palladium(II), complex (35 mg, 0.04 mmol) according to the method of Preparation 32 to give 100 mg (0.21 mmol, 50%) of the title compound. Purity 100%.
Partial ¹H NMR (300 MHz, CHLOROFORM-d) 58:42 mixture of two rotamers.
Major rotamer δ ppm 4.49 (s, 2 H), 3.24 (q, *J*=7.03 Hz, 2 H).
Minor rotamer δ ppm 4.47 (br. s., 2 H), 3.34 (q, *J*=7.16 Hz, 2 H).
UPLC/MS (3 min) retention time 2.11 min.
LRMS: m/z 477 (M+1).

### PREPARATION 153

### N-Ethyl-N-[2-(5-fluoro-1H-indol-3-yl)benzyl]cyclopropanecarboxamide

The title compound of Preparation 152 (100 mg, 0.21 mmol) was treated with 0.21 ml 4N sodium hydroxide solution according to the method of Preparation 148 to give 65 mg (0.19 mmol, 92%) of the title compound. Purity 100%.
UPLC/MS (3 min) retention time 1.90 min.
LRMS: m/z 337 (M+1).

### PREPARATION 154

### Ethyl [3-(2-{[(cyclopropylcarbonyl)(ethyl)amino]methyl}phenyl)-5-fluoro-1H-indol-1-yl]acetate

The title compound of Preparation 153 (65 mg, 0.19 mmol) was treated with caesium carbonate (125 mg, 0.29 mmol) and ethyl 2-bromoacetate (32 µl, 0.29 mmol) according to the method of Preparation 31. The resulting residue was partially purified using the Isolera Purification System (ether-hexane gradient, 0:100 rising to 100:0) to give 70 mg of the crude title compound. Purity 85%.
Partial ¹H NMR (300 MHz, CHLOROFORM-d) 50:50 mixture of two rotamers.
Rotamer 1 δ ppm 4.90 (s, 2 H), 4.68 (s, 2H), 3.43-3.36 (q, J=7.04Hz, 2 H).
Rotamer 2 δ ppm 4.87 (s, 2 H), 4.64 (s, 2H), 3.30-3.23 (q, J=7.04Hz, 2 H).
UPLC/MS (3 min) retention time 2.00 min.
LRMS: m/z 423 (M+1).

### PREPARATION 155

### 3-[2-[(5-Cyclopropyl-3-methylisoxazol-4-yl)methyl]-4-(trifluoromethyl)phenyl]-5-fluoro-1H-indole

### 3-[2-[(3-Cyclopropyl-5-methylisoxazol-4-yl)methyl]-4-(trifluoromethyl)phenyl]-5-fluoro-1H-indole

The mixture of the crude title compounds of Preparation 29 (100 mg) was treated with 4-fluorophenyl hydrazine hydrochloride (43 mg, 0.34 mmol) according to the method of Preparation 103 followed by reverse-phase chromatography using the SP1 Purification system to give 80 mg (0.19 mmol, 13% over six steps) of a mixture of the title compounds. Purity 100%.
UPLC/MS (3 min) retention time 2.11 min.
LRMS: m/z 415 (M+1).

### PREPARATION156

### Ethyl {3-[2-[(5-cyclopropyl-3-methylisoxazol-4-yl)methyl]-4-(trifluoromethyl)-phenyl]-5-fluoro-1H-indol-1-yl}acetate

### Ethyl {3-[2-[(3-cyclopropyl-5-methylisoxazol-4-yl)methyl]-4-(trfluoromethyl)-phenyl]-5-fluoro-1H-indol-1-yl}acetate

The mixture of the title compounds of Preparation 155 (80 mg, 0.19 mmol) was treated with sodium hydride (60% dispersion in oil, 16 mg, 0.40 mmol) and ethyl 2-bromoacetate (37 mg, 0.22 mmol) according to Method B of Preparation 99 to give 75 mg (0.15 mmol, 76%) of a mixture of the title compounds as a colourless oil. Purity 98%.
UPLC/MS (3 min) retention time 2.15 min.
LRMS: m/z 501 (M+1).

### EXAMPLES

### EXAMPLE 1

### {3-[2-{[(Cyclopropylcarbonyl)(ethyl)amino]methyl]-4-(trifluoromethyl)phenyl]-1H-indazol-1-yl}acetic acid

The title compound of Preparation 32 (310 mg, 0.62 mmol) was dissolved in 6 ml dichloromethane and trifluoroacetic acid (2.3 ml, 30.9 mmol) was added. The mixture was stirred at room temperature for 18 h. The mixture was evaporated and the residue was dissolved in dichloromethane. The organics were washed with saturated sodium bicarbonate solution, brine, dried over sodium sulphate, filtered and evaporated under reduced pressure. The resulting residue was purified using the Isolera Purification System (ethanol-methanol-dichloromethane gradient, 0:0:100 rising to 0:5:95 and then rising to 100:0:0) to give 170 mg (0.38 mmol, 62%) of the title compound. Purity 100%.
Partial ¹H NMR (300 MHz, DMSO-d) 50:50 mixture of two rotamers.
Rotamer 1 δ ppm 5.18 (s, 2 H), 4.70 (s, 2 H).
Rotamer 2 δ ppm 5.18 (s, 2 H).
UPLC/MS (3 min) retention time 1.88 min.
LRMS: m/z 446 (M+1).

### EXAMPLE 2

### {4-Chloro-3-[2-{[(cyccopropylcarbonyl)(ethyl)amino]methyl}-4-(trifluoromethyl)phenyl]-1H-indazol-1-yl}acetic acid

The title compound of Preparation 36 (175 mg, 0.33 mmol) was treated with trifluoroacetic acid (2.5 ml, 32.7 mmol) according to the method of Example 1. The resulting residue was purified using the Isolera Purification System (ethanol-methanol-dichloromethane gradient, 0:0:100 rising to 0:5:95 and then rising to 100:0:0) to give 110 mg (0.23 mmol, 66%) of the title compound. Purity 94%.
Partial ¹H NMR (300 MHz, DMSO-d) 65:35 mixture of two rotamers.
Major rotamer δ ppm 4.47 (s, 2 H).
Minor rotamer δ ppm 4.64 (s, 2 H).
UPLC/MS (3 min) retention time 1.93 min.
LRMS: m/z 480 (M+1).

### EXAMPLE 3

### {3-[2-{[(Cyclopropylcarbonyl)(ethyl)amino]methyl}-4-(trifluoromethyl)phenyl]-5-fluoro-1H-indazol-1-yl}acetic acid

The title compound of Preparation 40 (130 mg, 0.25 mmol) was treated with trifluoroacetic acid (0.95 ml, 12.33 mmol) according to the method of Example 1. The resulting residue was purified using the Isolera Purification System (ethanol-methanol-dichloromethane gradient, 0:0:100 rising to 0:5:95 and then rising to 100:0:0) to give 90 mg (0.19 mmol, 78%) of the title compound. Purity 100%.
Partial ¹H NMR (300 MHz, DMSO-d) 65:35 mixture of two rotamers.
Major rotamer δ ppm 4.92 (s, 2 H).
Minor rotamer δ ppm 5.05 (s, 2 H).
UPLC/MS (3 min) retention time 1.91 min.
LRMS: m/z 464 (M+1).

### EXAMPLE 4

### [4-Chloro-3-(2-{[(cyclopropylcarbonyl)(ethyl)amino]methyl}-4-fluorophenyl)-1H-indazol-1-yl]acetic acid

The title compound of Preparation 37 (70 mg, 0.14 mmol) was treated with trifluoroacetic acid (0.55 ml, 7.19 mmol) according to the method of Example 1. The resulting residue was purified using the Isolera Purification System (ethanol-methanol-dichloromethane gradient, 0:0:100 rising to 0:5:95 and then rising to 100:0:0) to give 32 mg (0.07 mmol, 47%) of the title compound. Purity 92%.
Partial ¹H NMR (300 MHz, DMSO-d) 65:35 mixture of two rotamers.
Major rotamer δ ppm 4.36 (s, 2 H).
Minor rotamer δ ppm 4.50 (s, 2 H).
HPLC/MS (3 min) retention time 1.81 min.
LRMS: m/z 430 (M+1).

### EXAMPLE 5

### {5-Chloro-3-[2-][(cyclopropylcarbonyl)(ethyl)amino]methyl}-4-(trifluoromethyl)phenyl]-1H-indazol-1-yl}acetic acid

The crude title compound of Preparation 46 (110 mg, 0.22 mmol) was dissolved in 2 ml tetrahydrofuran and 2 ml water. Lithium hydroxide monohydrate (90 mg, 2.14 mmol) was added and the mixture was agitated at room temperature for 3 h. The organic solvent was evaporated under reduced pressure and water was added. The aqueous layer was extracted with ethyl acetate. The organics were washed with brine, dried over magnesium sulphate and evaporated. The resulting residue was purified using the Isolera Purification System (ethanol-methanol-dichloromethane gradient, 0:0:100 rising to 0:5:95 and then rising to 100:0:0) to give 60 mg (0.12 mmol, 55%) of the title compound. Purity 95%.
Partial ¹H NMR (300 MHz, DMSO-d) 65:35 mixture of two rotamers.
Major rotamer δ ppm 4.84 (s, 2 H), 4.78 (s, 2 H).
Minor rotamer δ ppm 5.04 (s, 2 H), 4.84 (s, 2 H).
UPLC/MS (3 min) retention time 2.01 min.
LRMS: m/z 480 (M+1).

### EXAMPLE 6

### {3-[2-{[(Cyclopropylcarbonyl)(ethyl)amino]methyl}-4-(trifluoromethyl)phenyl]-6-fluoro-1H-indazol-1-yl}acetic acid

The title compound of Preparation 49 (80 mg, 0.15 mmol) was treated with trifluoroacetic acid (0.59 ml, 7.70 mmol) according to the method of Example 1. The resulting residue was purified using the Isolera Purification System (ethanol-methanol-dichloromethane gradient, 0:0:100 rising to 0:5:95 and then rising to 100:0:0) to give 62 mg (0.13 mmol, 87%) of the title compound. Purity 100%.
Partial ¹H NMR (300 MHz, DMSO-d) 70:30 mixture of two rotamers.
Major rotamer δ ppm 4.87 (s, 2 H), 4.78 (s, 2 H).
Minor rotamer δ ppm 5.04 (s, 2 H), 4.87 (s, 2 H).
UPLC/MS (3 min) retention time 1.89 min.
LRMS: m/z 464 (M+1).

### EXAMPLE 7

### {3-[2-{[(Cyclopropylcarbonyl)(ethyl)amino]methyl}-4-(trifluoromethyl)phenyl]-4-methoxy-1H-indazol-1-yl}acetic acid

The title compound of Preparation 52 (60 mg, 0.11 mmol) was treated with trifluoroacetic acid (0.43 ml, 5.58 mmol) according to the method of Example 1. The resulting residue was purified using the Isolera Purification System (ethanol-methanol-dichloromethane gradient, 0:0:100 rising to 0:5:95 and then rising to 100:0:0) to give 31 mg (0.13 mmol, 55%) of the title compound. Purity 95%.
Partial ¹H NMR (300 MHz, DMSO-d) 60:40 mixture of two rotamers.
Major rotamer δ ppm 4.60 (s, 2 H).
Minor rotamer δ ppm 4.76 (s, 2 H).
UPLC/MS (3 min) retention time 1.80 min.
LRMS:m/z476(M+1).

### EXAMPLE 8

### [3-(2-{[(Cyclopropylcarbonyl)(ethyl)amino]methyl}-4-fluorophenyl)-5-(trifluoromethyl)-1H-indazol-1-yl]acetic acid

The title compound of Preparation 43 (110 mg, 0.21 mmol) was treated with trifluoroacetic acid (0.81 ml, 10.5 mmol) according to the method of Example 1. The resulting residue was purified using the Isolera Purification System (ethanol-methanol-dichloromethane gradient, 0:0:100 rising to 0:5:95 and then rising to 100:0:0) to give 78 mg (0.17 mmol, 80%) of the title compound as a white solid. Purity 100%.
Partial ¹H NMR (300 MHz, DMSO-d) 60:40 mixture of two rotamers.
Major rotamer δ ppm 4.66 (s, 2 H).
Minor rotamer δ ppm 4.38 (s, 2 H).
HPLC/MS (3 min) retention time 1.91 min.
LRMS: m/z 464 (M+1).

### EXAMPLE 9

### 2-{4-Chloro-3-[2-{[(cyclopropylcarbonyl)(ethyl)amino]methyl}-4-(trifluoromethyl)phenyl]-1H-indazol-1-yl}propanoic acid

The title compound of Preparation 54 (44 mg, 0.09 mmol) was treated with lithium hydroxide monohydrate (36 mg, 0.86 mmol) according to the method of Example 5 to give 34 mg (0.07 mmol, 79%) of the title compound as a white solid. Purity 100%.
Partial ¹H NMR (300 MHz, DMSO-d) 70:30 mixture of two rotamers.
Major rotamer δ ppm 5.73-5.90 (m, 1 H), 4.46 (s, 2 H).
Minor rotamer δ ppm 5.73-5.90 (m, 1 H), 4.63 (s, 2 H).
UPLC/MS (3 min) retention time 2.02 min.
LRMS: m/z 494 (M+1).

### EXAMPLE 10

### [3-(2-{[(Cyclopropylcarbonyl)(ethyl)amino]methyl}-4-fluorophenyl)-4-(trifluoromethyl)-1H-indazol-1-yl]acetic acid

The crude title compound of Preparation 56 (160 mg) and the title compound of Preparation 16 (169 mg, 0.49 mmol) were treated with 2.0M caesium carbonate solution (2M, 0.56 ml, 1.13 mmol) and [1,1'bis(diphenylphosphino)-ferrocene] dichloropalladium(II), complex with dichloromethane (31 mg, 0.04 mmol) according to the method of Preparation 32. The resulting residue was purified using the Isolera Purification System (ethanol-methanol-dichloromethane gradient, 0:0:100 rising to 0:5:95 and then rising to 100:0:0) to give 53 mg (0.11 mmol, 20% over two steps) of the title compound as a white solid. Purity 98%.
Partial ¹H NMR (300 MHz, DMSO-d) 85:15 mixture of two rotamers.
Major rotamer δ ppm 4.21 (s, 2 H).
Minor rotamer δ ppm 4.48 (s, 2 H).
UPLC/MS (3 min) retention time 1.83 min.
LRMS: m/z 464 (M+1).

### EXAMPLE 11

### [5-Chloro-3-(2-{[(cyclopropylcarbonyl)(ethyl)amino]methyl}-4-fluorophenyl)-1H-indazol-1-yl]acetic acid

The title compound of Preparation 75 (154 mg, 0.34 mmol) was treated with lithium hydroxide monohydrate (141 mg, 3.36 mmol) according to the method of Example 5 to give 120 mg (0.28 mmol, 83%) of the title compound as a white solid. Purity 100%.
Partial ¹H NMR (300 MHz, DMSO-d) 65:35 mixture of two rotamers.
Major rotamer δ ppm 4.68 (s, 2H).
Minor rotamer δ ppm 4.92 (s, 2H).
UPLC/MS (3 min) retention time 1.89 min.
LRMS: m/z 430 (M+1).

### EXAMPLE12

### {4-Cyano-3-[2-{[(cyclopropylcarbonyl)(ethyl)amino]methyl}-4-(trifluoromethyl)phenyl]-1H-indazol-1-yl}acetic acid

The title compound of Preparation 74 (130 mg, 0.25 mmol) was treated with trifluoroacetic acid (0.95 ml, 12.3 mmol) according to the method of Example 1. The resulting residue was purified using the Isolera Purification System (ethanol-methanol-dichloromethane gradient, 0:0:100 rising to 0:5:95 and then rising to 100:0:0) to give 94 mg (0.20 mmol, 80%) of the title compound as a white solid. Purity 96%.
Partial ¹H NMR (300 MHz, DMSO-d) 70:30 mixture of two rotamers.
Major rotamer δ ppm 5.53 (s, 2 H), 4.57 (s, 2 H).
Minor rotamer δ ppm 5.55 (s, 2 H), 4.75 (s, 2 H).
UPLC/MS (3 min) retention time 1.69 min.
LRMS: m/z 471 (M+1).

### EXAMPLE 13

### 2-(5-Chloro-3-[2-{[(cyclopropylcarbonyl)(ethyl)amino]methyl-4-(trifluoromethyl)phenyl]-1H-indazol-1-yl}propanoic acid

The title compound of Preparation 61 (120 mg, 0.24 mmol) was treated with lithium hydroxide monohydrate (100 mg, 2.38 mmol) according to the method of Example 5 to give 102 mg (0.20 mmol, 87%) of the title compound as a white solid. Purity 100%.
Partial ¹H NMR (300 MHz, DMSO-d) 70:30 mixture of two rotamers.
Major rotamer δ ppm 4.78 (d, *J*=4,67 Hz, 2 H).
Minor rotamer δ ppm 5.05 (s, 2 H).
UPLC/MS (3 min) retention time 2.09 min.
LRMS: m/z 494 (M+1).

### EXAMPLE 14

### {3-[2-{[(Cyclopropylcarbonyl)(ethyl)amino]methyl}-4-(trifluoromethyl)phenyl]-4-fluoro-1H-indazol-1-yl}acetic acid

The title compound of Preparation 59 (115 mg, 0.20 mmol) was treated with trifluoroacetic acid (0.77 ml, 10 mmol) according to the method of Example 1. The resulting residue was purified using the Isolera Purification System (ethanol-methanol-dichloromethane gradient, 0:0:100 rising to 0:5:95 and then rising to 100:0:0) to give 40 mg (0.19 mmol, 40%) of the title compound as a white solid. Purity 95%.
Partial ¹H NMR (300 MHz, DMSO-d) 70:30 mixture of two rotamers.
Major rotamer δ ppm 5.43 (s, 2 H), 4.67 (s, 2H).
Minor rotamer δ ppm 5.45 (s, 2 H), 4.91 (s, 2H).
UPLC/MS (3 min) retention time 1.87 min.
LRMS: m/z 464 (M+1).

### EXAMPLE 15

### {3-[2-{[(Cyclopropylcarbonyl)(ethyl)amino]methyl}-4-(trifluoromethyl)phenyl]-5-methyl-1H-indazol-1-yl}acetic acid

The title compound of Preparation 64 (180 mg, 0.35 mmol) was treated with trifluoroacetic acid (1.2 ml, 16.2 mmol) according to the method of Example 1. The resulting residue was purified using the Isolera Purification System (methanol-dichloromethane gradient, 0:100 rising to 5:95) to give 120 mg (0.26 mmol, 75%) of the title compound as a white solid. Purity 100%.
Partial ¹H NMR (300 MHz, DMSO-d) 70:30 mixture of two rotamers.
Major rotamer δ ppm 5.29 (s, 2 H), 4.69 (s, 2 H).
Minor rotamer δ ppm 5.32 (s, 2 H), 4.94 (s, 2 H).
UPLC/MS (3 min) retention time 1.97 min.
LRMS: m/z 460 (M+1).

### EXAMPLE 16

### {3-[2-{[(Cyclopropylcarbonyl)(ethyl)ammo]methyl}-4-(trifluoromethyl)phenyl]-4-methyl-1H-indazol-1-yl}acetic acid

The title compound of Preparation 71 (280 mg, 0.54 mmol) was treated with trifluoroacetic acid (2 ml, 26 mmol) according to the method of Example 1. The resulting residue was purified using the Isolera Purification System (methanol-dichloromethane gradient, 0:100 rising to 5:95) to give 180 mg (0.38 mmol, 68%) of the title compound as a white solid. Purity 98%.
Partial ¹H NMR (400 MHz, DMSO-d) 70:30 mixture of two rotamers.
Major rotamer δ ppm 5.33 (s, 2 H), 4.37 (s, 2 H).
Minor rotamer δ ppm 5.35 (s, 2 H), 4.64 (s, 2 H).
UPLC/MS (3 min) retention time 1.89 min.
LRMS: m/z 460 (M+1).

### EXAMPLE 17

### {4-Chloro-3-[2-{[(cydopropylcarbonyl)(ethyl)amino]methyl}-4-(trifluoromethyl)phenyl]-5-fluoro-1H-indazol-1-yl}acetic acid

The title compound of Preparation 68 (95 mg, 0.17 mmol) was treated with trifluoroacetic acid (0.66 ml, 8.6 mmol) according to the method of Example 1. The resulting residue was purified using the Isolera Purification System (methanol-dichloromethane gradient, 0:100 rising to 5:95) to give 26 mg (0.05 mmol, 30%) of the title compound as a white solid. Purity 100%.
Partial ¹H NMR (300 MHz, DMSO-d) 70:30 mixture of two rotamers.
Major rotamer δ ppm 5.42 (s, 2 H), 4.46 (s, 2H).
Minor rotamer δ ppm 5.44 (s, 2 H), 4.64 (s, 2 H).
UPLC/MS (3 min) retention time 1.95 mm.
LRMS: m/z 498 (M+1).

### EXAMPLE 18

### [3-(2-{[(tert-Butoxycarbonyl)(ethyl)amino]methyl}-4-fluorophenyl)-5-chloro-1H-indazol-1-yl]acetic acid

The title compound of Preparation 76 (24 mg, 0.049 mmol) was dissolved in 2 ml tetrahydrofuran and 2 ml water. Lithium hydroxide monohydrate (12 mg, 0.29 mmol) was added and the mixture was agitated at room temperature for 90 min. The mixture was acidified to pH 5 with 2N hydrochloric acid and the aqueous was extracted with ether. The organic were dried over sodium sulphate, filtered and evaporated to give 20 mg (0.043 mmol, 88%) of the title compound as a white solid. Purity 100%.
¹H NMR (300 MHz, DMSO-d6) δ ppm 7.83 (d, *J*=8.8 Hz, 1 H), 7.62 - 7.75 (m, 2 H), 7.50 (d, *J*=9.1 Hz, 1 H), 7.22 - 7.34 (m, 1 H), 7.07 (d, *J*=10.4 Hz, 1 H), 5.38 (s, 2 H), 4.51 (br. s., *J*=1.6 Hz, 2 H), 3.09 (br. s., 2 H), 1.20 - 1.49 (m, 9 H), 0.88 (t, *J*=6.7 Hz, 3 H).
UPLC/MS (3 min) retention time 2.14 min.
LRMS: m/z 4.62 (M+1).

### EXAMPLE 19

### [5-Chloro-3-(2-{[(cyclobutylcarbonyl)(ethyl)amino]methyl}-4-fluorophenyl)-1H-indazol-1-yl]acetic acid

The title compound of Preparation 78 (39 mg, 0.08 mmol) was treated with lithium hydroxide monohydrate (21 mg, 0.50 mmol) according to the method of Example 5 to give 30 mg (0.07 mmol, 82%) of the title compound as a white solid. Purity 96%.
Partial ¹H NMR (400 MHz, DMSO-d) 65:35 mixture of two rotamers.
Major rotamer δ ppm 5.37 (s, 2 H), 4.61 (s, 2H).
Minor rotamer δ ppm 5.38 (s, 2 H), 4.56 (s, 2H).
UPLC/MS (3 min) retention time 1.98 min.
LRMS:m/z444(M+1).

### EXAMPLE 20

### [5-Chloro-3-(2-{[ethyl(isobutyryl)amino]methyl}-4-fluorophenyl)-1H-indaxol-1-yl]acetic acid

The title compound of Preparation 79 (30 mg, 0.07 mmol) was treated with lithium hydroxide monohydrate (16 mg, 0.38 mmol) according to the method of Example 5 to give 24 mg (0.05 mmol, 85%) of the title compound as a white solid. Purity 100%.
Partial ¹H NMR (400 MHz, DMSO-d) 70:30 mixture of two rotamers.
Major rotamer δ ppm 5.38 (s, 2 H), 4.62 (s, 2H).
Minor rotamer δ ppm 5.40 (s, 2 H), 4.71 (s, 2H).
UPLC/MS (3 min) retention time 1.93 min.
LRMS:m/z432(M+1).

### EXAMPLE 21

### [5-Chloro-3-(2-{[ethyl(methylsulfonyl)amino]methyl}-4-fluorophenyl)-1H-indazol-1-yl]acetic acid

The crude title compound of Preparation 80 (31 mg) was treated with lithium hydroxide monohydrate (17 mg, 0.41 mmol) according to the method of Example 5 to give 23 mg (0.052 mmol, 67% over two steps) of the title compound as a white solid. Purity 93%.
¹H NMR (300 MHz, CHLOROFORM-d) δ ppm 7.62 (s, 1 H), 7.41 - 7.56 (m, 3 H), 7.35 (d, *J*=9.00 Hz, 1 H), 7.15 (t, *J*=8.10 Hz, 1 H), 5.22 (s, 2 H), 4.54 (s, 2 H), 3.31 (br. s., 1 H), 3.15 (q, *J*=6.96 Hz, 2 H), 2.79 (s, 3 H), 0.91 (t, *J*=7.14Hz,3H).
HPLC/MS (9 min) retention time 6.55 min.
LRMS: m/z 440 (M+1).

### EXAMPLE 22

### [3-(2-{[Acetyl(ethyl)amino]]methyl}-4-fluorophenyl)-5-chloro-1H-indazol-1-yl]acetic acid

The title compound of Preparation 81 (29 mg, 0.067 mmol) was treated with lithium hydroxide monohydrate (17 mg, 0.41 mmol) according to the method of Example 5 to give 20 mg (0.050 mmol, 75%) of the title compound as a white solid. Purity 94%.
Martial ¹H NMR (300 MHz, CHLOROFORM-d) 65:35 mixture of two rotamers.
Major rotamer δ ppm 5.19 (s, 2 H), 4.74 (d, *J*= 9.00 Hz, 2H).
Minor rotamer δ ppm 5.22 (s, 2 H), 4.66 (d, *J*= 9.00 Hz, 2H).
UPLC/MS (3 min) retention time 1.77 min.
LRMS: m/z 404 (M+1).

### EXAMPLE 23

### (3-[2-{[[(Benzylamino)carbonyl](ethyl)amino]methyl}-4-(trifluoromethyl)phenyl]-4-chloro-1H-indazol-1-yl}acetic acid

The title compound of Preparation 84 (59 mg, 0.10 mmol) was treated with lithium hydroxide monohydrate (13 mg, 0.31 mmol) according to the method of Example 31 to give 46 mg (0.084 mmol, 82%) of the title compound as a white solid. Purity 100%.
¹H NMR (partial, 400 MHz, CHLOROFORM-d) δ ppm 7.69 (1 H, d, J=8.2 Hz), 7.57 - 7.64 (2 H, m), 7.41 (1 H, t, *J*=7.0 Hz), 7.37 (1 H, d, *J*=7.0 Hz), 7.20 - 7.31 (3 H, m), 7.17 (1 H, d, *J*=5.9 Hz), 6.98 (1 H, dd, *J*=7.6, 1.8 Hz), 5.25 (2 H, br. s.), 4.31 (1 H, br. s.), 3.68 (2 H, br. s.), 2.97 (2 H, q, *J*=7.2 Hz); 0.98 (3 H, t, *J*=7.0 Hz)
UPLC/MS (3 min) retention time 1.98 min.
LRMS: m/z 545 (M+1).

### EXAMPLE 24

### [5-Chloro-3-(2-{[(cycopropylcarbonyl))(ethyl)amino]methyl}phenyl)-1H-indazol-1-yl]acetic acid

The title compound of Preparation 45 (80 mg, 0.22 mmol) was treated with the title compound of Preparation 12 (82 mg, 0.24 mmol), caesium carbonate solution (2M, 0.33 ml, 0.66 mmol), [1,1'-bis(diphenylphosphino)ferrocene]dichloropalladium(II), complex with dichloromethane (18 mg, 0.02 mmol) according to the method of Preparation 32 and then with lithium hydroxide monohydrate (92 mg, 2.2 mmol) according to the method of Example 5 to give 50 mg (0.11 mmol, 52%) of the title compound as a brown solid. Purity 100%.
Martial H NMR (300 MHz, DMSO-d) 50:50 mixture of two rotamers. Rotamer 1 δ ppm 4.91 (s, 2 H), 4.87 (s, 2 H).
Rotamer 2 δ ppm 4.87 (s, 1 H), 4.70 (s, 2 H).
UPLC/MS (3 min) retention time 1.87 min.
LRMS: m/z 412 (M+1).

### EXAMPLE 25

### [5-Chloro-3-(2-{[(cyclopentylcarbonyl)(ethyl)amino]methyl}-4-fluorophenyl)-1H-indazol-1-yl]acetic acid

The crude title compound of Preparation 82 (40 mg) with treated with lithium hydroxide monohydrate (17 mg, 0.41 mmol) according to the method of Example 5. The resulting residue was purified using the Isolera Purification System (methanol-dichloromethane gradient, 0:100 rising to 10:9) to give 16 mg (0.032 mmol, 41% over two steps) of the title compound as a white solid. Purity 100%.
Partial ¹H NMR (400 MHz, DMSO-d) 65:35 mixture of two rotamers.
Major rotamer δ ppm 4.67 (d, *J*=12 Hz, 2H).
Minor rotamer δ ppm 4.76 (d, *J*=12 Hz, 2H).
HPLC/MS (3 min) retention time 2.04 min.
LRMS: m/z 458 (M+1).

### EXAMPLE 26

### [5-Chloro-3-(2-([(cyclopropylacetyl)(ethyl)amino]methyl}-4-fluorophenyl)-1H-indazol-1-yl]acetic acid

The title compound of Preparation 83 (18 mg, 0.038 mmol) with treated with lithium hydroxide monohydrate (10 mg, 0.24 mmol) according to the method of Example 5 to give 16 mg (0.036 mmol, 95%) of the title compound as a white solid. Purity 100%.
Martial ¹H NMR (400 MHz, DMSO-d) 70:30 mixture of two rotamers.
Major rotamer δ ppm 4.72 (s, 2H).
Minor rotamer δ ppm 4.74 (s, 2H).
HPLC/MS (9 min) retention time 6.82 min.
LRMS: m/z 442 (M-1).

### EXAMPLE 27

### (5-Chloro-3-[2-1[(cyclopropylcarbonyl)(ethyl)amino]methyl}-4-(trifluoromethyl)phenyl]-4-fluoro1-H-indazol-1-yl}acetic acid

The title compound of Preparation 91 (153 mg, 0.28 mmol) was suspended in 0.6 ml water. Hydrochloric acid (4N in dioxane, 6 ml, 24 mmol) was added and the mixture was stirred at room temperature for 1 h. The mixture was evaporated and the residue was triturated with hexane. The resulting solid was dried in the oven to give 107 mg (0.21 mmol, 78%) of the title compound. Purity 100%.
Martial ¹H NMR (300 MHz, CHLOROFORM-d) 75:25 mixture of two rotamers.
Major rotamer δ ppm 5.23 (s, 2H), 4.84 (s, 2H), 3.28 (q, J = 6.5 Hz, 2H), 3.28 (q, J = Hz, 2H).
Minor rotamer δ ppm 5.19 (s, 2 H), 4.84 (s, 2H), 3.40 (q, J = 6.5 Hz, 2H).
HPLC/MS (5 min) retention time 3.63 min.
LRMS: m/z 498 (M+1).

### EXAMPLE 28

### {1-[2-[[(Cyclopropylcarbonyl)(ethyl)amino]methyl)-4-(trifluoromethyl)phenyl]-1H-indol-3-yl}acetic acid

The crude title compound of Preparation 93 (21 mg) was treated with lithium hydroxide monohydrate (18 mg, 0.43 mmol) according to the method of Example 5. The residue was purified by reverse phase using Isolera Purification system to give 14 mg (0.03 mmol, 7% over two steps) of the title compound as a beige solid. Purity 100%.
Martial ¹H NMR (300 MHz, DMSO-d) 75:25 mixture of two rotamers.
Major rotamer δ ppm 7.90 (d, *J*= 6.0 Hz, 1H).
Minor rotamer δ ppm 7.97 (d, *J*= 6.0 Hz, 1H).
UPLC/MS (3 min) retention time 1.93 min.
LRMS: m/z 445 (M+1).

### EXAMPLE 29

### [1-(2-{[(Cycopropylcarbonyl)(ethyl)amino]methyl}-4-fluorophenyl)-1H-indol-3-yl]acetic acid

The title compound of Preparation 7 (304 mg, 1.01 mmol) was treated with the title compound of Preparation 92 (308 mg, 1.52 mmol), salicylaldoxime (4 mg, 0.03 mmol), caesium carbonate (742 mg, 2.28 mmol) and copper(I) oxide (5 mg, 0.03 mmol) according to the method of Preparation 93. The residue obtained was dissolved in 2N sodium hydroxide solution and extracted twice with ethyl acetate. The organics were dried over sodium sulphate, filtered and evaporated under reduced pressure. The resulting residue was purified using the Isolera Purification System (methanol-dichloromethane gradient, 0:100 rising to 10:90) to give 13 mg (0.03 mmol, 3%) of the title compound as a pale brown solid. Purity 95%.
Partial ¹H NMR (300 MHz, DMSO-d) 70:30 mixture of two rotamers. Major rotamer δ ppm 4.15 (s, 2 H), 3.77 (s, 2H).
Minor rotamer δ ppm 4.47 (d, *J=15* Hz, 1 H), 4.24 (d, *J*=15 Hz, 1 H), 3.77 (s, 2H).
UPLC/MS (3 min) retention time 1.81 min.
LRMS:m/z395(M+1).

### EXAMPLE 30

### {1-[2-[[(Cyclopropylcarbonyl)(ethyl)amino]methyl}-4-(trifluoromethyl)phenyl]-6-fluoro-1H-indol-3-yl}acetic acid

The title compound of Preparation 96 (72 mg, 0.14 mmol) was treated with trifluoroacetic acid (0.8 ml, 10.4 mmol) according to the method of Example 1. The residue was purified using the Isolera Purification System (methanol-dichloromethane gradient, 0:100 rising to 4:96) to give 32 mg (0.07 mmol, 50%) of the title compound as a white solid. Purity 100%.
Partial ¹H NMR (300 MHz, CHLOROFORM-d) 75:25 mixture of two rotamers.
Major rotamer δ ppm 4.70 (d, *J*= 15 Hz, 1H), 4.26 (d, *J*= 15 Hz, 1H), 3.86 (s, 2H).
Minor rotamer δ ppm 4.40 (s, 2 H), 3.06 (s, 2H).
UPLC/MS (3 min) retention time 1.94 min.
LRMS:m/z463(M+1).

### EXAMPLE 31

### (3-[2-[[(Cyclopropylcarbonyl)(ethyl)amino]methyl)-4-(trifluoromethyl)phenyl]-1H-indol-1-yl}acetic acid

The title compound of Preparation 99 (10 mg, 0.021mmol) was dissolved in 2 ml tetrahydrofuran and 2 water. Lithium hydroxide monohydrate (3 mg, 0.071 mmol) was added and the mixture was agitated at room temperature for 2 h. The organic solvent was evaporated under reduced pressure and the aqueous was acidified with 2 ml 2N hydrochloric acid, forming a white precipitate. The mixture was agitated for 30 min and the solid was collected by filtration. The solid was washed several times with water, then with hexane and was dried overnight in the oven to give 5 mg (0.011 mmol, 50%) of the title compound. Purity 95%.
Partial ¹H NMR (400 MHz, DMSO-d) 66:34 mixture of two rotamers.
Major rotamer δ ppm 4.86 (s, 2 H).
Minor rotamer δ ppm 4.92 (s, 2 H).
UPLC/MS (3 min) retention time 1.93 min.
LRMS: m/z 445 (M+1).

### EXAMPLE 32

### (3-[2-{[(Cyclopropylcarbonyl)(ethy)ammino]methyl)-4-(trifluoromethyl)phenyl]-2-methyl-1H-indol-1-yl}acetic acid

The title compound of Preparation 102 (20 mg, 0.041 mmol) was dissolved in 1 ml tetrahydrofuran and 1 water. Lithium hydroxide monohydrate (5 mg, 0.12 mmol) was added and the mixture was agitated at room temperature for 2 h. The organic solvent was evaporated under reduced pressure and the aqueous was acidified with 2 ml 2N hydrochloric acid, forming a white precipitate. The mixture evaporated to dryness and the residue was purified by reverse-phase chromatography using the SP1 Purification system to give 10 mg (0.021 mmol, 52%) of the title compound. Purity 98%.
¹H NMR (600 MHz, CHLOROFORM-*d*) δ ppm 7.70 (1 H, d, *J*=7.6 Hz), 7.63 (1 H, s), 7.49 (1 H, d, *J*=8.2 Hz), 7.24 - 7.27 (1 H, m), 7.21 (1 H, t, *J*=6.5 Hz), 7.04 - 7.11 (2 H, m), 5.52 (1 H, d, *J*= 14.1 Hz), 4.95 (1 H, d, *J*= 17.0 Hz), 4.89 (1 H, d, *J*=17.0 Hz), 3.62 (1 H, d, *J*=14.1 Hz), 3.20 (1 H, dq, *J*=14.9, 7.4 Hz), 3.02 (1 H, dq, *J*=14.9. 7.4 Hz), 2.28 (3 H, s), 0.95 (3 H, t, *J*=7.3 Hz), 0.79 - 0.86 (1 H, m), 0.44 - 0.53 (2 H, m), 0.01 - 0.07 (1 H, m), -0.19 0.12(1H, m).
UPLC/MS (3 min) retention time 1.96 min.
LRMS: m/z 459 (M+1).

### EXAMPLE 33

### (3-[2-{[(Cyclopropylcarbony))(ethyl)amino]methyl}-4-(trifluoromethyl)phenyl]-5-fluoro-1H-indol-1-yl}acetic acid

The title compound of Preparation 105 (35 mg, 0.070 mmol) was treated with lithium hydroxide monohydrate (10 mg, 0.24 mmol) according to the method of Example 31 to give 29 mg (0.061 mmol, 86%) of the title compound as a white solid. Purity 98%.
Martial ¹H NMR (300 MHz, DMSO-d) 55:45 mixture of two rotamers.
Major rotamer δ ppm 7.21 (1 H, dd, *J*=10.0, 2.1 Hz), 3.40 (2 H, q, *J*=6.8 Hz), 0.99 (3 H, t, *J*=6.8 Hz).
Minor rotamer δ ppm 7.27 (1 H, d, *J*=9.8 Hz), 3.26 (2 H, q, *J*=6.8 Hz), 0.87 (3 H, t, *J*=6.8 Hz).
UPLC/MS (3 min) retention time 1.95 min.
LRMS: m/z 463 (M+1).

### EXAMPLE 34

### {4-Chloro-3-[2-{[(cyclopropylcarbonyl)(ethyl)amino]methyl}-4-(trifluoromethyl)phenyl]-1H-indol-1-yl}lacetic acid

The title compound of Preparation 107 (25 mg, 0.048 mmol) was treated with lithium hydroxide monohydrate (3 mg, 0.07 mmol) according to the method of Example 31 to give 20 mg (0.040 mmol, 80%) of the title compound as a white solid. Purity 96%.
Martial H NMR (300 MHz, DMSO-d) 60:40 mixture of two rotamers.
Major rotamer δ ppm 7.64 (1 H, d, *J*=7.8 Hz), 7.36 (1 H, s), 4.50 (1 H, d, *J*= 16. 0 Hz), 4.32 (1 H, d, *J*=16. 0 Hz), 0. 89 (3 H, t, *J*= 7.0 Hz).
Minor rotamer δ ppm 7.71 (1 H, d, *J*=7.4 Hz), 7.46 (1 H, s), 4.59 (1 H, d, *J*= 17.0 Hz), 4.53 (1 H, d, *J*=17.0 Hz), 0.84 (3 H, t, *J*=7.0 Hz).
UPLC/MS (3 min) retention time 1.98 min.
LRMS: m/z 479 (M+1).

### EXAMPLE 35

### (6-Chloro-3-[2-1[(cyclopropylcarbonyl)(ethyl)amino]methyl}-4-(trifluoromethyl)phenyl]-1H-indol-1-yl}lacetic acid

The title compound of Preparation 109 (23 mg, 0.043 mmol) was treated with lithium hydroxide monohydrate (3 mg, 0.07 mmol) according to the method of Example 31 to give 13 mg (0.026 mmol, 60%) of the title compound as a white solid. Purity 95%.
Partial ¹H NMR (300 MHz, DMSO-d) 60:40 mixture of two rotamers.
Major rotamer δ ppm 4.60 (2 H, s), 1.93 - 2.04 (1 H, m), 0.97 (3 H, t, *J*=6.8 Hz).
Minor rotamer δ ppm 4.82 (2 H, s), 1.69 (1 H, m), 0.86 (3 H, t, *J*=7.0 Hz).
UPLC/MS (3 min) retention time 2.02 min.
LRMS:m/z479(M+1).

### EXAMPLE 36

### (5-Cyano-3-[2-1[(cyclopropylcarbonyl)(ethyl)amino]methyl}-4-(trifluoromethyl)phenyl]-1H-indol-1-yl}lacetic acid

The title compound of Preparation 111 (60 mg, 0.12 mmol) was treated with lithium hydroxide monohydrate (5 mg, 0.12 mmol) according to the method of Example 31 to give 25 mg (0.051 mmol, 42%) of the title compound as a white solid. Purity 95%.
Partial ¹H NMR (400 MHz, DMSO-d) 62:38 mixture of two rotamers.
Major rotamer δ ppm 5.17 (s, 2 H), 4.60 (s., 2 H).
Minor rotamer δ ppm 5.17 (s, 2 H), 4.83 (s., 2 H).
UPLC/MS (3 min) retention time 1.80min.
LRMS:m/z470(M+1).

### EXAMPLE 37

### [3-(2-[[(Cyclopropylcarbonyl)(ethyl)amino]methyl}-4-fluorophenyl)-1H-indol-1-yl]acetic acid

The title compound of Preparation 113 (54 mg, 0.13 mmol) was dissolved in 4 ml tetrahydrofuran and 4 water. Lithium hydroxide monohydrate (40 mg, 0.95 mmol) was added and the mixture was agitated at room temperature for 2 h. The mixture was acidified with 2 ml 2N hydrochloric acid. The mixture was extracted with ethyl acetate and the organics were dried over sodium sulphate, filtered and evaporated. The residue was triturated with ether under ultrasound sonication to give a white solid and was dried in the oven to give 47 mg (0.12 mmol, 90%) of the title compound as a solid. Purity 97%.
Partial ¹H NMR (300 MHz, DMSO-d) 80:20 mixture of two rotamers.
Major rotamer δ ppm 4.89 (2 H, s), 4.65 (2 H, s), 3.22 (2 H, q, *J*=7.0 Hz).
Minor rotamer δ ppm 4.92 (2 H, s), 4.59 (2 H, s), 3.34 (2 H, q, *J*=7.0 Hz).
UPLC/MS (3 min) retention time 1.82 min.
LRMS:m/z395(M+1).

### EXAMPLE 38

### (5-Cyclopropyl-3-[2-([(cyclopropylcarbonyl)(ethyl)amino]methyl}-4-(trifluoromethyl)phenyl]-1H-indol-1-yl}acetic acid

The title compound of Preparation 116 (20 mg, 0.039 mmol) was treated with lithium hydroxide monohydrate (4 mg, 0.10 mmol) according to the method of Example 31 to give 5 mg (0.098 mmol, 25%) of the title compound as a white solid. Purity 98%.
Partial ¹H NMR (400 MHz, DMSO-d) 55:45 mixture of two rotamers.
Major rotamer δ ppm 4.60 (s., 2 H).
Minor rotamer 8 ppm 4.82 (s., 2 H).
UPLC/MS (3 min) retention time 2.07 min.
LRMS:m/z485(M+1).

### EXAMPLE 39

### [3-(2-[[(Cyclopropylcarbonyl)(ethyl)amino]methyl}-4-fluorophenyl)-5-(trifluoromethyl)-1H-indol-1-yl]acetic acid

The title compound of Preparation 118 (75 mg, 0.15 mmol) was treated with lithium hydroxide monohydrate (8 mg, 0.19 mmol) according to the method of Example 31, followed by purification by reverse-phase HPLC to give 32 mg (0.068 mmol, 45%) of the title compound as a white solid. Purity 99%.
Partial ⁴H NMR (400 MHz, DMSO-d) 61:39 mixture of two rotamers.
Major rotamer δ ppm 5.19 (s, 2 H), 4.47 (s, 2H).
Minor rotamer δ ppm 5.20 (s, 2 H), 4.68 (s, 2H).
HPLC/MS (30 min) retention time 18.4 min.
LRMS:m/z463(M+1).

### EXAMPLE 40

### (3-[2-[[(Cyclopropylcarbonyl)(ethyl)amino]methyl}-4-(trifluoromethyl)phenyl]-4-fluoro-1H-indol-1-yl}acetic acid

The title compound of Preparation 120 (35 mg, 0.071 mmol) was treated with lithium hydroxide monohydrate (5 mg, 0.12 mmol) according to the method of Example 31, followed by purification by reverse-phase HPLC to give 4.5 mg (0.010 mmol, 13%) of the title compound. Purity 99%.
HPLC/MS (30 min) retention time 17.90 min.
LRMS:m/z463(M+1).

### EXAMPLE 41

### 2-(3-[2-1[(Cyclopropylcarbonyl)(ethyl)amino]methyl)-4-(trifluoromethyl)phenyl]-5-fluoro-1H-indol-1-yl}propanoic acid

The title compound of Preparation 129 (70 mg, 0.14 mmol) was treated with lithium hydroxide monohydrate (10 mg, 0.24 mmol) according to the method of Example 31 to give 60 mg (0.12 mmol, 76%) of the title compound as a white solid. Purity 99%.
Partial ¹H NMR (400 MHz, DMSO-d) 74:26 mixture of two rotamers.
Major rotamer δ ppm 7.79 (1 H, s), 7.41 (1 H, s), 7.19 (1 H, d, *J*=8.6 Hz), 0.96 (3 H, t, *J*=6.8 Hz).
Minor rotamer δ ppm 7.90 (1 H, s), 7.51 (1 H, s), 7.25 (1 H, d, *J*=9.8 Hz), 0.86 (3 H, t, *J*=6.8 Hz).
UPLC/MS (3 min) retention time 2.01 min.
LRMS: m/z 477 (M+1).

### EXAMPLE 42

### [3-(2-([(Cyclopropylcarbonyl)(ethyl)amino]methyl}-4-fluorophenyl)-5-fluoro-1H-indol-1-yl]acetic acid

The title compound of Preparation 122 (36 mg, 0.082 mmol) was treated with lithium hydroxide monohydrate (11 mg, 0.26 mmol) according to the method of Example 31 to give 25 mg (0.056 mmol. 71%) of the title compound as a white solid. Purity 96%.
Martial ¹H NMR (400 MHz, DMSO-d) 75:25 mixture of two rotamers.
Major rotamer δ ppm 4.64 (s, 2H).
Minor rotamer δ ppm 4.58 (s, 2H).
HPLC/MS (30 min) retention time 17.02 min.
LRMS: m/z 413 (M+1).

### EXAMPLE 4.3

### {3-[2-{[[(Benzylamino)carbonyl](ethyl)aaino]methyl]-4-(trifluoromethyl)phenyl]-5-fluoro-1H-indol-1-yl}acetic acid

The title compound of Preparation 132 (105 mg, 0.19 mmol) was treated with lithium hydroxide monohydrate (25 mg, 0.60 mmol) according to the method of Example 31 to give 91 mg (0.16 mmol, 82%) of the title compound as a white solid. Purity 90%.
¹H NMR (400 MHz, CHLOROFORM-d) δ ppm 7.50 (2 H, br. s.), 7.34 (1 H, d, *J*=7.0 Hz), 7.11 - 7.23 (3 H, m), 6.94 - 7.07 (3 H, m), 6.89 (2 H, br. s.), 6.67 - 6.83 (1 H, m), 4.57 (1 H, br. s.), 4.47 (2 H, br. s.), 4.43 (2 H, br. s.), 3.99 (2 H, br. s.), 2.95 - 3.03 (2 H, m), 0.75 - 0.90 (3 H, m).
UPLC/MS (3 min) retention time 1.97 min.
LRMS: m/z 528 (M+1).

### EXAMPLE 44

### 2-[4-Chloro-3-[2-([(cyclopropylcarbonyl)(ethyl)amino]methyl}-4-(trifluoromethyl)phenyl]-1H-indol-1-yl}propanoic acid

The title compound of Preparation 130 (35 mg, 0.69 mmol) was treated with lithium hydroxide monohydrate (15 mg, 0.36 mmol) according to the method of Example 31 to give 30 mg (0.061 mmol, 88%) of the title compound as a white solid. Purity 100%.
Partial ¹H NMR (300 MHz, CHLOROFORM-d) 60:40 mixture of two rotamers.
Major rotamer δ ppm 7.39 (1 H, s), 4.30 (1 H, dd).
Minor rotamer δ ppm 7.47 (1 H, br. s.).
UPLC/MS (3 min) retention time 2.04 min.
LRMS: m/z 493 (M+1).

### EXAMPLE 45

### [4-Chloro₋3-(2-1[(cyclopropylcarbonyl)(ethyl)amino]methyl)-4-fluorophenyl)-1H-indol-1-yl]acetic acid

The title compound of Preparation 124 (53 mg, 0.12 mmol) was treated with lithium hydroxide monohydrate (29 mg, 0.69 mmol) according to the method of Example 31 to give 47 mg (0.11 mmol, 95%) of the title compound as a white solid. Purity 100%.
Partial ¹H NMR (400 MHz, DMSO-d) 55:45 mixture of two rotamers.
Major rotamer δ ppm 6.84 (dd, J = 10.1, 2.6 Hz, 2H).
Minor rotamer δ ppm 6.94 (dd, J = 10.1, 2.6 Hz, 2H).
UPLC/MS (3 min) retention time 1.88 min.
LRMS: m/z 429 (M+1).

### EXAMPLE 46

### (3-[2-[(3-Cyclopropyl-5-methyl-4H-1,2,4-triazol-4-yl)methyl]-4-(trifluoromethyl)phenyl]-5-fluoro-1H-indol-1-yl}acetic acid

The title compound of Preparation 134 (60 mg, 0.12 mmol) was treated with lithium hydroxide monohydrate (16 mg, 0.38 mmol) according to the method of Example 31 to give 39 mg (0.082 mmol, 68%) of the title compound as a white solid. Purity 99%. ¹H NMR (400 MHz, DMSO-*d*₆) δ ppm 13.17 (1 H, br. s.), 7.77 - 7.87 (2 H, m), 7.73 (1 H, d, *J*=7.8 Hz), 7.61 (1 H, dd, *J*=8.2, 3.9 Hz), 7.22 - 7.35 (2 H, m), 7.12 (1 H, t, *J*=8.4 Hz), 5.47 (2 H, s), 5.16 (2 H, s), 2.24 (3 H, s), 1.65 - 1.82 (1 H, m), 0.63 - 0.95 (4 H, m).
UPLC/MS (3 min) retention time 1.74 min.
LRMS: m/z 473 (M+1).

### EXAMPLE 47

### (4-Chloro-3-[2-1[(cyclopropylcarbonyl)(ethyl)amino]methyl}-4-(trifluoromethyl)phenyl]-5-fluoro-1H-indol-1-yl}acetic acid

The title compound of Preparation 136 (25 mg, 0.048 mmol) was treated with lithium hydroxide monohydrate (5 mg, 0.12 mmol) according to the method of Example 31 to give 21 mg (0.041 mmol, 87%) of the title compound as a white solid. Purity 98%.
Partial ¹H NMR (400 MHz, DMSO-d₆) 65:35 mixture of two rotamers.
Major rotamer δ ppm 4.57 (s, 2H). Minor rotamer δ ppm 4.80 (s, 2H).
UPLC/MS (3 min) retention time 2.01 min.
LRMS: m/z 497 (M+1).

### EXAMPLE 48

### [4-Chloro-3-(2-1[(cyclopropylcarbonyl)(ethyl)amino]methyl)-4-fluorophenyl)-5-fluoro-1H-indol-1-yl]acetic acid

The title compound of Preparation 138 (45 mg, 0.095 mmol) was treated with lithium hydroxide monohydrate (20 mg, 0.48 mmol) according to the method of Example 31 to give 26 mg (0.058 mmol, 61 %) of the title compound as a white solid. Purity 100%.
Partial ¹H NMR (400 MHz, DMSO-d) 60:40 mixture of two rotamers.
Major rotamer δ 7.58 (1 H, s), 6.89 (1 H, d, *J*=10.2 Hz), 4.45 (2 H, s), 0.94 (3 H, t, *J*=7.0 Hz).
Minor rotamer δ ppm 7.65 (1 H, s), 6.99 (1 H, d, *J*=10,2 Hz), 4.66 (2 H, s), 0.83 (3 H, t, *J*=7.0 Hz).
UPLC/MS (3 min) retention time 1.93 min.
LRMS:m/z447(M+1).

### EXAMPLE 49

### [3-(3-([(Cyclopropylcarbonyl)(ethyl)amino]methyl}pyridin-2-yl)-5-fluoro-1H-indol-1-yl]acetic acid

The crude title compound of Preparation 140 (37 mg) was treated with lithium hydroxide monohydrate (27 mg, 0.64 mmol) according to the method of Example 5 to give 15 mg (0.032 mmol, 20% over two steps) of the title compound as a white solid. Purity 95%.
Partial ¹H NMR (400 MHz, DMSO-d) 57:43 mixture of two rotamers.
Major rotamer δ ppm 5.05 (s, 2H), 4.70 (s, 2H). Minor rotamer 8 ppm 5.06 (s, 2H), 4.93 (s, 2H).
UPLC/MS (3 min) retention time 1.82 min.
LRMS:m/z396(M+1).

### EXAMPLE 50

### {3-[2-{[(Cyclopropylcarbonyl)(ethyl)amino]methyl)-4-(trifluoromethyl)phenyl]-4,5-difluoro-1H-indol-1-yl}acetic acid

The title compound of Preparation 142 (40 mg, 0.079 mmol) was treated with lithium hydroxide monohydrate (20 mg, 0.48 mmol) according to the method of Example 5 to give 36 mg (0.075 mmol, 95%) of the title compound as a white solid. Purity 100%.
Martial ¹H NMR (400 MHz, DMSO-d) 60:40 mixture of two rotamers.
Major rotamer δ ppm 5.09 (2 H, s), 3.37 (2 H, q, *J*=7.0 Hz), 0.96 (3 H, t, *J*=7.0 Hz).
Minor rotamer δ ppm 5.10 (2 H, s), 3.23 (2 H, q, *J*=6.9 Hz), 0.84 (3 H, t, *J*=6.8 Hz).
UPLC/MS (3 min) retention time 1.96 min.
LRMS: m/z 481 (M+1).

### EXAMPLE 51

### [5-Chloro-3-(2-([(cyclopropylcarbonyl)(ethyl)amino]methyl)-4-fluorophenyl)-1H-indol-1-yl]acetic acid

The crude title compound of Preparation 128 (19 mg) was treated with lithium hydroxide monohydrate (5 mg, 0.12 mmol) according to the method of Example 5 followed by purification by HPLC to give 15 mg (0.034 mmol, 21% over two steps) of the title compound as a white solid. Purity 100%.
Partial ¹H NMR (400 MHz, DMSO-d) 60:40 mixture of two rotamers.
Major rotamer δ ppm 4.46 (s, 2H).
Minor rotamer δ ppm 4.66 (s, 2H).
UPLC/MS (3 min) retention time 1.91 min.
LRMS: m/z 429 (M+1).

### EXAMPLE 52

### [3-(2-{[(Cyclopropylcarbonyl)(ethyl)amino]methyl}-4-fluorophenyl)-4-fluoro-1H-indol-1-yl]acetic acid

The crude title compound of Preparation 126 (18 mg) was treated with lithium hydroxide monohydrate (5 mg, 0.12 mmol) according to the method of Example 5 followed by purification by HPLC to give 15 mg (0.036 mmol, 23% over two steps) of the title compound as a white solid. Purity 100%.
Partial ¹H NMR (400 MHz, DMSO-d) 60:40 mixture of two rotamers.
Major rotamer δ ppm 5.08 (s, 2H), 4.43 (s, 2H).
Minor rotamer δ ppm 5.09 (s, 2H), 4.58 (s, 2H).
UPLC/MS (3 min) retention time 1.76 min.
LRMS:m/z413(M+1).

### EXAMPLE 53

### (5-Chloro-3-[2-1[(cyclopropylcarbonyl)(ethyl)amino]methyl}-4-(trifluoromethyl)phenyl]-1H-indol-1-yl}acetic acid

The title compound of Preparation 144 (200 mg, 0.39 mmol) was treated with lithium hydroxide monohydrate (85 mg, 2.0 mmol) according to the method of Example 31 to give 173 mg (0.36 mmol, 92%) of the title compound as a white solid. Purity 100%.
Partial ¹H NMR (400 MHz, DMSO-d) 60:40 mixture of two rotamers.
Major rotamer δ ppm 3.40 (2 H, q, *J*=7.0 Hz), 0.98 (3 H, t, *J*=6.6 Hz).
Minor rotamer δ ppm 3.26 (2 H, q, *J*=7.0 Hz), 0.88 (3 H, t, *J*=6.4 Hz).
HPLC/MS (9 min) retention time 7.28 min.
LRMS:m/z479(M+1).

### EXAMPLE 54

### [3-(2-([(Cyclopropylcarbonyl)(ethyl)amino]methyl)phenyl)-5-fluoro-1H-indol-1-yl]acetic acid

The crude title compound of Preparation 154 (70 mg) was treated with lithium hydroxide monohydrate (59 mg, 1.4 mmol) according to the method of Example 5 to give 21 mg (0.053 mmol, 28% over two steps) of the title compound as a white solid. Purity 100%.
Partial ¹H NMR (300 MHz, DMSO-d) 50:50 mixture of two rotamers.
Rotamer 1 δ ppm 4.72 (s, 2 H).
Rotamer 2 δ ppm 4.54 (s, 2 H).
UPLC/MS (3 min) retention time 1.82 min.
LRMS: m/z 395 (M+1).

### EXAMPLE 55

### [3-(3-[[(Cyclopropylcarbonyl)(ethyl)amino]methyl)pyridin-2-yl)-5-(trifluoromethyl)-1H-indol-1-yl]acetic acid

The title compound of Preparation 149 (40 mg, 0.085 mmol) with treated with lithium hydroxide monohydrate (32 mg, 0.76 mmol) according to the method of Example 5 to give 20 mg (0.045 mmol, 59%) of the title compound as a white solid. Purity 100%.
Partial ¹H NMR (300 MHz, DMSO-d) 50:50 mixture of two rotamers.
Rotamer 1 δ ppm 4.97 (s, 2 H), 4.69 (s, 2 H).
Rotamer 2 δ ppm 4.74 (s, 2 H), 4.69 (s, 2 H).
UPLC/MS (3 min) retention time 1.53 min.
LRMS: m/z 446 (M+1).

### EXAMPLE 56

### (3-[2-[(5-Cyclopropyl-3-methylisoxazol-4-yl)methyl]-4-(trifluoromethyl)phenyl]-5-fluoro-1H-indol-1-yl}acetic acid

The mixture of the title compounds of Preparation 156 (75 mg, 0.15 mmol) was treated with lithium hydroxide monohydrate (8 mg, 0.19 mmol) according to the method of

Example 5. The residue was further purified by Preparative HPLC to give 29 mg (0.061 mmol, 40%) of the title compound as a white solid. Purity 100%.
¹H NMR (400 MHz, DMSO-d) δ 13.11 (s, 1H), 7.72 (d, J = 6.2 Hz, 1 H), 7.67 (d, J = 7.9 Hz, 1 H), 7.62 (d, J = 7.9 Hz, 1H), 7.56 (dd, J = 8.9, 4.4 Hz, 1H), 7.43 (s, 1H), 7.18 (dd, J = 9.9, 2.4 Hz, 1H), 7.08 (td, J = 9.2, 2.3 Hz, 1H), 5.13 (s, 2H), 3.95 (s, 2H), 1.81 - 1.75 (m, 3H), 1.40 (s, 1H), 0.89 - 0.82 (m, 2H), 0.81 - 0.72 (m, 2H).
HPLC/MS (3 min) retention time 2.02 min.
LRMS: m/z 473 (M+1).

### PHARMACOLOGICAL ACTIVITY

### CRTh2 radioligand bending assay

The assay is performed using membranes obtained from CHO.K1 cells stably overexpressing the CRTh2 receptor and ³[H]PGD₂ as radioligand. The assay is performed incubating 2-4 µg of membranes per well with the compound to be tested and 5 nM ³[H]PGD₂ in incubation buffer (50 mM HEPES pH 7.0, 10 mM MgCl₂, 1 mM EDTA, 0.2% BSA) for 2 h at room temperature. The reaction is terminated by filtering in GF/C plates pre-treated with 0.3% de polyetilenimine buffer and washing 3 times with wash buffer (50 mM HEPES pH 7.4, 0.5 M NaCl). After washing, plates are dried and scintillation cocktail Optiphase Hisafe II added. The radioactivity retained in the filter is counted using a Microbeta liquid scintillation counter. Compound IC₅₀ are determined using Excel XL-fit for calculations.

### CRTh2 GTPγS antagonism bending assay

The assay is performed using membranes obtained from CHO.K1 cells stably overexpressing the CRTh2 receptor and [³⁵S]-GTPγS as radioligand. Assay is performed pre-incubating 4-8 µg of membranes per well with the compound to be tested for 1h, followed by incubation with PGD₂ at 50 nM (EC₈₀), 0.1 nM [³⁵S]-GTPγS in incubation buffer (20 mM Herpes, 10 mM MgCl₂, 100 mM NaCl, 10 µM GDP, 10 µg/ml Saponine and 0.2% BSA) for 2 h at room temperature. The reaction is terminated by filtering in GF/C plates pre-treated with 20 mM HEPES, 10 mM MgCl₂, 100 mM NaCl and washing 6 times with wash buffer (20 mM NaH₂PO4, 20 mM Na₂HPO4). After washing, plates are dried and scintillation buffer Optiphase added. The radioactivity retained in the filter is counted using a Microbeta liquid scintillation counter. Compound IC₅₀ are determined using Excel XL-fit for calculations.

### CRTh2 Isolated cell eosinophil cell shape assay

Citrated whole blood is obtained from consenting donors and polymorphonuclear leukocytes obtained by gradient centrifugation on Polymorphprep for 40 min at 500G brake off. PMN fraction is resuspended in calcium free buffer (10 mM HEPES, 10 mM glucose, 0.1 % BSA in calcium free PBS) and centrifuged for 10 min at 400G at room temperature. Lysis of red blood cells is performed by adding 20 ml of 0.2% saline buffer (0.2% NaCl) for 40 s and stopped by adding 20 ml of 1.6% saline buffer (1.6% NACl). For compound treatment 90 µl of PMNs cells resuspended at a density of 5 x 10⁶ cells/ml in 10 mM HERPES, 10 mM glucose, 0.1 % BSA in calcium containing PBS are incubated for 10 min with 10 µl of compound to be tested followed by incubation with 10 µl of 500 nM PGD₂ for exactly 4 min at 37ºC. Fixation is performed by addition of 0.2 ml of ice-cold 1:5 diluted CellFix (BD Biosciences) and immediately analysed on a FACSCalibur (BD Biosciences). Eosinophils are gated on the basis of autofluorescence in the FL2 channels and shape change of 600 cells is assayed by forward scatter and side scatter analysis. Compound IC₅₀ are determined using Excel XL-fit for calculations.

### CRTh2 whole blood eosinophil cell shape assay

Whole blood is obtained from consenting donors mixed with heparin and kept in rotation until use. For compound treatment, 90 µl of blood are mixed with 10 µl of compound to be tested and incubated for 10 min at room temperature followed by addition of 10 µl of 500 nM PGD₂ for exactly 4 min at 37°C. Reaction was stopped by placing the tubes on ice and adding of 0.25 ml of ice-cold 1:5 diluted CellFix (BD Biosciences). Red blood cells are lysed in two steps by adding 2 ml of lysis solution (150 mM ammonium chloride, 10 mM potassium hydrogencarbonate), incubating for 20 and 10 min respectively and centrifuging at 300G for 5 min at 6ºC. The pellet is resuspended in 0.2 ml of fixative solution and immediately canalized on a FACSCalibur (BD Biosciences). Eosinophils are gated on basis of autofluorescence in the FL2 channels and shape change of 600 cells is assayed by forward scatter and side scatter analysis. Compound IC₅₀ are determined using Excel XL-fit for calculations.

| **Example** | **GTPγ Binding (nM)** |
|---|---|
| 1 | 18 |
| 2 | 6 |
| 3 | 15 |
| 4 | 9 |
| 5 | 16 |
| 8 | 7 |
| 9 | 13 |
| 11 | 6 |
| 14 | 4 |
| 15 | 8 |
| 16 | 17 |
| 17 | 10 |
| 18 | 13 |
| 19 | 7 |
| 22 | 10 |
| 23 | 8 |
| 24 | 9 |
| 27 | 14 |
| 30 | 18 |
| 31 | 19 |
| 33 | 18 |
| 37 | 11 |
| 39 | 13 |
| 40 | 3 |
| 42 | 6 |
| 43 | 16 |
| 46 | 9 |
| 50 | 16 |
| 52 | 7 |
| 54 | 4 |

### COMBINATION PRODUCT

The compounds of the invention may also be combined with other active compounds in the treatment of diseases indicated above. For example the compounds of the present invention can be combined with active substances which are known to be useful in the treatment of these diseases.

Examples of such active substances are (a) anticholinergics, such as Aclidinium Bromide or Tiotropium, (b) corticosteroids, or gluococorticoids such as prednisone or methylprednisolone; (c) antihistamines, such as Ebastine, Ranitidine, ABT-239 or JNJ 7777120, (d) beta-2 agonists, such as salmeterol or formoterol, (e) chemokine receptor antagonists, such as Maraviroc or Enfuvirtide, (f) Leukotriene receptor antagonists, (g) JAK inhibitors such as tasocitinib citrate or INCB018424, (h) Syk inhibitors such as R-112, (i) Phosphosdiesterase IV inhibitors such as GRC-4039, (j) p38 Inhibitors such as ARRY-797; (k) PKC inhibitors such as NVP-AEB071; (I) 5-lipoxygenase activating protein inhibitors, such as Veliflapon; (m) 5-lipoxygenase inhibitors, (n) CYSLTR1 antagonists, such as Montelukast, Pranlukast or Zafirlukast; (o) CYSLTR2 antagonists, such as Pranlukast, Zafirlukast or Tipilukast; (p) BLT1 antagonists, (q) BLT2 antagonists, (r) Thromboxane A2 antagonists such as Ramatroban; (s) DP1 receptor antagonists, such as Laropiprant; (t) DP1 receptor agonists, such as BW-245C; (u) IP receptor agonists, such as RO-1138452; (v) anti-IgE, such as Omalizumab; (w) IL5 antibody, such as Mepolizumab; (x) leukotriene formation inhibitors, (y) bronchodilators, such as pirbuterol, epinephrine, ephedrine, salbutamol or salmeterol; (z) decongestants, such as ephedrine, Levo-methamphetamine, Naphazoline, Oxymetazoline, Phenylephrine, Phenylpropanolamine, Propylhexedrine, Pseudoephedrine, Synephrine or Tetrahydrozoline; (aa) mucolytics such as Acetylcysteine, Ambroxol, Bromhexine, Carbocisteine, Domiodol, Eprazinone, Erdosteine, Letosteine, Neltenexine, Sobrerol, Stepronin or Tiopronin; (bb) antitussives, such as dectromethorphan; (cc) analgesics such as aspirin, paracetamol, rofecoxid, celecoxib, morphine, codeine, oxycodone, hydrocodone, dihydromorphine or flupirtine and (dd) Expectorants such Antimony pentasulfide, Guaiacolsulfonate, Guaifenesin, Potassium iodide or Tyloxapol.

Accordingly, another embodiment of the invention is a combination product comprising (i) at least a compound of formula (I) as defined previously, and (ii) one or more active ingredients as described above, for simultaneous, separate or sequential use in the treatment of the human or animal body.

The combinations of the invention may be used in the treatment of disorders which are susceptible to amelioration by CRTh2 receptor antagonists. Thus, the present application encompasses methods of treatment of these disorders, as well as the use of the combinations of the invention in the manufacture of a medicament for the treatment of these disorders.

Preferred examples of such disorders are asthma, chronic obstructive pulmonary disease (COPD), allergic rhinitis, atopic dermatitis, psoriasis, emphysema, eosinophilic bronchitis, being more preferably asthma, chronic obstructive pulmonary disease (COPD), allergic rhinitis and atopic dermatitis.

The amount of each active which is required to achieve a therapeutic effect will, of course, vary with the particular active, the route of administration, the subject under treatment, and the particular disease or disorder being treated.

The active ingredients may be administered from 1 to 6 times a day, sufficient to exhibit the desired activity. Preferably, the active ingredients are administered once or twice a day, most preferably once a day.

When combinations of actives are used, it is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

Examples of suitable β2-agonists that can be combined with the compounds of formula (I) are terbutaline sulphate, eformoterol fumarate, formoterol fumarate, bambuterol, ibuterol, isoprenaline hydrochloride, dopexamine, metaprotenerol, tulobuterol, procaterol hydrochloride, sibenadet hydrochloride, mabuterol hydrochloride, albuterol sulphate, salbutamol sulphate, salmefamol, salmeterol xinafoate, carmoterol hydrochloride, (R)-albuterol hydrochloride, Levalbuterol hydrochloride; Levosalbutamol hydrochloride; (-)-Salbutamol hydrochloride, formoterol, (R,R)-Formoterol tartrate; Arformoterol tartrate, sulfonterol, Bedoradrine sulphate, indacaterol, Trantinterol hydrochloride, Milveterol hydrochloride, Olodaterol, fenoterol hydrobromide, rimoterol hydrobromide, riproterol hydrochloride, Vilanterol broxaterol, pirbuterol hydrochloride, bitolterol mesylate, clenbuterol hydrochloride, AZD-3199, GSK-159802; GSK-597901, GSK-678007, GSK-961081; 4-[2-[3-(1H-Benzimidazol-1-yl)-1,1-dimethylpropylamino]-1-hydroxyethyl]-2-(4-methoxybenzylamino)phenol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-domethoxyphenyl)-2-methyl-2-propylamino]ethanol, 1-[2H-5-hydroxy-3-oxo-4H-1,4-benzoxazin-8-yl]-2-[3-(4-n-butyloxyhenyl)-2-methyl-2-propylamino]ethanol, KUL-1248, HOKU-81, SM-110444, RP-58802B, LAS 100977 and compounds described in PCT patent applications Nos. WO 2007/124898, WO 2006/122788A1, WO 2008/046598, WO 2008095720, WO 2009/068177 and WO 2010/072354.

Examples of suitable corticosteroids and glucocorticoids that can be combined with the compounds of formula (I) are prednisolone, methylprednisolone, dexamethasone, dexamethasone cipacilata, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, butixocort propionate, RS-85095, CGP-13774, GW-250495, deltacortisone, NO-Prednisolone, etiprednol dicloacetate, QAE-397, 7beta-OH-EPIA, RPR-106541, deprodone propionate, fluticasone propionate, fluticasone furoate, halobetasol propionate, Ioteprednol etabonate, betamethasone butyrate propionate, flunisolide, prednisone, dexamethasone sodium phosphate, triamcinolone, betamethasone 17-valerate, betamethasone, betamethasone dipropionate, 21-Chloro-11beta-hydroxy-17alpha-[2-(methylsulfanyl)acetoxy]-4-pregnene-3,20-dione, Desisobutyrylciolesonide, hydrocortisone acetate, hydrocortisone sodium succinate, prednisolone sodium phosphate and hydrocortisone probutate, Prednisolone sodium metasulfobenzoate and clobetasol propionate.

Examples of suitable anticholinergics that can be combined with the compounds of formula (I) are tiotropium salts, oxitropium salts, flutropium salts, ipratropium salts, glycopyrronium salts, trospium salts, zamifenacin, revatropate, espatropate, darotropium bromide, CI-923, NPC-14695, BEA-2108, 3-[2-Hydroxy-2,2-bis(2-thienyl)acetoxy]-1-(3-phenoxypropyl)-1-azoniabicyclo[2.2.2]octane salts (in particular aclidinium salts, more preferably aclidinium bromide), 1-(2-Phenylethyl)-3-(9H-xanthen-9-ylcarbonyloxy)-1-azoniabicyclo[2.2.2]octane salts, 2-oxo-1,2,3,4-tetrahydroquinazoline-3-carboxylic acid endo-8-methyl-8-azabicyclo[3.2.1]oct-3-yl ester salts (DAU-5884), 3-(4-Benzylpiperazin-1-yl)-1-cyclobutyl-1-hydroxy-1-phenylpropan-2-one (NPC-14695), N-[1-(6-Aminopyridin-2-ylmethyl)piperidin-4-yl]-2(R)-[3,3-difluoro-1 (R)-cyclopentyl]-2-hydroxy-2-phenylacetamide (J-104135), 2(R)-Cyclopentyl-2-hydroxy-N-[1-[4(S)-methylhexyl]piperidin-4-yl]-2-phenylacetamide (J-106366), 2(R)-Cyclopentyl-2-hydroxy-N-[1-(4-methyl-3-pentenyl)-4-piperidinyl]-2-phenylacetamide (J-104129), 1-[4-(2-Aminoethyl)piperidin-1-yl]-2(R)-[3,3-difluorocyclopent-1(R)-yl]-2-hydroxy-2-phenylethan-1-one (Banyu-280634), N-[N-[2-[N-[1-(Cyclohexylmethyl)piperidin-3(R)-ylmethyl]carbamoyl]ethyl]carbamoylmethyl]-3,3,3-triphenylpropionamide (Banyu CPTP), 2(R)-Cyclopentyl-2-hydroxy-2-phenylacetic acid 4-(3-azabicyclo[3.1.0]hex-3-yl)-2-butynyl ester (Ranbaxy 364057), 3(R)-[4,4-Bis(4-fluorophenyl)-2-oxoimidazolidin-1-yl]-1-methyl-1-[2-oxo-2-(3-thienyl)ethyl]pyrrolidinium iodide, N-[1-(3-Hydroxybenzyl)-1-methylpiperidinium-3(S)-yl]-N-[N-[4-(isopropoxycarbonyl)phenyl]carbamoyl]-L-tyrosinamide trifluoroacetate, UCB-101333, Merck's OrM3, 7-endo-(2-hydroxy-2,2-diphenylacetoxy)-9,9-dimethyl-3-oxa-9-azoniatricyclo[3.3.1.0(2,4)]nonane salts, 3(R)-[4,4-Bis(4-fluorophenyl)-2-oxoimidazolidin-1-yl]-1-methyl-1-(2-phenylethyl)pyrrolidinium iodide, trans-4-[2-[Hydroxy-2,2-(dithien-2-yl)acetoxy]-1-methyl-1-(2-phenoxyethyl)piperidinium bromide from Novartis (412682), 7-(2,2-diphenylpropionyloxy)-7,9,9-trimethyl-3-oxa-9-azoniatricyclo[3.3.1.0*2,4*]nonane salts, 7-hydroxy-7,9,9-trimethyl-3-oxa-9-azoniatricyclo[3.3.1.0*2,4*]nonane 9-methyl-9H-fluorene-9-carboxylic acid ester salts, all of them optionally in the form of their racemates, their enantiomers, their diastereomers and mixtures thereof, and optionally in the form of their pharmacologically-compatible acid addition salts. Among the salts chlorides, bromides, iodides and methanesulphonates are preferred.

Examples of suitable anti-histamines that can be combined with the compounds of formula (I) are Methapyrilene, Mequitazine, Azelastine hydrochloride, Ranitinide, Acrivastine, Emedastine difumarate, Emedastine fumarate, Loratadine, Cyproheptadine hydrochloride, Diphenhydramine hydrochloride, Doxepin hydrochloride, Promethazine hydrochloride, Levocabastine hydrochloride, Desloratadine, Cinnarizine, Setastine hydrochloride, Mizolastine, Ebastine, Cetirizine hydrochloride, Epinastine hydrochloride, Olopatadine hydrochloride, Bepotastine besilate, Triprolidine hydrochloride, Rupatadine fumarate, Fexofenadine hydrochloride, Levocetirizine dihydrochloride, Ketotifen, Azatadine maleate, Dimethindene maleate, Clemastine fumarate, Alcaftadine, Bilastine, ABT-239, JNJ7777120, Vapitadine hydrochloride, AZD-1744, GSK-1004723D, GSK-835726, SUN-1334H.

Examples of suitable PDE4 inhibitors that can be combined with the compounds of formula (I) are benafentrine dimaleate, etazolate, denbufylline, rolipram, cipamfylline, zardaverine, arofylline, filaminast, tipelukast, tofimilast, piclamilast, tolafentrine, mesopram, drotaverine hydrochloride, lirimilast, roflumilast, cilomilast, oglemilast, apremilast, tetomilast, filaminast, (R)-(+)-4-[2-(3-Cyclopentyloxy-4-methoxyphenyl)-2-phenylethyl]pyridine (CDP-840), N-(3,5-Dichloro-4-pyridinyl)-2-[1-(4-fluorobenzyl)-5-hydroxy-1H-indol-3-yl]-2-oxoacetamide (GSK-842470), 9-(2-Fluorobenzyl)-N6-methyl-2-(trifluoromethyl)adenine (NCS-613), N-(3,5-Dichloro-4-pyridinyl)-8-methoxyquinoline-5-carboxamide (D-4418), 3-[3-(Cyclopentyloxy)-4-methoxybenzyl]-6-(ethylamino)-8-isopropyl-3H-purine hydrochloride (V-11294A), 6-[3-(N,N-Dimethylcarbamoyl)phenylsulfonyl]-4-(3-methoxyphenylamino)-8-methylquinoline-3-carboxamide hydrochloride (GSK-256066), 4-[6,7-Diethoxy-2,3-bis(hydroxymethyl)naphthalen-1-yl]-1-(2-methoxyethyl)pyridin-2(1H)-one (T-440), (-)-trans-2-[3'-[3-(N-Cyclopropylcarbamoyl)-4-oxo-1,4-dihydro-1,8-naphthyridin-1-yl]-3-fluorobiphenyl-4-yl]cyclopropanecarboxylic acid, MK-0873, CDC-801, UK-500001, BLX-914, 2-carbomothoxy-4-cyano-4-(3-cyclopropylmethoxy-4-difluroromethoxyphenyl)cyclohexan1-one, cis [4-cyano-4-(3-cyclopropylmethoxy-4-difluoromethoxyphenyl)cyclohexan-1-ol, 5(S)-[3-(Cyclopentyloxy)-4-methoxyphenyl]-3(S)-(3-methylbenzyl)piperidin-2-one (IPL-455903), ONO-6126 (Eur Respir J 2003, 22(Suppl. 45): Abst 2557) and the salts claimed in the PCT patent applications number WO 03/097613, WO 2004/058729, WO 2005/049581, WO 2005/123693, WO 2005/123692, and WO 2010/069504.

Examples of suitable Syk kinase inhibitors that can be combined with the compounds of formula (I) are fosfamatinib (from Rigel), R-348 (from Rigel), R-343 (from Rigel), R-112 (from Rigel), piceatannol, 2-(2-Aminoethylamino)-4-[3-(trifluoromethyl)phenylamino] pyrimidine-5-carboxamide, R-091 (from Rigel), 6-[5-Fluoro-2-(3,4,5-trimethoxyphenylamino)pyrimidin-4-ylamino]-2,2-dimethyl-3,4-dihydro-2H-pyrido[3,2-b][1,4]oxazin-3-one benzenesulfonate (R-406 from Rigel), 1-(2,4,6-Trihydroxyphenyl)-2-(4-methoxyphenyl)ethan-1-one, N-[4-[6-(Cyclobutylamino)-9H-purin-2-ylamino]phenyl]-N-methylacetamide (QAB-205 from Novartis), 2-[7-(3,4-Dimethoxyphenyl)imidazo[1,2-c]pyrimidin-5-ylamino]pyridine-3-carboxamide dihydrochloride (BAY-61-3606 from Bayer) and AVE-0950 (from Sanofi-Aventis).

Particularly preferred combination products according to the invention comprise a compound of formula (I) and a therapeutically effective amount of one or more additional therapeutic agents selected from the group consisting of mometasone furoate, ciclesonide, budesonide, fluticasone propionate, fluticasone furoate, betamethasone valerate, clobetasol propionate, tiotropium salts, glycopyrronium salts, 3-[2-Hydroxy-2,2-bis(2-thienyl)acetoxy]-1-(3-phenoxypropyl)-1-azoniabioyclo[2.2.2]octane salts (in particular aclidinium salts, preferably aclidinium bromide), 1-(2-Phenylethyl)-3-(9H-xanten-9-ylcarbonyloxy)-1-azoniabicyclo[2.2.2]octane salts, formoterol, salmeterol, indacaterol, carmoterol, LAS 100977, compounds described in PCT patent applications Nos. WO 2008/077639, WO 2009/021696, WO 2009/153043, and WO 2010/083975 (in particular amino(iso)nicotinic acid derivatives selected from the group consisting of 2-(3'-ethoxy-3-(trifluoromethoxy)biphenyl-4-ylamino)nicotinic acid, 2-(3,5-difluoro-3'-methoxybiphenyl-4-ylamino)nicotinic acid and 2-(3,5-difluoro-2-methylbiphenyl-4-ylamino)nicotinic acid; and azabiphenylaminobenzoic acid derivatives selected from the group consisting of 5-cyclopropyl-2-(2-(2,6-difluorophenyl)pyrimidin-5-ylamino)benzoic acid, 5-cyclopropyl-2-((2-(2-(trifiuoromethyl)phenyl)pyrimidin-5-yl)amino)benzoic acid and 5-methyl-2-((6-(2,3-difluorophenyl)pyridin-3-yl)amino)benzoic acid), methapyrilene, cetirizine, loratadine, ebastine, desloratadine, fexofenadine, azelastine, levocabastine, olopatadine, Montelukast, picremolimus, tacrolimus, mupiricin, retapamulin, clotrimazole, ketoconazole and terbinafine.

Preferred combination products according to the invention comprises a compound of formula (I) and a therapeutically effective amount of one or more additional therapeutic agents selected from the group consisting of mometasone furoate, ciclesonide, budesonide, fluticasone propionate, fluticasone furoate, tiotropium salts, glycopyrronium salts, 3-[2-Hydroxy-2,2-bis(2-thienyl)acetoxy]-1-(3-phenoxypropyl)-1-azoniabicyclo[2.2.2]octane salts (in particular aclidinium salts, preferably aclidinium bromide), 1-(2-Phenylethyl)-3-(9H-xanthen-9-ylcarbonyloxy)-1-azoniabicyclo[2.2.2]octane salts, rolipram, roflumilast, cilomilast and the compounds claimed in the PCT patent applications number WO 03/097613, WO 2004/058729, WO 2005/049581, WO 2005/123693, WO 2005/123692, and WO 2010/069504.

Thus, in one aspect of the invention, the combination product comprises a compound of formula (I) and a corticosteroid. Particularly preferred corticosteroids are those selected from the group consisting of mometasone furoate, ciclesonide, budesonide, fluticasone furoate, fluticasone propionate, betamethasone valerate and clobetasol propionate.

In another aspect of the invention, the combination product comprises a compound of formula (I) and an anticholinergic agent. Particularly preferred anticholinergic agents are those selected from the group consisting of tiotropium salts, glycopirronium salts, 3-[2-Hydroxy-2,2-bis(2-thienyl)acetoxy]-1-(3-phenoxypropyl)-1-azoniabicyclo[2.2.2]octane salts and 1-(2-Phenylethyl)-3-(9H-xanthen-9-ylcarbonyloxy)-1-azoniabicyclo[2.2.2]-octane salts. The combination product may further comprise a corticosteroid selected from the group consisting of mometasone faroata, ciclesonide, budesonide, fluticasone furoate and fluticasone propionate.

In a still other aspect of the invention, the combination product comprises a compound of formula (I) and a suitable β2-agonist. Particularly preferred β2-agonists are those selected from the group consisting of formoterol, salmeterol, indacatarol, carmoterol, LAS100977 and compound described in PCT patent applications Nos. WO 2007/124898, WO 2006/122788A1, WO 2008/046598, WO 2008095720, WO 2009/068177 and WO 2010/072354. The composition may further comprise a corticosteroid selected from the group consisting of mometasone furoate, ciclesonide, budesonide, fluticasone furoate, and fluticasone propionate. In addition to the compound of formula (I) and to the β2-agonist, the combination product may further comprise an anticholinergic agent selected from the group consisting of tiotropium salts, glycopyrronium salts, 3-[2-Hydroxy-2,2-bis(2-thienyl)acetoxy]-1-(3-phanaxyprapyl)-1-azoniabicyclo[2.2.2] octane salts and 1-(2-Phenylethyl)-3-(9H-xanthen-9-ylcarbonyloxy)-1-azoniabicydo[2.2.2] octane salts.

In a still other aspect of the invention, the combination product comprises a compound of formula (I) and a PDE4 inhibitor. Particularly preferred PDE4 inhibitors are those selected from the group consisting of rolipram, roflumilast, cilomilast and the compounds claimed in the PCT patent applications number WO 03/097613, WO 2004/058729, WO 2005/049581, WO 2005/123693, WO 2005/123692, and WO 2010/069504. The combination product may further comprise a corticosteroid selected from the group consisting of mometasone furoate, ciclesonide, budesonide, fluticasone furoate and fluticasone propionate. In addition to the compound of the invention and to the PDE4 inhibitor, the combination product may further comprise an anticholinergic agent selected from the group consisting of tiotropium salts, glycopirronium salts, 3-[2-Hydroxy-2,2-bis(2-thienyl)acetoxy]-1-(3-phenoxypropyl)-1-azoniabicyclo[2.2.2] octane salts and 1-(2-Phenylethyl)-3-(9H-xanthen-9-ylcarbonyloxy)-1-azoniabicyclo[2.2.2] octane salts.

The active compounds in the combination product may be administered together in the same pharmaceutical composition or in different compositions intended for separate, simultaneous, concomitant or sequential administration by the same or a different route.

It is contemplated that all active agents would be administered at the same time, or very dose in time. Alternatively, one or two actives could be administered in the morning and the other (s) later in the day. Or in another scenario, one or two actives could be administered twice daily and the other (s) once daily, either at the same time as one of the twice-a-day dosing occurred, or separately. Preferably at least two, and more preferably all, of the actives would be administered together at the same time. Preferably, at least two, and more preferably all actives would be administered as an admixture.

### PHARMACEUTICAL COMPOSITIONS

Pharmaceutical compositions according to the present invention comprise the compounds of the invention in association with a pharmaceutically acceptable diluent or carrier.

As used herein, the term pharmaceutical composition refers to a mixture of one or more of the compounds described herein, or physiologically/pharmaceutically acceptable salts, solvates, N-oxides, stereoisomers, deuterated derivatives thereof or prodrugs thereof, with other chemical components, such as physiologically/pharmaceutically acceptable carriers and excipient. The purpose of a pharmaceutical composition is to facilitate administration of a compound to an organism.

As used herein, a physiologically/pharmaceutically acceptable diluent or carrier refers to a carrier or diluent that does not cause significant irritation to an organism and does not abrogate the biological activity and properties of the administered compound.

A pharmaceutically acceptable excipient refers to an inert substance added to a pharmaceutical composition to further facilitate administration of a compound.

The invention further provides pharmaceutical compositions comprising the compounds of the invention in association with a pharmaceutically acceptable diluent or carrier together with one or more other therapeutic agents such as the previously described for use in the treatment of a pathological condition or disease susceptible to amelioration by CRTh2 receptor antagonists.

The invention is also directed to pharmaceutical compositions of the invention for use in the treatment of a pathological disease or disorder susceptible to amelioration by CRTh2 receptor antagonists, in particular wherein the pathological disease or disorder is as described above.

The invention also provides a method of treatment of a pathological condition or disease susceptible to amelioration by CRTh2 receptor antagonists in particular wherein the pathological condition or disease described above, comprising administering a therapeutically effective amount of a pharmaceutical composition of the invention.

The present invention also provides pharmaceutical compositions which comprise, as an active ingredient, at least a compound of formula (I) or a pharmaceutically acceptable salt, solvate, N-oxide or deuterated derivative thereof in association with a pharmaceutically acceptable excipient such as a carrier or diluent. The active ingredient may comprise 0.001% to 99% by weight, preferably 0.01% to 90% by weight, of the composition depending upon the nature of the formulation and whether further dilution is to be made prior to applications. Preferably the compositions are made up in a form suitable for oral, inhalation, topical, nasal, rectal, percutaneous or injectable administration.

Pharmaceutical compositions suitable for the delivery of compounds of the invention and methods for their preparation will be readily apparent to those skilled in the art. Such compositions and methods for their preparation can be found, for example, in Remington: The Science and Practice of Pharmacy, 21st Edition, Lippincott William & Wilkins, Philadelphia, Pa., 2001.

The pharmaceutically acceptable excipients which are admixed with the active compound or salts of such compound, to form the compositions of this invention are well-known per se and the actual excipients used depend inter alia on the intended method of administering the compositions. Examples, without limitation, of excipients include calcium carbonate, calcium phosphate, various sugars and types of starch, cellulose derivatives, gelatin, vegetable oils and polyethylene glycols.

Additional suitable carriers for formulations of the compounds of the present invention can be found in Remington: The Science and Practice of Pharmacy, 21st Edition, Lippincott Williams & Wilkins, Philadelphia, Pa., 2001.

### i) Oral Administration

The compounds of the invention may be administered orally (peroral administration; per os (latin)). Oral administration involve swallowing, so that the compound is absorbed from the gut and delivered to the liver via the portal circulation (hepatic first pass metabolism) and finally enters the gastrointestinal (GI) tract.

Compositions for oral administration may take the form of tablets, retard tablets, sublingual tablets, capsules, inhalation aerosols, inhalation solutions, dry powder inhalation, or liquid preparations, such as mixtures, solutions, elixirs, syrups or suspensions, all containing the compound of the invention; such preparations may be made by methods well-known in the art. The active ingredient may also be presented as a bolus, electuary or paste.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent.

Moulded tablets may be made by moulding in a suitable machine a mixture of the powdered compound moistened with an inert liquid diluent. The tablets may optionally be coated or scored and may be formulated so as to provide slow or controlled release of the active ingredient therein.

For tablet dosage forms, depending on dose, the drug may make up from 1 wt% to 80 wt% of the dosage form, more typically from 5 wt% to 60 wt% of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl- substituted hydroxypropyl cellulose, starch, pregelatinized starch and sodium alginate. Generally, the disintegrant will comprise from 1 wt% to 25 wt%, preferably from 5 wt% to 20 wt% of the dosage form.

Binders are generally used to impart cohesive qualities to a tablet formulations. Suitable binders include microcrystalline cellulose, gelatin, sugars, polyethylene glycol, natural and synthetic gums, polyvinylpyrrolidone, pregelatinized starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate. Tablets may also optionally include surface active agents, such as sodium lauryl sulfate and polysorbate 80, and glidants such as silicon dioxide and talc. When present, surface active agents are typically in amounts of from 0.2 wt% to 5 wt% of the tablet, and glidants typically from 0.2 wt% to 1 wt% of the tablet.

Tablets also generally contain lubricants such as magnesium stearate, calcium stearate, zinc stearate, sodium stearyl fumarate, and mixtures of magnesium stearate with sodium lauryl sulphate. Lubricants generally are present in amounts from 0.25 wt% to 10 wt%, preferably from 0.5 wt% to 3 wt% of the tablet. Other conventional ingredients include anti-oxidants, colorants, flavoring agents, preservatives and taste-masking agents.

Exemplary tablets contain up to about 80 wt% drug, from about 10 wt% to about 90 wt% binder, from about 0 wt% to about 85 wt% diluent, from about 2 wt% to about 10 wt% disintegrant, and from about 0.25 wt% to about 10 wt% lubricant. Tablet blends may be compressed directly or by roller to form tablets. Tablet blends or portions of blends may alternatively be wet-, dry-, or melt-granulated, melt congealed, or extruded before tabletting. The final formulation may include one or more layers and may be coated or uncoated; or encapsulated.

The formulation of tablets is discussed in detail in "Pharmaceutical Dosage Forms: Tablets, Vol. 1 ", by H. Lieberman and L. Lachman, Marcel Dekker, N.Y.,, 1980.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Solid formulations for oral administration may be formulated to be immediate and/or modified release. Modified release formulations induce delayed-, sustained-, pulsed-, controlled-, targeted and programmed release.

Suitable modified release formulations are described in U.S. Patent No. 6,106,864. Details of other suitable release technologies such as high energy dispersions and osmotic and coated particles can be found in Verma et al, Pharmaceutical Technology On-line, 25(2), 1-14 (2001). The use of chewing gum to achieve controlled release is described in WO 00/35298. The disclosures of these references are incorporated herein by reference in their entireties.

Liquid formulations include suspensions, solutions, syrups and elixirs. Such formulations may be used as fillers in soft or hard capsules and typically include a carrier, for example, water, ethanol, polyethylene glycol, propylene glycol, methylcellulose, or a suitable oil, and one or more emulsifying agents and/or suspending agents. The solutions may be aqueous solutions of a soluble salt or other derivative of the active compound in association with, for example, sucrose to form a syrup. The suspensions may comprise an insoluble active compound of the invention or a pharmaceutically acceptable salt thereof in association with water, together with a suspending agent or flavouring agent. Liquid formulations may also be prepared by the reconstitution of a solid, for example, from a sachet.

### ii) Oral mucosal administration

The compounds of the invention can also be administered via the oral mucosal administration. Within the oral mucosal cavity, delivery of drugs is classified into three categories: (a) sublingual delivery, which is systemic delivery of drugs through the mucosal membranes lining the floor of the mouth, (b) buccal delivery, which is drug administration through the mucosal membranes lining the cheeks (buccal mucosa), and (c) local delivery, which is drug delivery into the oral cavity.

Pharmaceutical products to be administered via the oral mucosal can be designed using mucoadhesive, quick dissolve tablets and solid lozenge formulations, which are formulated with one or more mucoadhesive (bioadhesive) polymers (such as hydroxy propyl cellulose, polyvinyl pyrrolidone, sodium carboxymethyl cellulose, hydroxy propyl methyl cellulose, hydroxy ethyl cellulose, polyvinyl alcohol, polyisobutylene or polyisoprene); and oral mucosal permeation enhancers (such as butanol, butyric acid, propranolol, sodium lauryl sulphate and others)

### iii) Inhaled administration

The compounds of the invention can also be administered by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurized container, pump, spray, atomizer (preferably an atomizer using electrohydrodynamics to produce a fine mist), or nebulizer, with or without the use of a suitable propellant, such as 1 ,1 ,1 ,2-tatrafluoroethane or 1 ,1 ,1 ,2,3,3,3-heptafluoropropane. For intranasal use, the powder may include a bioadhesive agent, for example, chitosan or cyclodextrin.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 0.001-500 mg of active ingredient or the equivalent amount of a pharmaceutically acceptable salt thereof. Alternatively, the active ingredient (s) may be presented without excipients.

Packaging of the formulation may be suitable for unit dose or multi-dose delivery. In the case of multi- dose delivery, the formulation can be pre-metered or metered in use. Dry powder inhalers are thus classified into three groups: (a) single dose, (b) multiple unit dose and (c) multi dose devices.

For inhalers of the first type, single doses have been weighed by the manufacturer into small containers, which are mostly hard gelatine capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients.

Other drawbacks related to the use of hard gelatine capsules for inhalation powders are (a) poor protection against moisture uptake from the ambient air, (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported (e. g. Nielsen et al, 1997).

Some capsule inhalers have a magazine from which individual capsules can be transferred to a receiving chamber, in which perforation and emptying takes place, as described in WO 92/03175. Other capsule inhalers have revolving magazines with capsule chambers that can be brought in line with the air conduit for dose discharge (e. g. WO91/02558 and GB 2242134). They comprise the type of multiple unit dose inhalers together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose inhalers do not contain pre-measured quantities of the powder formulation. They consist of a relatively large container and a dose measuring principle that has to be operated by the patient. The container bears multiple doses that are isolated individually from the bulk of powder by volumetric displacement. Various dose measuring principles exist, including rotatable membranes (Ex. EP0069715) or disks (Ex. GB 2041763; EP 0424790; DE 4239402 and EP 0674533), rotatable cylinders (Ex. EP 0166294; GB 2165159 and WO 92/09322) and rotatable frustums (Ex. WO 92/0771), all having cavities which have to be filled with powder from the container. Other multi dose devices have measuring slides (Ex. US 5201308 and WO 97/00703) or measuring plungers with a local or circumferential recess to displace a certain volume of powder from the container to a delivery chamber or an air conduit (Ex. EP 0505321, WO 92/04068 and WO 92/04928), or measuring slides such as the Genuair® (formerly known as Novolizer SD2FL), which is described the following patent applications Nos: WO97/000703, WO03/000325 and WO2006/008027.

Reproducible dose measuring is one of the major concerns for multi dose inhaler devices.

The powder formulation has to exhibit good and stable flow properties, because filling of the dose measuring cups or cavities is mostly under the influence of the force of gravity.

For reloaded single dose and multiple unit dose inhalers, the dose measuring accuracy and reproducibility can be guaranteed by the manufacturer. Multi dose inhalers on the other hand, can contain a much higher number of doses, whereas the number of handlings to prime a dose is generally lower.

Because the inspiratory air stream in multi-dose devices is often straight across the dose measuring cavity, and because the massive and rigid dose measuring systems of multi dose inhalers can not be agitated by this inspiratory air stream, the powder mass is simply entrained from the cavity and little de-agglomeration is obtained during discharge.

Consequently, separate disintegration means are necessary. However in practice, they are not always part of the inhaler design. Because of the high number of doses in multi-dose devices, powder adhesion onto the inner walls of the air conduits and the de-agglomeration means must be minimized and/or regular cleaning of these parts must be possible, without affecting the residual doses in the device. Some multi dose inhalers have disposable drug containers that can be replaced after the prescribed number of doses has been taken (Ex. WO 97/000703). For such semi-permanent multi dose inhalers with disposable drug containers, the requirements to prevent drug accumulation are even more strict.

Apart from applications through dry powder inhalers the compositions of the invention can be administered in aerosols which operate via propellant gases or by means of so-called atomisers, via which solutions of pharmacologically-active substances can be sprayed under high pressure so that a mist of inhalable particles results. The advantage of these atomisers is that the use of propellant gases can be completely dispensed with. Such atomiser is the Respimat® which is described, for example, in PCT Patent Applications Nos. WO 91/14468 and WO 97/12687, reference here is being made to the contents thereof.

Spray compositions for topical delivery to the lung by inhalation may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain the active ingredient (s) and a suitable propellant such as a fluorocarbon or hydrogen-containing chlorofluorocarbon or mixtures thereof, particularly hydrofluoroalkanes, e. g. dichlorodifluoromethane, trichlorofluoromethane, dichlorotetra-fluoroethane, especially 1, 1, 1, 2-tetrafluoroethane, 1, 1, 1, 2, 3, 3, 3-heptafluoro-n-propane or a mixture thereof. Carbon dioxide or other suitable gas may also be used as propellant.

The aerosol composition may be excipient free or may optionally contain additional formulation excipients well known in the art such as surfactants (eg oleic acid or lecithin) and cosolvens (eg ethanol). Pressurised formulations will generally be retained in a canister (eg an aluminium canister) closed with a valve (eg a metering valve) and fitted into an actuator provided with a mouthpiece.

Medicaments for administration by inhalation desirably have a controlled particle size. The optimum particle size for inhalation into the bronchial system is usually 1-10 µm, preferably 2-5 µm, Particles having a size above 20 µm are generally too large when inhaled to reach the small airways. To achieve these particle sizes the particles of the active ingredient as produced may be size reduced by conventional means eg by micronisation. The desired fraction may be separated out by air classification or sieving. Preferably, the particles will be crystalline.

Achieving high dose reproducibility with micronised powders is difficult because of their poor flowability and extreme agglomeration tendency. To improve the efficiency of dry powder compositions, the particles should be large while in the inhaler, but small when discharged into the respiratory tract. Thus, an excipient such as lactose or glucose is generally employed. The particle size of the excipient will usually be much greater than the inhaled medicament within the present invention. When the excipient is lactose it will typically be present as milled lactose, preferably crystalline alpha lactose monohydrate.

Pressurized aerosol compositions will generally be filled into canisters fitted with a valve, especially a metering valve. Canisters may optionally be coated with a plastics material e. g. a fluorocarbon polymer as described in WO96/32150. Canisters will be fitted into an actuator adapted for buccal delivery.

### iv) Nasal mucosal administration

The compounds of the invention may also be administered via the nasal mucosal.

Typical compositions for nasal mucosa administration are typically applied by a metering, atomizing spray pump and are in the form of a solution or suspension in an inert vehicle such as water optionally in combination with conventional excipients such as buffers, anti-microbials, tonicity modifying agents and viscosity modifying agents.

### v) Parenteral Administration

The compounds of the invention may also be administered directly into the blood stream, into mucle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilization, may readily be accomplished using standard pharmaceutical techniques well known to those skilled in the art. The solubility of compounds of the invention used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and PGLA microspheres.

### vi) Topical Administration

The compounds of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibers, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated; see, for example, J Pharm Sci, 88 (10), 955-958 by Finnin and Morgan (October 1999). Other means of topical administration include delivery by electroporation, iontophoresis, phonophoresis, sonophoresis and microneedle or needle-free injection.

Formulations for topical administration may be formulated to be immediate and/or modified release. Modified release formulations include delayed-, sustained-, pulsed-, controlled-, targeted and programmed release.

### vii) Rectal/Intravaginal Administration

Compounds of the invention may be administered rectally or vaginally, for example, in the form of a suppository, pessary, or enema. Cocoa butter is a traditional suppository base, but various alternatives may be used as appropriate. Formulations for rectal/vaginal administration may be formulated to be immediate and/or modified release. Modified release formulations include delayed-, sustained-, pulsed-, controlled-, targeted and programmed release.

### viii) Ocular Administration

Compounds of the invention may also be administered directly to the eye or ear, typically in the form of drops of a micronized suspension or solution in isotonic, pH-adjusted, sterile saline. Other formulations suitable for ocular and aural administration include ointments, biodegradable (e.g. absorbable gel sponges, collagen) and nonbiodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

### ix) Other Technologie

Compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

The amount of the active compound administered will be dependent on the subject being treated, the severity of the disorder or condition, the rate of administration, the disposition of the compound and the discretion of the prescribing physician. However, an effective dosage is typically in the range of 0.01-3000 mg, more preferably 0.5-1000 mg of active ingredient or the equivalent amount of a pharmaceutically acceptable salt thereof per day. Daily dosage may be administered in one or more treatments, preferably from 1 to 4 treatments, per day.

The pharmaceutical formulations may conveniently be presented in unit dosage form and may be prepared by any of the methods well known in the art of pharmacy.

Preferably the composition is in unit dosage form, for example a tablet, capsule or metered aerosol dose, so that the patient may administer a single dose.

The following preparations forms are cited as formulation examples:

### Formulation Example 1 (Oral suspension)

| **Ingredient** | **Amount** |
|---|---|
| Active Compound | 3 mg |
| Citric acid | 0,5 g |
| Sodium chloride | 2,0 g |
| Methyl paraben | 0,1 g |
| Granulated sugar | 25 g |
| Sorbitol (70% solution) | 11 g |
| Veegum K | 1,0 g |
| Flavoring | 0,02 g |
| Dye | 0,5 mg |
| Distilled water | q.s. to 100 mL |

### Formulation Example 2 (Hard gelatine capsule for oral administration)

| **Ingredient** | **Amount** |
|---|---|
| Active Compound | 1 mg |
| Lactose | 150 mg |
| Magnesium stearate | 3 mg |

### Formulation Example 3 (Gelatin cartridge for inhalation)

| **Ingredient** | **Amount** |
|---|---|
| Active Compound (micronized) | 0,2 mg |
| Lactose | 25 mg |

### Formulation Example 4 (Formulation for inhalation with a DPI)

| **Ingredient** | **Amount** |
|---|---|
| Active Compound (micronized) | 15 mg |
| Lactose | 3000 mg |

### Formulation Example 5 (Formulation for a MQI)

| **Ingredient** | **Amount** |
|---|---|
| Active Compound (micronized) | 10 g |
| 1,1,1 2,3,3,3-heptafluoro-n-propane | q.s. to 200 ml |

Modifications, which do not affect, alter, change or modify the essential aspects of the compounds, combinations or pharmaceutical compositions described, are included within the scope of the present invention.

## Claims

1. A compound of formula (I), or a pharmaceutically acceptable salt, solvate, N-oxide, stereoisomer or deuterated derivative thereof: 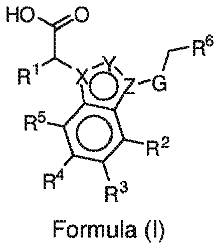 wherein:
• either X represents a nitrogen atom, Z represents a carbon atom and Y represents a nitrogen atom or a -CR⁹ group; or X represents a carbon atom, Z represents a nitrogen atom and Y represents a nitrogen atom or a -CR⁹ group:
• R¹ is selected from the group consisting of a hydrogen atom and a linear or branched C₁₋₄ alkyl group;
• R² R³, R⁴ and R⁵ are each independently selected from the group consisting of a hydrogen atom, a halogen atom, a linear or branched C₁₋₄ alkyl group, a C₃₋₇ cycloalkyl group, a linear or branched 6₁₋₄ haloalkyl group, a -CN group, a -OH group, a -OR^{a} group, a -CO₂H group, a -CO₂R^{a} group, a -SR^{a} group, and a-SO₂R^{a} group;
• G is selected from the group consisting of a phenyl group and a 5 to 6-membered N-containing heteroaryl group, wherein the phenyl and heteroaryl groups are optionally substituted by one or more substituents selected from the group consisting of a halogen atom, a cyano group, a nitro group, a -CHF₂ group, a -CF₃ group, a linear or branched C₁₋₄ alkyl group, a C₃₋₇ cycloalkyl group, a -OH group, a -OR^{a} group, a -SR^{a} group and a -SO₂R^{a} group;
• R⁶ is selected from the group consisting of a -N(R⁷)C(O)R⁸ group, a-N(R⁷)SO₂R⁸ group and a heteroaryl group selected from the group consisting of a triazolyl group, an isoxazolyl group, a pyrazolyl group and an oxazolyl group, wherein the heteroaryl group is optionally substituted by one or more substituents selected from the group consisting of a halogen atom, a -CF₃ group, a linear or branched C₁₋₄ alkyl group, a C₃₋₇ cycloalkyl group, a benzyl, group, a phenyl group, a hydroxy group and a carboxy group;
• R⁷ is selected from the group consisting of a linear or branched C₁₋₄ alkyl group; aC₃₋₇cycloalkyl group, a linear or branched C₁-C₄ haloalkyl group and a benzyl, group;
• R⁸ is selected from the group consisting of a linear or branched C₁₋₄ alkyl group, a -(CH₂)ₙ-C₃₋₇ cycloalkyl group, a -CF₃ group, a linear or branched C₁₋₄ alkoxy group, a -C₁₋₄ alkyl-C₁₋₄ alkoxy group, a -(CH₂)ₙNR^{b}R^{c} group, a 4- to 6-membered heterocyclyl group containing at least one heteroatom selected from N, S and O, a -(CH₂)ₙ-phenyl group, a -(CH₂)ₙ-(5- to 6-membered heteroaryl) group containing at least one heteroatom selected from N, S and O, wherein the phenyl, heteroaryl and heterocyclyl groups are optionally substituted by one or more substituents selected from the group consisting of a halogen atom, a linear or branched C₁₋₄ alkyl group, an acyl group and a linear or branched C₁₋₄ alkoxy group;
• R⁹ is selected from the group consisting of a hydrogen atom, a linear or branched C₁₋₄ alkyl group, a -CF₃ group, a C₃₋₇ cycloalkyl group and a halogen atom;
• R^{a} is selected from the group consisting of a linear or branched C₁₋₄ alkyl group, a linear or branched C₁₋₄ haloakyl group and a C₃₋₇ cycloalkyl group;
• R^{b} and R^{c} are independently selected from the group consisting of a hydrogen atom, a linear or branched C₁₋₄ alkyl group, a C₃-₇ cycloalkyl group, a phenyl group, a 5- to 6-membered heteroaryl containing at least one heteroatom selected from N, S and O, a -C₁₋₄alkyl-phenyl group and -C₁₋₄alkyl-5- to 6-membered heteroaryl group; or R^{b} and R^{c} together with the nitrogen atom to which they are attached form a 3-to 7-membered N-containing heterocycyl group which is optionally substituted by one or more substituents selected by the group consisting of a halogen atom, a linear or branched C₁₋₄ alkyl group and a linear or branched C₁₋₄ alkoxy group; and
• n is an integer selected from 0 to 2.

2. A compound according to claim 1, wherein either X represents a nitrogen atom, Z represents a carbon atom and Y represents a nitrogen atom or a -CR⁹ group; or X represents a carbon atom, Z represents a nitrogen atom and Y represents a -CR⁹ group.

3. A compound according to claim 1 or 2, wherein R¹ is selected from the group consisting of a hydrogen atom and methyl group.

4. A compound according to any one of claims 1 to 3, wherein R², R³, R⁴ and R³ are each independently selected from the group consisting of a hydrogen atom, a fluorine atom, a chlorine atom, a methyl group, a methoxy group, a -CN group, a cyclopropyl group, a -CF₃ group and a -CHF₂ group.

5. A compound according to any one of claims 1 to 4, wherein G is selected from the group consisting of a phenyl group, a pyridyl group, a pyrimidinyl group, a pyridazinyl group and a pyrazolyl group, wherein said ring groups are optionally substituted by one or two substituents selected from the group consisting of a halogen atom, a methyl group, a -SO₂Me group, a -CHF₂ group and a -CF₃ group.

6. A compound according to claim 5, wherein G represents a phenyl group optionally substituted by one substitutent selected from the group consisting of a fluorine atom and a -CF₃ group.

7. A compound according to any preceding claim, wherein R⁶ is selected from the group consisting of a -N(R⁷)COR⁸ group, -N(R⁷)SO₂R⁸ and a heteroaryl group selected from the group consisting of a triazolyl group and an isoxazolyl group, wherein the heteroaryl group is optionally substituted by one or more substituents selected from the group consisting of a methyl group and a cyclopropyl group.

8. A compound according to any preceding claim, wherein R⁷ is selected from the group consisting of an ethyl group and a benzyl group.

9. A compound according to any preceding claim, wherein R⁸ is selected from the group consisting of a linear or branched C₁₋₄ alkyl group, a -(CH₂)ₙ-C₃₋₇ cycloalkyl group, a linear or branched C₁₋₄ alkoxy group, a -C₁₋₂ alkyl-C₁₋₂ alkoxy group, a benzyl group, a -(CH₂)ₙNR^{b}R^{c} group, a 4- to 6-membered heterocyclyl group containing at least one heteroatom selected from N, S and O, and a -(CH₂)ₙ-(5- to 6-membered N-containing heteroaryl) group, wherein the benzyl, heterocyclyl and heteroaryl groups are optionally substituted by one or more substituents selected from the group consisting of a halogen atom, an acyl group and a methyl group.

10. A compound according to any preceding claim, wherein R⁹ is selected from the group consisting of a hydrogen atom and a methyl group.

11. A compound according to any preceding claim, wherein
• either X represents a nitrogen atom, Z represents a carbon atom and Y represents a nitrogen atom or a -CR⁹ group; or X represents a carbon atom, Z represents a nitrogen atom and Y represents a -CR⁹ group;
• R¹ is selected from the group consisting of a hydrogen atom and methyl group;
• R², R³, R⁴ and R⁵ are each independently selected from the group consisting of a hydrogen atom, a fluorine atom, a chlorine atom, a methyl group, a methoxy group, a -CN group, a cyclopropyl group, a -CF₃ group and a -CHF₂ group;
• G is selected from the group consisting of a phenyl group, a pyridyl group, a pyrimidinyl group, a pyridazinyl group and a pyrazolyl group, wherein said ring groups are optionally substituted by one or two substituents selected from the group consisting of a halogen atom, a methyl group, a -CHF₂ group and a -CF₃ group; preferably G represents a phenyl group optionally substituted by one substitutent selected from the group consisting of a fluorine atom and a -CF₃ group;
• R⁶ is selected from the group consisting of a -N(R⁷)C(O)R⁸ group, -N(R⁷)SO₂R⁸ and a heteroaryl group selected from the group consisting of a triazolyl group and an isoxazolyl group, wherein the heteroaryl group is optionally substituted by one or more substituents selected from the group consisting of a methyl group and a cyclopropyl group;
• R⁷ is selected from the group consisting of an ethyl group and a benzyl group;
• R⁸ is selected from the group consisting of a linear or branched C₁₋₄ alkyl group, a -(CH₂)ₙ-C₃₋₇ cycloalkyl group, a linear or branched C₁₋₄ alkoxy group, a -C₁₋₂ alkyl-C₁₋₂ alkoxy group, a benzyl group, a -(CH₂)ₙNR^{b}R^{c} group, a 4- to 6-membered heterocyclyl group containing at least one heteroatom selected from N, S and O, and a -(CH₂)ₙ-(5- to 6-membered N-containing heteroaryl) group, wherein the benzyl, heterocyclyl and heteroaryl groups are optionally substituted by one or more substituents selected from the group consisting of a halogen atom, an acyl group and a methyl group, wherein R^{b} and R^{c} are preferably independently selected from a hydrogen atom, a linear or branched C₁₋₄ alkyl group, a C₃₋₇ cycloalkyl group, a phenyl group and a benzyl group;
• R⁹ is selected from the group consisting of a hydrogen atom and a methyl group; and
• n is 0 or 1.

12. A compound according to claim 1, wherein
• either X represents a nitrogen atom, Z represents a carbon atom and Y represents a nitrogen atom or a -CR⁹ group; or X represents a carbon atom, Z represents a nitrogen atom and Y represents a -CR⁹ group;
• R¹ is selected from the group consisting of a hydrogen atom and methyl group;
• R², R³, R⁴ and R⁵ are each independently selected from the group consisting of a hydrogen atom, a fluorine atom, a chlorine atom, a methyl group, a methoxy group, a -CN group, a cyclopropyl group and a -CF₃ group;
• G is selected from the group consisting of a pyridyl group and a phenyl group optionally substituted by one substitutent selected from the group consisting of a fluorine atom and a -CF₃ group;
• R⁶ is selected from the group consisting of a -N(R⁷)C(O)R⁸ group, -N(R⁷)SO₂R⁸ and a heteroaryl group selected from the group consisting of a triazolyl group and an isoxazolyl group, wherein the heteroaryl group is optionally substituted by one or more substituents selected from the group consisting of a methyl group and a cyclopropyl group;
• R⁷ is an ethyl group;
• R⁸ is selected from the group consisting of a linear or branched C₁₋₄ alkyl group, a -(CH₂)ₙ-C₃₋₇ cycloalkyl group, a linear or branched C₁₋₄ alkoxy group, and a-(CH₂)ₙNR^{b}R^{c} group wherein R^{b} represents a hydrogen atom and R^{c} represents a benzyl group;
• R⁹ is selected from the group consisting of a hydrogen atom and a methyl group; and
• n is 0 or 1.

13. A compound according to claim 1 which is one of:
{3-[2-{[(Cyclopropylcarbonyl)(ethyl)amino]methyl}-4-(trifluoromethyl)phenyl]-1H-indazol-1-yl}acetic acid;
{4-Chloro-3-[2-{[(cyclopropylcarbonyl)(ethyl)amino]methyl}-4-(trifluoromethyl)phenyl]-1H-indazol-1-yl}acetic acid;
{3-[2-{[(Cyclopropylcarbonyl)(ethyl)amino]methyl}-4-(trifluoromethyl)phenyl]-5-fluoro-1H-indazol-1-yl}acetic acid;
[4-Chloro-3-(2-{[(cyclopropylcarbonyl)(ethyl)amino]methyl}-4-fluorophenyl)-1H-indazol-1-yl]acetic acid;
{5-Chloro-3-[2-{[(cyclopropylcarbonyl)(ethyl)amino]methyl}-4-(trifluoromethyl)phenyl]-1H-indazol-1-yl}acetic acid;
{3-[2-{[(Cyclopropylcarbonyl)(ethyl)amino]methyl}-4-(trifluoromethyl)phenyl]-6-fluoro-1H-indazol-1-yl}acetic acid;
{3-[2-{[(Cyclopropylcarbonyl)(ethyl)amino]methyl}-4-(trifluoromethyl)phenyl]-4-methoxy-1H-indazol-1-yl}acetic acid;
[3-(2-{[(Cyclopropylcarbonyl)(ethyl)amino]methyl}-4-fluorophenyl)-5-(trifluoromethyl)-1H-indazol-1-yl]acetic acid;
2-{4-Chloro-3-[2-{[(cyclopropylcarbonyl){ethyl)amino]methyl}-4-(trifluoromethyl)phenyl]-1H-indazol-1-yl}propanoic acid;
[3-(2-{[(Cyclopropylcarbonyl)(ethyl)amino]methyl}-4-fluorophenyl}-4-(trifluoromethyl)-1H-indazol-1-yl]acetic acid;
[5-Chloro-3-(2-{[(cyclopropylcarbonyl)(ethyl)amino]methyl}-4-fluorophenyl)-1H-indazol-1-yl]acetic acid;
{4-Cyano-3-[2-{[(cyclopropylcarbonyl)(ethyl)amino]methyl}-4-(trifluoromethyl)phenyl]-1H-indazol-1-yl}acetic acid;
2-{5-Chloro-3-[2-{[(cyclopropylcarbonyl)(ethyl)amino]methyl)-4-(trifluoromethyl)phenyl]-1H-indazol-1-yl}propanoic acid;
{3-[2-{[(Cyclopropylcarbonyl)(ethyl)amino]methyl}-4-(trifluoromethyl)phenyl]-4-fluoro-1H-indazol-1-yl}acetic acid;
{3-[2-{[(Cyclopropylcarbonyl)(ethyl)amino]methyl}-4-(trifluoromethyl)phenyl]-5-methyl-1H-indazol-1-yl}acetic acid;
{3-[2-{[(Cyclopropylcarbonyl)(ethyl)amino]methyl}-4-(trifluoromethyl)phenyl]-4-methyl-1H-indazol-1-yl}acetic acid;
{4-Chloro-3-[2-{[(cyclopropylcarbonyl)(ethyl)amino]methyl}-4-(trifluoromethyl)phenyl]-5-fluoro-1H-indazol-1-yl}acetic acid;
[3-(2-{[(tert-Butoxycarbonyl)(ethyl)amino]methyl}-4-fluorophenyl)-5-chloro-1H-indazol-1-yl]acetic acid;
[5-Chloro-3-(2-{[(cyclobutylcarbonyl)(ethyl)amino]methyl}-4-fluorophenyl)-1H-indazol-1-yl]acetic acid;
[5-Chloro-3-(2-{[ethyl(isobutyryl)amino]methyl}-4-fluorophenyl)-1H-indazol-1-yl]acetic acid;
[5-Chloro-3-(2-{[ethyl(methylsulfonyl)amino]methyl}-4-fluorophenyl)-1H-indazol-1-yl]acetic acid;
[3-(2-{[Acetyl(ethyl)amino]methyl}-4-fluorophenyl)-5-chloro-1H-indazol-1-yl]acetic acid;
{3-[2-{[[(Benzylamino)carbonyl](ethyl)amino]methyl}-4-(trifluoromethyl)phenyl]-4-chloro-1H-indazol-1-yl}acetic acid;
[5-Chloro-3-(2-{[(cyclopropylcarbonyl)(ethyl)amino]methyl}phenyl)-1H-indazol-1-yl]acetic acid;
[5-Chloro-3-(2-{[(cyclopentylcarbonyl)(ethyl)amino]methyl}-4-fluorophenyl)-1H-indazol-1-yl]acetic acid;
[5-Chloro-3-(2-{[(cyclopropylacetyl)(ethyl)amino]methyl}-4-fluorophenyl)-1H-indazol-1-yl]acetic acid;
{5-Chloro-3-[2-{[(cyclopropylcarbonyl)(ethyl)amino]methyl}-4-(trifluoromethyl)phenyl]-4-fluoro-1H-indazol-1-yl}acetic acid;
{1-[2-{[(Cyclopropylcarbonyl)(ethyl)amino]methyl}-4-(trifluoromethyl)phenyl]-1H-indol-3-yl}acetic acid;
[1-(2-{[(Cyclopropylcarbonyl)(ethyl)amino]methyl}-4-fluorophenyl)-1H-indol-3-yl]acetic acid;
{1-[2-{[(Cyclopropylcarbonyl)(ethyl)amino]methyl}-4-(trifluoromethyl)phenyl]-6-fluoro-1 H-indol-3-yl}acetic acid;
{3-[2-{[(Cyclopropylcarbonyl)(ethyl)amino]methyl}-4-(trifluoromethyl)phenyl]-1H-indol-1-yl}acetic acid;
{3-[2-{[(Cyclopropylcarbonyl)(ethyl)amino]methyl}-4-(trifluoromethyl)phenyl]-2-methyl-1H-indol-1-yl}acetic acid;
{3-[2-{[(Cyclopropylcarbonyl)(ethyl)amino]methyl}-4-(trifluoromethyl)phenyl]-5-fluoro-1 H-indol-1-yl}acetic acid;
{4-Chloro-3-[2-{[(cyclopropylcarbonyl)(ethyl)amino]methyl}-4-(trifluoromethyl)phenyl]-1 H-indol-1-yl}acetic acid;
{6-Chloro-3-[2-{[(cyclopropylcarbonyl)(ethyl)amino]methyl}-4-(trifluoromethyl)phenyl]-1H-indol-1-yl}acetic acid;
{5-Cyano-3-[2-{[(cyclopropylcarbonyl)(ethyl)amino]methyl}-4-(trifluoromethyl)phenyl]-1 H-indol-1-yl}acetic acid;
[3-(2-{[(Cyclopropylcarbonyl)(ethyl)amino]methyl}-4-fluorophenyl)-1H-indol-1-yl]acetic acid;
{5-Cyclopropyl-3-[2-{[(cyclopropylcarbonyl)(ethyl)amino]methyl}-4-(trifluoromethyl)phenyl]-1H-indol-1-yl}acetic acid;
[3-(2-{[(Cyclopropylcarbonyl)(ethyl)amino]methyl}-4-fluorophenyl)-5-(trifluoromethyl)-1 H-indol-1-yl]acetic acid;
{3-[2-{[(Cyclopropylcarbonyl)(ethyl)amino]methyl}-4-(trifluoromethyl)phenyl]-4-fluoro-1 H-indol-1-yl}acetic acid;
2-{3-[2-{[(Cyclopropylcarbonyl)(ethyl)amino]methyl}-4-(trifluoromethyl)phenyl]-5-fluoro-1 H-indol-1-yl}propanoic acid;
[3-(2-{[(Cyclopropylcarbonyl)(ethyl)amino]methyl}-4-fluorophenyl)-5-fluoro-1H-indol-1-yl]acetic acid;
{3-[2-{[[(Benzylamino)carbonyl](ethyl)amino]methyl}-4-(trifluoromethyl)phenyl]-5-fluoro-1 H-indol-1-yl}acetic acid;
2-{4-Choro-3-[2-{[(cyclopropylcarbonyl)(ethyl)amino]methyl}-4-(trifluoromethyl)phenyl]-1 H-indol-1-yl}propanoic acid;
[4-Chloro-3-(2-{[(cyclopropylcarbonyl)(ethyl)amino]methyl}-4-fluorophenyl)-1H-indol-1-yl]acetic acid;
{3-[2-[(3-Cyclopropyl-5-methyl-4H-1,2,4-triazol-4-yl)methyl]-4-(trifluoromethyl)phenyl]-5-fluoro-1H-indol-1-yl}acetic acid;
{4-Chloro-3-[2-{[(cyclopropylcarbonyl)(ethyl)amino]methyl}-4-(trifluoromethyl)phenyl]-5-fluoro-1H-indol-1-yl}acetic acid;
[4-Chloro-3-(2-{[(cyclopropylcarbonyl)(ethyl)amino]methyl}-4-fluorophenyl)-5-fluoro-1H-indol-1-yl]acetic acid;
[3-(3-{[(Cyclopropylcarbonyl)(ethyl)amino]methyl}pyridin-2-yl)-5-fluoro-1H-indol-1-yl]acetic acid;
{3-[2-{[(Cyclopropylcarbonyl)(ethyl)amino]methyl}-4-(trifluoromethyl)phenyl]-4,5-difluoro-1 H-indol-1-yl}acetic acid;
[5-Chloro-3-(2-{[(cyclopropylcarbonyl)(ethyl)amino]methyl}-4-fluorophenyl)-1H-indol-1-yl]acetic acid;
[3-(2-{[(Cyclopropylcarbonyl)(ethyl)amino]methyl}-4-fluorophenyl)-4-fluoro-1H-indol-1-yl]acetic acid;
{5-Chloro-3-[2-{[(cyclopropylcarbonyl)(ethyl)amino]methyl}-4-(trifluoromethyl)phenyl]-1 H-indol-1-yl}acetic acid;
[3-(2-{[(Cyclopropylcarbonyl)(ethyl)amino]methyl}phenyl)-5-fluoro-1H-indol-1-yl]acetic acid;
[3-(3-{[(Cyclopropylcarbonyl)(ethyl)amino]methyl}pyridin-2-yl)-5-{trifluoromethyl)-1H-indol-1-yl]acetic acid; or
{3-[2-[(5-Cyclopropyl-3-methylisoxazol-4-yl)methyl]-4-(trifluoromethyl)phenyl]-5-fluoro-1H-indol-1-yl}acetic acid.

14. A compound according to any one of claims 1 to 13 for use in the treatment of the human or animal body by therapy.

15. A compound according to any one of claims 1 to 13 for use in the treatment of a pathological condition or disease mediated by prostaglandin D₂, which condition or disease is preferably selected from asthma, chronic obstructive pulmonary disease (COPD), rhinitis, emphysema, allergic airway syndrome, allergic rhinobronchitis, psoriasis, dermatitis, allergic conjunctivitis, atopic dermatitis, pulmonary hypertension, interstitial lung fibrosis, allergy, psoriasis, adult respiratory distress syndrome, myocardial infarction, aneurysm, stroke, cancer, wound healing, endotoxic shock, pain, inflammatory conditions, eosinophilic esophagitis, eosinophil-associated gastrointestinal disorders (EGID), idiopathic, otitis, airway constriction, mucus secretion, nasal congestion, increased microvascular permeability and recruitment of eosinophils, urticaria, sinusitis, angioedema, anaphylaxia, chronic cough, Churg Strauss syndrome, arthritis, Crohn's disease, ulcerative colitis, irritable bowel disease and inflammatory bowel disease, more preferably selected from asthma, chronic obstructive pulmonary disease (COPD), allergic rhinitis and atopic dermatitis.

16. Use of a compound as defined in any one of claims 1 to 13 for the manufacture of a medicament for the treatment of a pathological condition or disease as defined in claim 15.

17. A pharmaceutical composition comprising a compound as defined in any one of claim 1 to 13 in association with a pharmaceutically acceptable diluent or carrier.

18. A method for treating a subject afflicted with a pathological condition or disease as defined in claim 15, which comprises administering to said subject an effective amount of a compound as defined in any one of claims 1 to 13.

19. A combination product comprising (i) a compound according to any one of claims 1 to 13; and (ii) another compound selected from
a. Anticholinergics, such as aclidinium bromide or tiotropium,
b. Corticosteroids, or gluococorticoids such as prednisone or methylprednisolone,
c. Antihistamines, such as ebastine, ranitidine, ABT-239 or JNJ 7777120;
d. Beta-2 agonists, such as salmeterol or formoterol,
e. Chemokine receptor antagonists, such as maraviroc or enfuvirtide,
f. Leukotriene receptor antagonists,
g. JAK inhibitors such as tasocitinib citrate or INCB018424,
h. Syk inhibitors such as R-112,
i. Phosphosdiesterase IV inhibitors such as roflumilast or revamilast,
j. Dual beta-2 adrenoceptor agonist/M3 receptor antagonist (MABA compounds) such as GSK-961081,
k. p38 Inhibitors such as ARRY-797,
l. PKC inhibitors such as NVP-AEB071,
m. 5-lipoxygenase activating protein inhibitors, such as veliflapon,
n. 5-lipoxygenase inhibitors,
o. CYSLTR1 antagonists, such as montelukast, pranlukast or zafirlukast;
p. CYSLTR2 antagonists, such as pranlukast, zafirlukast or tipilakast;
q. BLT1 antagonists,
r. BLT2 antagonists,
s. Thromboxane A2 antagonists such as ramatroban,
t. DP1 receptor antagonists, such as laropiprant,
u. DP1 receptor agonists, such as BW-245C,
v. IP receptor agonists, such as RO-1138452,
w. Anti-IgE, such as omalizumab,
x. IL5 antibody, such as mepolizumab,
y. Leukotriene formation inhibitors,
z. Bronchodilators, such as pirbuterol, epinephrine, ephedrine or salbutamol;
aa. Decongestants, such as ephedrine, levo-methamphetamine, naphazoline, oxymetazoline, phenylephrine, phenylpropanolamine, propylhexedrine, pseudoephedrine, synephrine or tetrahydrozoline;
bb. Mucolytics such as acetylcysteine, ambroxol, bromhexine, carbocisteine, domiodol, eprazinone, erdosteine, letosteine, neltenexine, sobrerol, stepronin or tiopronin;
cc. Antitussives, such as dextromethorphan,
dd. Analgesics such as aspirin, paracetamol, rofecoxid, celecoxib, morphine, codeine, oxycodone, hydrocodone, dihydromorphine or flupirtine; and
ee. Expectorants such antimony pentasulfide, guaiacolsulfonate, guaifenesin, potassium iodide or tyloxapol.
